# EUROPEAN PATENT SPECIFICATION

(11) **EP 3 810 607 B1**
(45) Date of publication and mention of the grant of the patent: **02.12.2026**
(21) Application number: 19734158.9
(22) Date of filing: 24.06.2019
(51) Int. Cl.: C07D 413/14, C07D 451/02, C07D 451/14, C07D 453/06, C07D 471/10, C07D 487/10, C07D 498/08, C07D 498/10, A61P 25/28, A61K 31/5386, A61K 31/454, A61K 31/46, A61K 31/4245, A61K 31/55

(54) **OXADIAZOLES AS AGONISTS OF THE MUSCARINIC M1 AND/OR M4 RECEPTOR**
OXADIAZOLE ALS AGONISTEN DES MUSKARINISCHEN M1- UND/ODER M4-REZEPTORS
OXADIAZOLES À UTILISER EN TANT QU'AGONISTES DU RÉCEPTEUR MUSCARINIQUE M1 ET/OU M4

(30) Priority: 22.06.2018 GB 201810245
(43) Date of publication of application: 28.04.2021
(60) Divisional application: 26214080.9
(73) Proprietor: Nxera Pharma UK Limited, Cambridge, Cambridgeshire CB21 6DG (GB)
(72) Inventor: BROWN, Giles Albert, Cambridge CB21 6DG (GB); TEOBALD, Barry John, Cambridge CB21 6DG (GB); TEHAN, Benjamin Gerald, Cambridge CB21 6DG (GB)
(74) Representative: Mewburn Ellis LLP
(86) International application number: PCT/GB2019/051778
(87) International publication number: WO 2019/243850

(56) References cited:
- WO-A1-2012/037393
- WO-A1-2014/045031
- WO-A1-2014/122474
- WO-A1-2015/118342
- WO-A1-2015/140559
- WO-A1-2017/021729
- WO-A1-2017/021730
- WO-A1-2018/069732
- WO-A1-99/32486

## Description

### Technical Field

This invention relates to a class of novel heterocyclic compounds, their salts, pharmaceutical compositions containing them and their use in therapy of the human body. In particular, the invention is directed to a class of compounds, which are agonists of the muscarinic M₁ receptor and/or M₄ receptor, and hence are useful in the treatment of Alzheimer's Disease, schizophrenia, cognitive disorders and other diseases mediated by the muscarinic M₁/M₄ receptors, as well as the treatment or alleviation of pain.

### Background of the Invention

Muscarinic acetylcholine receptors (mAChRs) are members of the G protein-coupled receptor superfamily which mediate the actions of the neurotransmitter acetylcholine in both the central and peripheral nervous system. Five mAChR subtypes have been cloned, M₁ to M₅. The M₁ mAChR is predominantly expressed post-synaptically in the cortex, hippocampus, striatum and thalamus; M₂ mAChRs are located predominantly in the brainstem and thalamus, though also in the cortex, hippocampus and striatum where they reside on cholinergic synaptic terminals (Langmead et al., 2008 Br J Pharmacol). However, M₂ mAChRs are also expressed peripherally on cardiac tissue (where they mediate the vagal innervation of the heart) and in smooth muscle and exocrine glands. M₃ mAChRs are expressed at relatively low level in the CNS but are widely expressed in smooth muscle and glandular tissues such as sweat and salivary glands (Langmead et al., 2008 Br J Pharmacol).

Muscarinic receptors in the central nervous system, especially the M₁ mAChR, play a critical role in mediating higher cognitive processing. Diseases associated with cognitive impairments, such as Alzheimer's disease, are accompanied by loss of cholinergic neurons in the basal forebrain (Whitehouse et al., 1982 Science)*.* In schizophrenia, which is also characterised by cognitive impairments, mAChR density is reduced in the pre-frontal cortex, hippocampus and caudate putamen of schizophrenic subjects (Dean et al., 2002 Mol Psychiatry)*.* Furthermore, in animal models, blockade or lesion of central cholinergic pathways results in profound cognitive deficits and non-selective mAChR antagonists have been shown to induce psychotomimetic effects in psychiatric patients. Cholinergic replacement therapy has largely been based on the use of acetylcholinesterase inhibitors to prevent the breakdown of endogenous acetylcholine. These compounds have shown efficacy versus symptomatic cognitive decline in the clinic, but give rise to dose-limiting side effects resulting from stimulation of peripheral M₂ and M₃ mAChRs including disturbed gastrointestinal motility, bradycardia, nausea and vomiting (http://www.drugs.com/pro/donepezil.html; http://www.drugs.com/pro/rivastigmine.html).

Further discovery efforts have targeted the identification of direct M₁ mAChR agonists to target increases in cognitive function. Such efforts resulted in the identification of a range of agonists, exemplified by compounds such as xanomeline, AF267B, sabcomeline, milameline and cevimeline. Many of these compounds have been shown to be highly effective in pre-clinical models of cognition in both rodents and / or non-human primates. Milameline has shown efficacy versus scopolamine-induced deficits in working and spatial memory in rodents; sabcomeline displayed efficacy in a visual object discrimination task in marmosets and xanomeline reversed mAChR antagonist-induced deficits in cognitive performance in a passive avoidance paradigm.

Alzheimer's disease (AD) is the most common neurodegenerative disorder (26.6 million people worldwide in 2006) that affects the elderly, resulting in profound memory loss and cognitive dysfunction. The aetiology of the disease is complex, but is characterised by two hallmark brain sequelae: aggregates of amyloid plaques, largely composed of amyloid-β peptide (Aβ), and neurofibrillary tangles, formed by hyperphosphorylated tau proteins. The accumulation of Aβ is thought to be the central feature in the progression of AD and, as such, many putative therapies for the treatment of AD are currently targeting inhibition of Aβ production. Aβ is derived from proteolytic cleavage of the membrane bound amyloid precursor protein (APP). APP is processed by two routes, non-amyloidgenic and amyloidgenic. Cleavage of APP by γ-secretase is common to both pathways, but in the former APP is cleaved by an α-secretase to yield soluble APPα. The cleavage site is within the Aβ sequence, thereby precluding its formation. However, in the amyloidgenic route, APP is cleaved by β-secretase to yield soluble APPβ and also Aβ. *In vitro* studies have shown that mAChR agonists can promote the processing of APP toward the soluble, non-amyloidogenic pathway. *In vivo* studies showed that the mAChR agonist, AF267B, altered disease-like pathology in the 3xTgAD transgenic mouse, a model of the different components of Alzheimer's disease (Caccamo et al., 2006 Neuron)*.* Finally, the mAChR agonist cevimeline has been shown to give a small, but significant, reduction in cerebrospinal fluid levels of Aβ in Alzheimer's patients, thus demonstrating potential disease modifying efficacy (Nitsch et al., 2000 Neurol).

Furthermore, preclinical studies have suggested that mAChR agonists display an atypical antipsychotic-like profile in a range of pre-clinical paradigms. The mAChR agonist, xanomeline, reverses a number of dopamine driven behaviours, including amphetamine induced locomotion in rats, apomorphine induced climbing in mice, dopamine agonist driven turning in unilateral 6-OH-DA lesioned rats and amphetamine induced motor unrest in monkeys (without EPS liability). It also has been shown to inhibit A10, but not A9, dopamine cell firing and conditioned avoidance and induces c-fos expression in prefrontal cortex and nucleus accumbens, but not in striatum in rats. These data are all suggestive of an atypical antipsychotic-like profile (Mirza et al., 1999 CNS Drug Rev)*.* Muscarinic receptors have also been implicated in the neurobiology of addiction. The reinforcing effects of cocaine and other addictive substances are mediated by the mesolimbic dopamine system where behavioral and neurochemical studies have shown that the cholinergic muscarinic receptor subtypes play important roles in regulation of dopaminergic neurotransmission. For example M(4) (-/-) mice demonstrated significantly enhanced reward driven behaviour as result of exposure to cocaine (Schmidt et al Psychopharmacology (2011) Aug;216(3):367-78). Furthermore xanomeline has been demonstrated to block the effects of cocaine in these models.

Muscarinic receptors are also involved in the control of movement and potentially represent novel treatments for movement disorders such as Parkinson's disease, ADHD, Huntington's disease, tourette's syndrome and other syndromes associated with dopaminergic dysfunction as an underlying pathogenetic factor driving disease.

Xanomeline, sabcomeline, milameline and cevimeline have all progressed into various stages of clinical development for the treatment of Alzheimer's disease and/or schizophrenia. Phase II clinical studies with xanomeline demonstrated its efficacy versus various cognitive symptom domains, including behavioural disturbances and hallucinations associated with Alzheimer's disease (Bodick et al., 1997 Arch Neurol). This compound was also assessed in a small Phase II study of schizophrenics and gave a significant reduction in positive and negative symptoms when compared to placebo control (Shekhar et al., 2008 Am J Psych)*.* However, in all clinical studies xanomeline and other related mAChR agonists have displayed an unacceptable safety margin with respect to cholinergic side effects, including nausea, gastrointestinal pain, diarrhea, diaphoresis (excessive sweating), hypersalivation (excessive salivation), syncope and bradycardia.

Muscarinic receptors are involved in central and peripheral pain. Pain can be divided into three different types: acute, inflammatory, and neuropathic. Acute pain serves an important protective function in keeping the organism safe from stimuli that may produce tissue damage however management of post-surgical pain is required. Inflammatory pain may occur for many reasons including tissue damage, autoimmune response, and pathogen invasion and is triggered by the action of inflammatory mediators such as neuropeptides and prostaglandins which result in neuronal inflammation and pain. Neuropathic pain is associated with abnormal painful sensations to non-painful stimuli. Neuropathic pain is associated with a number of different diseases/traumas such as spinal cord injury, multiple sclerosis, diabetes (diabetic neuropathy), viral infection (such as HIV or Herpes). It is also common in cancer both as a result of the disease or a side effect of chemotherapy. Activation of muscarinic receptors has been shown to be analgesic across a number of pain states through the activation of receptors in the spinal cord and higher pain centres in the brain. Increasing endogenous levels of acetylcholine through acetylcholinesterase inhibitors, direct activation of muscarinic receptors with agonists or allosteric modulators has been shown to have analgesic activity. In contrast blockade of muscarinic receptors with antagonists or using knockout mice increases pain sensitivity. Evidence for the role of the M₁ receptor in pain is reviewed by D. F. Fiorino and M. Garcia-Guzman, 2012.

More recently, a small number of compounds have been identified which display improved selectivity for the M₁ mAChR subtype over the peripherally expressed mAChR subtypes (Bridges et al., 2008 Bioorg Med Chem Lett*;* Johnson et al., 2010 Bioorg Med Chem Lett*;* Budzik et al., 2010 ACS Med Chem Lett). Despite increased levels of selectivity versus the M₃ mAChR subtype, some of these compounds retain significant agonist activity at both this subtype and the M₂ mAChR subtype. Herein we describe a series of compounds which unexpectedly display high levels of selectivity for the M₁ and/or M₄ mAChR over the M₂ and M₃ receptor subtypes.

WO 2018/069732 A1 describes certain compounds of the following structural formula as modulators of muscarinic M1 and/or M4 receptors, which are allegedly useful in the treatment of CNS diseases and pains:

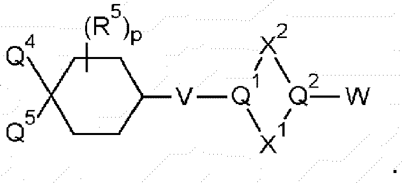

### SUMMARY OF THE INVENTION

The invention is set out in the appended claims.

The references herein to methods of treatment and medical uses are to be interpreted as references to pharmaceutical compositions and medicaments of the present invention for use in those methods or uses.

### Definitions

In this application, the following definitions apply, unless indicated otherwise.

The term "treatment", in relation to the uses of the compounds of the formula (1), formula (1a), formula (2), formula (3) or formula (4), is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" as used herein (for example in relation to methods of treatment of a disease or condition) refers to an amount of the compound which is effective to produce a desired therapeutic effect. For example, if the condition is pain, then the effective therapeutic amount is an amount sufficient to provide a desired level of pain relief. The desired level of pain relief may be, for example, complete removal of the pain or a reduction in the severity of the pain.

The term "alkyl", "heterocyclic" and "ether" are used in their conventional sense (e.g. as defined in the IUPAC Gold Book) unless indicated otherwise.

The term "monocyclic" as used herein refers to an arrangement of atoms arranged in such a way as to form a single ring structure. The term "bicyclic" as used herein refers to an arrangement of atoms arranged in such a way as to form two joined rings. A bicyclic compound can be carbocyclic, or heterocyclic. Moreover, the two rings can both be aliphatic, or can be aromatic, or a combination of aliphatic and aromatic.

In the definitions of X¹, X², X³, X⁴, R¹, R², R⁴, R⁵, R⁶ or R⁷ above, where stated, one or two but not all, carbon atoms of the non-aromatic hydrocarbon groups or alkyl or cycloalkyl groups may optionally be replaced by a heteroatom selected from O and N. Where the group is a single carbon (C₁) group, the carbon can not be replaced. It will be appreciated that when a carbon atom is replaced by a heteroatom, the lower valencies of the heteroatoms compared to carbon means that fewer atoms will be bonded to the heteroatoms than would have been bonded to the carbon atom that has been replaced. Thus, for example, replacement of of a carbon atom (valency of four) in a CH₂ group by oxygen (valency of two) will mean that the resulting molecule will contain two less hydrogen atoms and replacement of a carbon atom (valency of four) in a CH₂ group by nitrogen (valency of three) will mean that the resulting molecule will contain one less hydrogen atom.

Examples of a heteroatom replacements for carbon atoms include replacement of a carbon atom in a -CH₂-CH₂-CH₂- chain with oxygen or sulfur to give an ether -CH₂-O-CH₂- or thioether -CH₂-S-CH₂-, replacement of a carbon atom in a group CH₂-C≡C-H with nitrogen to give a nitrile (cyano) group CH₂-C=N, replacement of a carbon atom in a group -CH₂-CH₂-CH₂- with C=O to give a ketone -CH₂-C(O)-CH₂-, replacement of a carbon atom in a group - CH₂-CH₂-CH₂- with S=O or SO₂ to give a sulfoxide -CH₂-S(O)-CH₂- or sulfone -CH₂-S(O)₂-CH₂-, replacement of a carbon atom in a -CH₂-CH₂-CH₂- chain with C(O)NH to give an amide -CH₂-CH₂-C(O)-NH-, replacement of a carbon atom in a -CH₂-CH₂-CH₂- chain with nitrogen to give an amine -CH₂-NH-CH₂-, and replacement of a carbon atom in a -CH₂-CH₂-CH₂- chain with C(O)O to give an ester (or carboxylic acid) -CH₂-CH₂-C(O)-O-. In each such replacement, at least one carbon atom of the hydrocarbon group must remain.

### Salts

Many compounds of the formula (1), formula (1a), formula (2), formula (3) or formula (4), can exist in the form of salts, for example acid addition salts or, in certain cases salts of organic and inorganic bases such as carboxylate, sulfonate and phosphate salts. All such salts are within the scope of this invention, and references to compounds of the formula (1), formula (1a), formula (2), formula (3) or formula (4), include the salt forms of the compounds as defined in Embodiments 1.40 to 1.47.

The salts are typically acid addition salts.

The salts of the present invention can be synthesized from the parent compound that contains a basic or acidic moiety by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two; generally, nonaqueous media such as ether, ethyl acetate, ethanol, isopropanol, or acetonitrile are used.

Acid addition salts (as defined in Embodiment 1.46) may be formed with a wide variety of acids, both inorganic and organic. Examples of acid addition salts falling within Embodiment 1.46 include mono- or di-salts formed with an acid selected from the group consisting of acetic, 2,2-dichloroacetic, adipic, alginic, ascorbic (e.g. L-ascorbic), L-aspartic, benzenesulfonic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, D-gluconic, glucuronic (e.g. D-glucuronic), glutamic (e.g. L-glutamic), α-oxoglutaric, glycolic, hippuric, hydrohalic acids (e.g. hydrobromic, hydrochloric, hydriodic), isethionic, lactic (e.g. (+)-L-lactic, (±)-DL-lactic), lactobionic, maleic, malic, (-)-L-malic, malonic, (±)-DL-mandelic, methanesulfonic, naphthalene-2-sulfonic, naphthalene-1,5-disulfonic, 1-hydroxy-2-naphthoic, nicotinic, nitric, oleic, orotic, oxalic, palmitic, pamoic, phosphoric, propionic, pyruvic, L-pyroglutamic, salicylic, 4-amino-salicylic, sebacic, stearic, succinic, sulfuric, tannic, (+)-L-tartaric, thiocyanic, *p-*toluenesulfonic, undecylenic and valeric acids, as well as acylated amino acids and cation exchange resins.

Where the compounds of the formula (1), formula (1a), formula (2), formula (3) or formula (4), contain an amine function, these may form quaternary ammonium salts, for example by reaction with an alkylating agent according to methods well known to the skilled person. Such quaternary ammonium compounds are within the scope of formula (1), formula (1a), formula (2), formula (3) or formula (4).

The compounds of the invention may exist as mono- or di-salts depending upon the pKa of the acid from which the salt is formed.

The salt forms of the compounds of the invention are typically pharmaceutically acceptable salts, and examples of pharmaceutically acceptable salts are discussed in Berge et al., 1977, "Pharmaceutically Acceptable Salts," J. Pharm. Sci., Vol. 66, pp. 1-19. However, salts that are not pharmaceutically acceptable may also be prepared as intermediate forms which may then be converted into pharmaceutically acceptable salts. Such non-pharmaceutically acceptable salts forms, which may be useful, for example, in the purification or separation of the compounds of the invention, also form part of the invention.

### Stereoisomers

Stereoisomers are isomeric molecules that have the same molecular formula and sequence of bonded atoms but which differ only in the three-dimensional orientations of their atoms in space. The stereoisomers can be, for example, geometric isomers or optical isomers.

### Geometric Isomers

With geometric isomers, the isomerism is due to the different orientations of an atom or group about a double bond, as in *cis* and *trans* (*Z* and *E*) isomerism about a carbon-carbon double bond, or *cis* and *trans* isomers about an amide bond, or *syn* and *anti* isomerism about a carbon nitrogen double bond (e.g. in an oxime), or rotational isomerism about a bond where there is restricted rotation, or *cis* and *trans* isomerism about a ring such as a cycloalkane ring.

Accordingly, in another embodiment (Embodiment 1.48), the invention provides a geometric isomer of a compound according to any one of Embodiments 1.1 to 1.47.

### Optical Isomers

Where compounds of the formula contain one or more chiral centres, and can exist in the form of two or more optical isomers, references to the compounds include all optical isomeric forms thereof (e.g. enantiomers, epimers and diastereoisomers), either as individual optical isomers, or mixtures (e.g. racemic mixtures) or two or more optical isomers, unless the context requires otherwise.

Accordingly, in another embodiment (Embodiment 1.49) the invention provides a compound according to any one of Embodiments 1.1 to 1.48 which contains a chiral centre.

The optical isomers may be characterised and identified by their optical activity (i.e. as + and - isomers, or d and *l* isomers) or they may be characterised in terms of their absolute stereochemistry using the "R and S" nomenclature developed by Cahn, Ingold and Prelog, see Advanced Organic Chemistry by Jerry March, 4th Edition, John Wiley & Sons, New York, 1992, pages 109-114, and see also Cahn, Ingold & Prelog, Angew. Chem. Int. Ed. Engl., 1966, 5, 385-415. Optical isomers can be separated by a number of techniques including chiral chromatography (chromatography on a chiral support) and such techniques are well known to the person skilled in the art. As an alternative to chiral chromatography, optical isomers can be separated by forming diastereoisomeric salts with chiral acids such as (+)-tartaric acid, (-)-pyroglutamic acid, (-)-di-toluoyl-L-tartaric acid, (+)-mandelic acid, (-)-malic acid, and (-)-camphorsulphonic, separating the diastereoisomers by preferential crystallisation, and then dissociating the salts to give the individual enantiomer of the free base.

Where compounds of the invention exist as two or more optical isomeric forms, one enantiomer in a pair of enantiomers may exhibit advantages over the other enantiomer, for example, in terms of biological activity. Thus, in certain circumstances, it may be desirable to use as a therapeutic agent only one of a pair of enantiomers, or only one of a plurality of diastereoisomers.

Accordingly, in another embodiment (Embodiment 1.50), the invention provides compositions containing a compound according to Embodiment 1.49 having one or more chiral centres, wherein at least 55% (e.g. at least 60%, 65%, 70%, 75%, 80%, 85%, 90% or 95%) of the compound of Embodiment 1.44 is present as a single optical isomer (e.g. enantiomer or diastereoisomer).

In one general embodiment (Embodiment 1.51), 99% or more (e.g. substantially all) of the total amount of the compound (or compound for use) of Embodiment 1.49 is present as a single optical isomer.

For example, in one embodiment (Embodiment 1.52) the compound is present as a single enantiomer.

In another embodiment (Embodiment 1.53), the compound is present as a single diastereoisomer.

The invention also provides mixtures of optical isomers, which may be racemic or non-racemic. Thus, the invention provides:
1.54 A compound according to Embodiment 1.49 which is in the form of a racemic mixture of optical isomers.

1.55 A compound according to Embodiment 1.49 which is in the form of a non-racemic mixture of optical isomers.

### Isotopes

The compounds of the invention as defined in any one of Embodiments 1.1 to 1.55 may contain one or more isotopic substitutions, and a reference to a particular element includes within its scope all isotopes of the element. For example, a reference to hydrogen includes within its scope ¹H, ²H (D), and ³H (T). Similarly, references to carbon and oxygen include within their scope respectively ¹²C, ¹³C and ¹⁴C and ¹⁶O and ¹⁸O.

In an analogous manner, a reference to a particular functional group also includes within its scope isotopic variations, unless the context indicates otherwise. For example, a reference to an alkyl group such as an ethyl group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group).

The isotopes may be radioactive or non-radioactive. In one embodiment of the invention (Embodiment 1.56), the compound of any one of Embodiments 1.1 to 1.53 contains no radioactive isotopes. Such compounds are preferred for therapeutic use. In another embodiment (Embodiment 1.57), however, the compound of any one of Embodiments 1.1 to 1.55 may contain one or more radioisotopes. Compounds containing such radioisotopes may be useful in a diagnostic context.

### Solvates

Compounds of the formula (1), formula (1a), formula (2), formula (3) or formula (4), as defined in any one of Embodiments 1.1 to 1.57 may form solvates. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGE), differential scanning calorimetry (DSC) and X-ray crystallography. The solvates can be stoichiometric or non-stoichiometric solvates. Particularly preferred solvates are hydrates, and examples of hydrates include hemihydrates, monohydrates and dihydrates.

Accordingly, in further embodiments 1.58 and 1.59, the invention provides:
1.58 A compound according to any one of Embodiments 1.1 to 1.57 in the form of a solvate.

1.59 A compound according to Embodiment 1.58 wherein the solvate is a hydrate.

For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.

Alternatively, rather than existing as a hydrate, the compound of the invention may be anhydrous. Therefore, in another embodiment (Embodiment 1.60), the invention provides a compound as defined in any one of Embodiments 1.1 to 1.58 in an anhydrous form (e.g. anhydrous crystalline form).

### Crystalline and amorphous forms

The compounds of any one of Embodiments 1.1 to 1.60 may exist in a crystalline or non-crystalline (e.g. amorphous) state. Whether or not a compound exists in a crystalline state can readily be determined by standard techniques such as X-ray powder diffraction (XRPD). Crystals and their crystal structures can be characterised using a number of techniques including single crystal X-ray crystallography, X-ray powder diffraction (XRPD), differential scanning calorimetry (DSC) and infra red spectroscopy, e.g. Fourier Transform infra-red spectroscopy (FTIR). The behaviour of the crystals under conditions of varying humidity can be analysed by gravimetric vapour sorption studies and also by XRPD. Determination of the crystal structure of a compound can be performed by X-ray crystallography which can be carried out according to conventional methods such as those described herein and as described in Fundamentals of Crystallography, C. Giacovazzo, H. L. Monaco, D. Viterbo, F. Scordari, G. Gilli, G. Zanotti and M. Catti, (International Union of Crystallography/Oxford University Press, 1992 ISBN 0-19-855578-4 (p/b), 0-19-85579-2 (h/b)). This technique involves the analysis and interpretation of the X-ray diffraction of single crystal. In an amorphous solid, the three dimensional structure that normally exists in a crystalline form does not exist and the positions of the molecules relative to one another in the amorphous form are essentially random, see for example Hancock et al. J. Pharm. Sci. (1997), 86, 1).

Accordingly, in further embodiments, the invention provides:
1.61 A compound according to any one of Embodiments 1.1 to 1.60 in a crystalline form.

1.62 A compound according to any one of Embodiments 1.1 to 1.61 which is:
(a) from 50% to 100% crystalline, and more particularly is at least 50% crystalline, or at least 60% crystalline, or at least 70% crystalline, or at least 80% crystalline, or at least 90% crystalline, or at least 95% crystalline, or at least 98% crystalline, or at least 99% crystalline, or at least 99.5% crystalline, or at least 99.9% crystalline, for example 100% crystalline.

1.63 A compound according to any one of Embodiments 1.1 to 1.62 which is in an amorphous form.

### Prodrugs

The compounds of the formula (1), formula (1a), formula (2), formula (3) or formula (4), as defined in any one of Embodiments 1.1 to 1.60 may be presented in the form of a pro-drug. By "prodrugs" is meant for example any compound that is converted *in vivo* into a biologically active compound of the formula (1), formula (1a), formula (2), formula (3) or formula (4), as defined in any one of Embodiments 1.1 to 1.60.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any hydroxyl groups present in the parent compound with, where appropriate, prior protection of any other reactive groups present in the parent compound, followed by deprotection if required.

Also, some prodrugs are activated enzymatically to yield the active compound, or a compound which, upon further chemical reaction, yields the active compound (for example, as in ADEPT, GDEPT, LIDEPT, etc.). For example, the prodrug may be a sugar derivative or other glycoside conjugate, or may be an amino acid ester derivative.

Accordingly, described herein is a pro-drug of a compound as defined in any one of Embodiments 1.1 to 1.60 wherein the compound contains a functional group which is convertible under physiological conditions to form a hydroxyl group or amino group.

### Complexes and clathrates

Also encompassed by formula (1), formula (1a), formula (2), formula (3) or formula (4), in Embodiments 1.1 to 1.64 are complexes (e.g. inclusion complexes or clathrates with compounds such as cyclodextrins, or complexes with metals) of the compounds of Embodiments 1.1 to 1.64.

Accordingly, described herein is a compound according to any one of Embodiments 1.1 to 1.64 in the form of a complex or clathrate.

### Biological activity and therapeutic uses

The compounds of the present invention have activity as muscarinic M₁ and/or M₄ receptor agonists. The muscarinic activity of the compounds can be determined using the Phospho-ERK1/2 assay described in Example A below.

A significant advantage of compounds of the invention is that they are highly selective for the M₁ and/or M₄ receptor relative to the M₂ and M₃ receptor subtypes. Compounds of the invention are neither agonists nor antagonists of the M₂ and M₃ receptor subtypes. For example, whereas compounds of the invention typically have pEC₅₀ values of at least 6 (preferably at least 6.5) and Eₘₐₓ values of greater than 80 (preferably greater than 95) against the M₁ receptor in the functional assay described in Example A, they may have pEC₅₀ values of less than 5 and Eₘₐₓ values of less than 20% when tested against the M₂ and M₃ subtypes in the functional assay of Example A.

Some compounds of the invention have activity at both the M₁ and M₄ receptors.

Accordingly, in Embodiments 2.1, 2.2, and 2.17, the invention provides:
2.1 A compound according to any one of Embodiments 1.1 to 1.65 for use in medicine.

2.2 A compound according to any one of Embodiments 1.1 to 1.65 for use as a muscarinic M₁ and/or M₄ receptor agonist.

2.17 A compound according to any one of Embodiments 1.1 to 1.65 for use in the treatment of a disease or condition mediated by the muscarinic M₁ receptor.

Furthermore:
A compound described herein may be a muscarinic M₁ receptor agonist having a pEC₅₀ greater than 6.9 and an Eₘₐₓ of at least 80 against the M₁ receptor in the assay of Example A herein or an assay substantially similar thereto.

For example, the compound described herein may be a muscarinic M₁ receptor agonist having a pEC₅₀ greater than 7.0.

A compound according to Embodiment 2.3 or Embodiment 2.4 having an Eₘₐₓ of at least 90 against the M₁ receptor.

A compound described herein may be a muscarinic M₁ and/or M₄ receptor agonist having a pEC₅₀ in the range from 6.0 to 8.7 and an Eₘₐₓ of at least 60 against the M₄ receptor in the assay of Example A herein or an assay substantially similar thereto.

A compound described herein may be a muscarinic M₁ and/or M₄ receptor agonist having a pEC₅₀ in the range from 6.0 to 8.1 and an Eₘₐₓ of at least 90 against the M₄ receptor in the assay of Example A herein or an assay substantially similar thereto.

For example, the compound described herein may be a muscarinic M₄ receptor agonist having a pEC₅₀ in the range from 7.5 to 8.7.

For example, the compound described herein may be a muscarinic M₄ receptor agonist having a pEC₅₀ in the range from 6.5 to 7.5.

For example, the compound described herein may have an Eₘₐₓ of at least 75 against the M₄ receptor.

For example, the compound described herein may have an Eₘₐₓ of at least 95 against the M₄ receptor.

For example, the compound described herein may be selective for the M₁ and M₄ receptor compared to the muscarinic M₂ and M₃ receptors.

For example, the compound described herein may be selective for the M₁ receptor compared to the muscarinic M₂ and M₃ receptors.

For example, the compound described herein may be selective for the M₁ receptor compared to the muscarinic M₂, M₃ and M₄ receptors.

For example, the compound described herein may have a pEC₅₀ of less than 5 and an Eₘₐₓ of less than 50 against the muscarinic M₂ and M₃ receptor subtypes.

For example, the compound described herein may have a pEC₅₀ of less than 4.5 and/or an Eₘₐₓ of less than 30 against the muscarinic M₂ and M₃ receptor subtypes.

By virtue of their muscarinic M₁ and/or M₄ receptor agonist activity, compounds of the invention can be used in the treatment of Alzheimer's disease, schizophrenia and other psychotic disorders, cognitive disorders and other diseases mediated by the muscarinic M₁ and/or M₄ receptor, and can also be used in the treatment of various types of pain.

Accordingly, in Embodiments 2.18 to 2.39, the invention provides:
2.18 A compound according to any one of Embodiments 1.1 to 1.65 for use in the treatment of a cognitive disorder or psychotic disorder.

2.19 A compound for use according to Embodiment 2.18 wherein the cognitive disorder or psychotic disorder comprises, arises from or is associated with a condition selected from cognitive impairment, Mild Cognitive Impairment (including mild cognitive impairment due to Alzhimer's disease and/or prodromal Alzheimer's disease), frontotemporal dementia, vascular dementia, dementia with Lewy bodies, presenile dementia, senile dementia, Friederich's ataxia, Down's syndrome, Huntington's chorea, hyperkinesia, mania, Tourette's syndrome, Alzheimer's disease (including prodromal Alzheimer's disease and stages 1, 2, and 3 early Alzheimer's disease as defined by the US Food and Drug Admistration's "Early Alzheimer's disease: Developing Drugs for Treatment" available at fda.gov/downloads/Drugs/GuidanceComplianceRegulatorylnformation/Guidances/UCM5967 28.pdf), progressive supranuclear palsy, impairment of cognitive functions including attention, orientation, learning disorders, memory (i.e. memory disorders, amnesia, amnesic disorders, transient global amnesia syndrome and age-associated memory impairment) and language function; cognitive impairment as a result of stroke, Huntington's disease, Pick disease, AIDS-related dementia or other dementia states such as multi-infarct dementia, alcoholic dementia, hypotiroidism-related dementia, and dementia associated to other degenerative disorders such as cerebellar atrophy and amyotropic lateral sclerosis; other acute or sub-acute conditions that may cause cognitive decline such as delirium or depression (pseudodementia states) trauma, head trauma, age related cognitive decline, stroke, neurodegeneration, drug-induced states, neurotoxic agents, age related cognitive impairment, autism related cognitive impairment, Down's syndrome, cognitive deficit related to psychosis, and post-electroconvulsive treatment related cognitive disorders; cognitive disorders due to drug abuse or drug withdrawal including nicotine, cannabis, amphetamine, cocaine, Attention Deficit Hyperactivity Disorder (ADHD) and dyskinetic disorders such as Parkinson's disease, neuroleptic-induced parkinsonism, and tardive dyskinesias, schizophrenia, schizophreniform diseases, psychotic depression, mania, acute mania, paranoid, hallucinogenic and delusional disorders, personality disorders, obsessive compulsive disorders, schizotypal disorders, delusional disorders, psychosis due to malignancy, metabolic disorder, endocrine disease or narcolepsy, psychosis due to drug abuse or drug withdrawal, bipolar disorders and schizo-affective disorder.

2.20 A compound according to any one of Embodiments 1.1 to 1.65 for use in the treatment of Alzheimer's disease.

2.21 A compound according to any one of Embodiments 1.1 to 1.65 for use in the treatment of Schizophrenia.

2.26 A compound according to any one of Embodiments 1.1 to 1.65 for use in the treatment of a cognitive disorder.

2.27 The compound for use according to Embodiment 2.26 wherein the cognitive disorder comprises, arises from or is associated with a condition as defined in Embodiment 2.19.

2.28 The compound for use according to Embodiment 2.27 wherein the cognitive disorder arises from or is associated with Alzheimer's disease.

2.29 The compound for use according to Embodiment 2.28 wherein the cognitive disorder is Schizophrenia.

2.30 A compound according to any one of Embodiments 1.1 to 1.65 for use in the treatment or lessening the severity of acute, chronic, neuropathic, or inflammatory pain, arthritis, migraine, cluster headaches, trigeminal neuralgia, herpetic neuralgia, general neuralgias, visceral pain, osteoarthritis pain, postherpetic neuralgia, diabetic neuropathy, radicular pain, sciatica, back pain, head or neck pain, severe or intractable pain, nociceptive pain, breakthrough pain, postsurgical pain, or cancer pain.

2.32 A compound according to any one of Embodiments 1.1 to 1.65 for use in the treatment of peripheral disorders such as reduction of intra ocular pressure in Glaucoma and treatment of dry eyes and dry mouth including Sjogren's Syndrome.

2.35 A compound according to any one of Embodiments 1.1 to 1.65 for use in the treatment of skin lesions for example due to pemphigus vulgaris, dermatitis herpetiformis, pemphigoid and other blistering skin conditions.

2.36 A compound according to any one of Embodiments 1.1 to 1.65 for use in treating, preventing, ameliorating or reversing conditions associated with altered gastro-intestinal function and motility such as functional dyspepsia, irritable bowel syndrome, gastroesophageal acid reflux (GER) and esophageal dysmotility, symptoms of gastroparesis and chronic diarrhea.

2.37 A compound according to any one of Embodiments 1.1 to 1.65 for use in in the treatment of olfactory dysfunction such as Bosma-Henkin-Christiansen syndrome, chemical poisoning (e.g. selenium and silver), hypopituitarism, Kallmann Syndrome, skull fractures, tumour therapy and underactive thyroid gland.

### The Invention

The present invention provides compounds having activity as muscarinic M₁ and/or M₄ receptor agonists. More particularly, the invention provides compounds that exhibit selectivity for the M₁ and/or M₄ receptor relative to the M₂ and M₃ receptor subtypes.

Accordingly, in a first embodiment (Embodiment 1.1), the invention provides a compound of the formula (1): 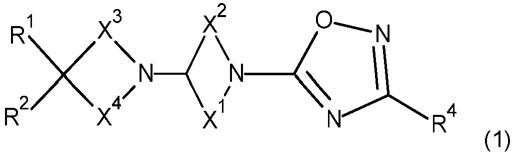 or a salt thereof, wherein:
X¹ and X² are saturated hydrocarbon groups which together contain a total of five to nine carbon atoms and zero or one oxygen atoms and which link together such that the moiety: 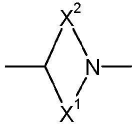
forms a monocyclic or bicyclic ring system;
X³ and X⁴ are saturated hydrocarbon groups which together contain a total of three to six carbon atoms and which link together such that the moiety: 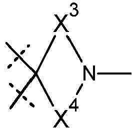
forms a monocyclic or bicyclic ring system;
R¹ is selected from NR⁵R⁶; CONR⁵R⁶; COOR⁷; an optionally substituted 5- or 6-membered ring containing 0, 1, 2 or 3 heteroatoms selected from O, N and S and oxidized forms thereof, wherein the optional substituent of the 5- or 6-membered ring is selected from C₁-C₃ alkyl, C₁-C₃ alkoxy, CONR⁵R⁶, halogen, cyano, oxo, hydroxyl, amino or pyrazole optionally substituted with C₁-C₃ alkyl; or R¹ is linked to R² to form an optionally substituted monocyclic or bicyclic ring containing 0, 1, 2 or 3 heteroatoms selected from O, N and S and oxidized forms thereof, wherein the optional substituents of the monocyclic or bicyclic ring are selected from C₁-C₆ alkyl, C₁-C₃ alkoxy, halogen, cyano, oxo, hydroxyl, amino or a group - (CH₂)ₙ-aryl wherein n is 0-3;
R² is selected from hydrogen; fluorine; cyano; hydroxy; amino; and a C₁₋₃ hydrocarbon group which is optionally substituted with one to six fluorine atoms and wherein one of the carbon atoms of the hydrocarbon group may optionally be replaced by a heteroatom selected from O, N and S and oxidized forms thereof, or R² is linked to R¹ to form an optionally substituted monocyclic or bicyclic ring containing 0, 1, 2 or 3 heteroatoms selected from O, N and S and oxidized forms thereof, wherein the optional substituents of the monocyclic or bicyclic ring are selected from C₁-C₆ alkyl, C₁-C₃ alkoxy, halogen, cyano, oxo, hydroxyl, amino or a group -(CH₂)ₙ-aryl wherein n is 0-3;
R⁴ is H, halo, OH, CN, a C₁₋₆ alkyl group or a C₃₋₆ cycloalkyl group, wherein the alkyl and cycloalkyl groups are optionally substituted with one or more fluorine atoms, and wherein any one atom of the alkyl or cycloalkyl group may be optionally replaced by an O heteroatom;
R⁵ is selected from hydrogen, COCH₃, a non-aromatic C₁₋₁₀ hydrocarbon group optionally substituted with one or more fluorine atoms wherein any one atom of the C₁₋₁₀ hydrocarbon group may be optionally replaced by a heteroatom selected from O, N and S, a group - (CH₂)ₙ-aryl, wherein n is 0-3, or R⁶ can be joined together with R⁶ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino;
R⁶ is selected from hydrogen, a non-aromatic C₁₋₁₀ hydrocarbon group optionally substituted with one or more fluorine atoms wherein any one atom of the C₁₋₁₀ hydrocarbon group may be optionally replaced by a heteroatom selected from O, N and S, a group -(CH₂)ₙ-aryl, wherein n is 0-3, or R⁶ can be joined together with R⁶ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino; and
R⁷ is a non-aromatic C₁₋₆ hydrocarbon group optionally substituted with one or more fluorine atoms.

In a further embodiment (Embodiment 1.2), the invention provides a compound of the formula (1a) 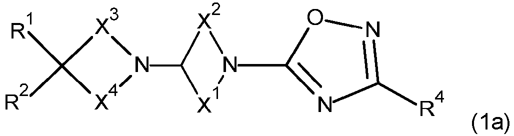 or a salt thereof, wherein:
X¹ and X² are saturated hydrocarbon groups which together contain a total of five to nine carbon atoms and zero or one oxygen atoms and which link together such that the moiety: 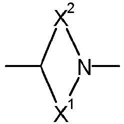
forms a monocyclic or bicyclic ring system;
X³ and X⁴ are saturated hydrocarbon groups which together contain a total of three to five carbon atoms and which link together such that the moiety: 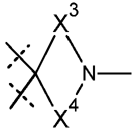
forms a monocyclic or bicyclic ring system;
R¹ is selected from NR⁵R⁶; CONR⁵R⁶; COOR⁷; an optionally substituted 5- or 6-membered ring containing 0, 1, 2 or 3 heteroatoms selected from O, N and S and oxidized forms thereof; or R¹ is linked to R² to form an optionally substituted monocyclic or bicyclic ring containing 0, 1, 2 or 3 heteroatoms selected from O, N and S and oxidized forms thereof; R² is selected from hydrogen; fluorine; cyano; hydroxy; amino; and a C₁₋₃ hydrocarbon group which is optionally substituted with one to six fluorine atoms and wherein one of the carbon atoms of the hydrocarbon group may optionally be replaced by a heteroatom selected from O, N and S and oxidized forms thereof, or R² is linked to R¹ to form an optionally substituted monocyclic or bicyclic ring containing 0, 1, 2 or 3 heteroatoms selected from O, N and S and oxidized forms thereof;
R⁴ is H or a C₁₋₃ alkyl group;
R⁵ is selected from hydrogen, COCH₃, a non-aromatic C₁₋₆ hydrocarbon group optionally substituted with one or more fluorine atoms, or R⁵ can be joined together with R⁶ to form an optionally substituted monocyclic or bicyclic ring containing 0, 1, 2 or 3 heteroatoms selected from O, N and S and oxidized forms thereof;
R⁶ is selected from hydrogen, a non-aromatic C₁₋₆ hydrocarbon group optionally substituted with one or more fluorine atoms,or R⁶ can be joined together with R⁵ to form an optionally substituted monocyclic or bicyclic ring containing 0, 1, 2 or 3 heteroatoms selected from O, N and S and oxidized forms thereof; and
R¹ is a non-aromatic C₁₋₆ hydrocarbon group optionally substituted with one or more fluorine atoms.

Particular and preferred compounds of the formula (1) are as defined in the following Embodiments 1.3 to 1.42:
1.3 A compound according to Embodiment 1.1 or 1.2 wherein R¹ is an optionally substituted 5- or 6-membered ring containing 0, 1, 2 or 3 heteroatoms selected from O, N and S and oxidized forms thereof.

1.4 A compound according to Embodiment 1.3 wherein the optionally substituted ring is phenyl, pyrazolyl, pyrrolidinyl or pyrrolidinonyl.

In the compound according to any of Embodiments 1.3 or 1.4 the optional substituent of the 5- or 6-membered ring is selected from C₁-C₃ alkyl, C₁-C₃ alkoxy, CONR⁵R⁶, halogen, cyano, oxo, hydroxyl or amino; or the optional substituent of the 5- or 6-membered ring is an optionally substituted pyrazole ring; wherein the optional substituent of the pyrazole ring is C₁-C₃ alkyl.

1.8 A compound according to Embodiment 1.1 or 1.2 wherein R¹ is selected from NR⁵R⁶; CONR⁵R⁶; COOR⁷.

1.9 A compound according to Embodiment 1.1 or 1.2 wherein R¹ is selected from:

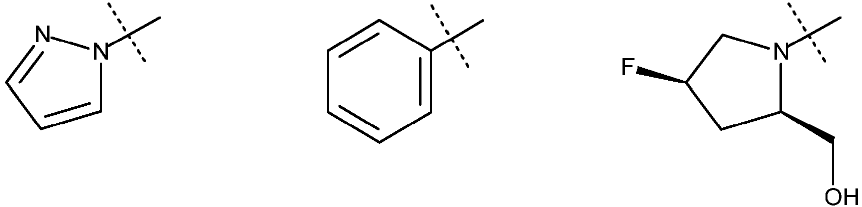

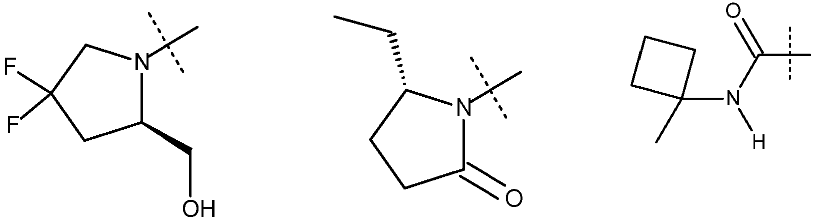

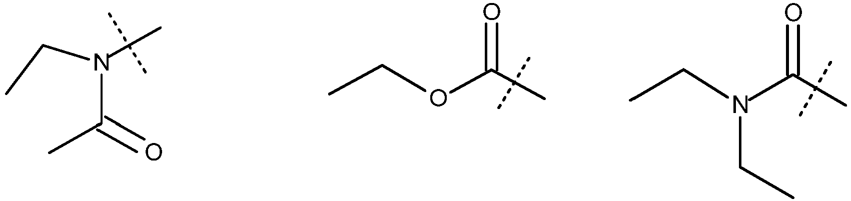

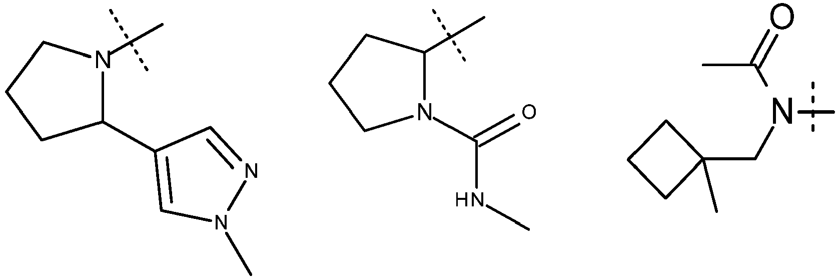

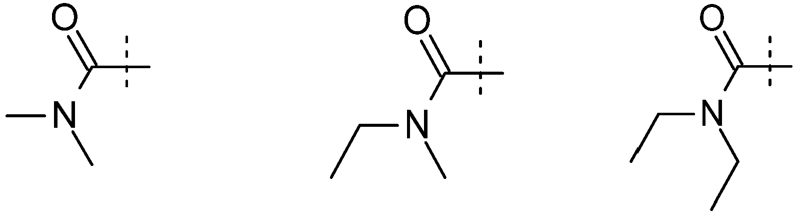

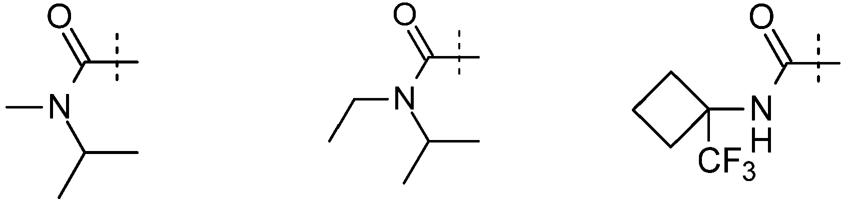

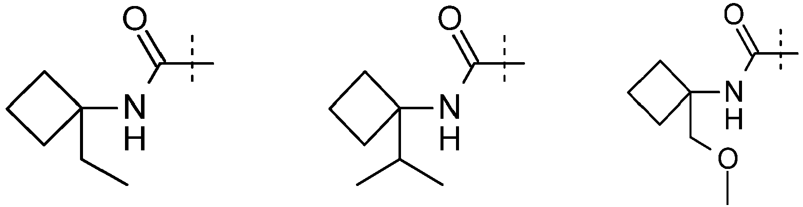

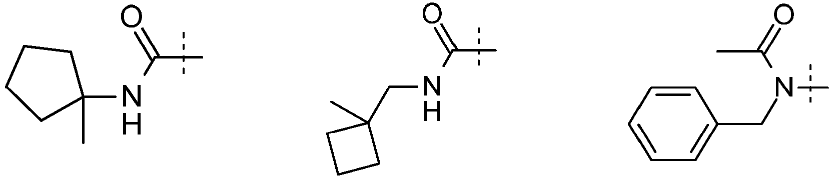

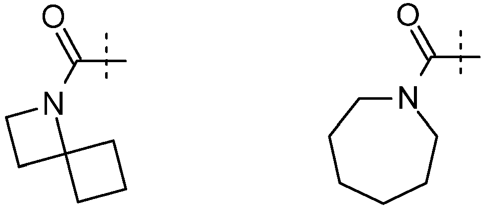

1.10 A compound according to Embodiment 1.1 or 1.2 wherein R¹ is linked to R² to form an optionally substituted monocyclic or bicyclic ring containing 0, 1, 2 or 3 heteroatoms selected from O, N and S and oxidized forms thereof.

1.11 A compound according to Embodiment 1.10 wherein R¹ and R² are linked to form an optionally substituted monocyclic ring selected from pyrrolidinonyl and oxazolidinonyl.

1.12 A compound according to Embodiment 1.10 wherein R¹ and R² are linked to form an optionally substituted indolinonyl ring.

1.13 A compound according to Embodiment 1.10 wherein the monocyclic or bicyclic ring is selected from:

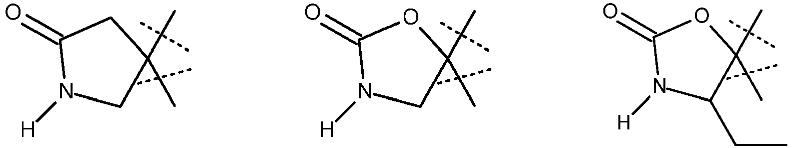

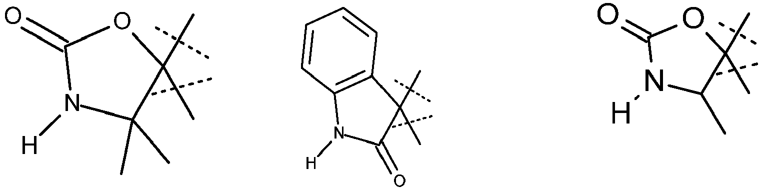

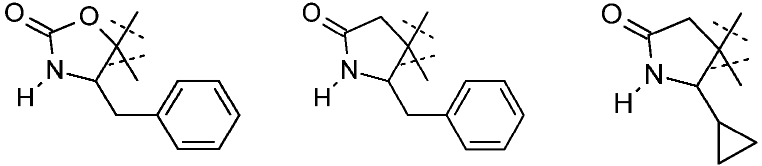

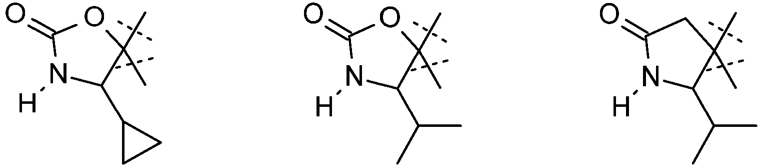

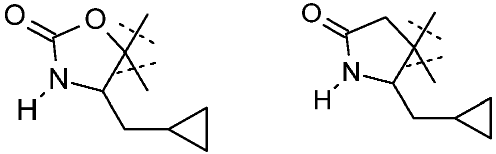

In the compound according to any of Embodiments 1.10 to 1.13 the optional substituents of the monocyclic or bicyclic ring are selected from C₁-C₆ alkyl, C₁-C₃ alkoxy, halogen, cyano, oxo, hydroxyl, amino or a group -(CH₂)ₙ-aryl wherein n is 0-3.

1.15 A compound according to any of Embodiments 1.10 to 1.14 wherein the optional substituents of the monocyclic or bicyclic ring are methyl or ethyl.

In the compound according to any of Embodiments 1.5 or 1.8 R⁵ is selected from hydrogen, COCH₃, a non-aromatic C₁₋₁₀ hydrocarbon group optionally substituted with one or more fluorine atoms, wherein any one atom of the C₁₋₁₀ hydrocarbon group may be optionally replaced by a heteroatom selected from O, N and S, a group -(CH₂)ₙ-aryl, wherein n is 0-3, or R⁵ can be joined together with R⁶ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino.

In the compound according to any of Embodiments 1.5 or 1.8 R⁶ is selected from hydrogen, a non-aromatic C₁₋₁₀ hydrocarbon group optionally substituted with one or more fluorine atoms, wherein any one atom of the C₁₋₁₀ hydrocarbon group may be optionally replaced by a heteroatom selected from O, N and S, a group -(CH₂)ₙ-aryl, wherein n is 0-3, or R⁶ can be joined together with R⁵ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino.

1.18 A compound according to any of Embodiments 1.1 to 1.17 wherein R⁵ and R⁶ are independently selected from hydrogen, COCH₃ and C₁₋₆ alkyl.

1.19 A compound according to Embodiment 1.18 wherein R⁵ and R⁶ are independently selected from methyl, ethyl or methylcyclobutyl.

1.20 A compound according to any of Embodiments 1.1 to 1.17 wherein R⁵ is COCH₃.

1.21 A compound according to Embodiment 1.1 wherein R⁷ is C₁-C₃ alkyl.

1.22 A compound according to Embodiment 1.1 wherein R⁷ is ethyl.

1.23 A compound according to any of Embodiments 1.1 to 1.22 wherein R² is selected from hydrogen; fluorine; cyano; hydroxy; amino; and a C₁₋₃ hydrocarbon group which is optionally substituted with one to six fluorine atoms and wherein one of the carbon atoms of the hydrocarbon group may optionally be replaced by a heteroatom selected from O, N and S and oxidized forms thereof, or R² is linked to R¹ to form an optionally substituted monocyclic or bicyclic ring containing 0, 1, 2 or 3 heteroatoms selected from O, N and S and oxidized forms thereof.

1.24 A compound according to any of Embodiments 1.1 to 1.23 wherein R² is hydrogen or cyano.

1.25 A compound according to any of Embodiments 1.1 to 1.24 wherein R² is hydrogen.

1.26 A compound according to any of Embodiments 1.1 to 1.24 wherein R² is cyano.

1.27 A compound according to any of Embodiments 1.1 to 1.23 wherein R² is linked to R¹ to form an optionally substituted spirocyclic group.

1.28 A compound according to Embodiment 1.1 wherein R⁴ is hydrogen or a C₁₋₆ alkyl group or a C₃₋₆ cycloalkyl group, wherein the alkyl and cycloalkyl groups are optionally substituted with one or more fluorine atoms, and wherein any one atom of the alkyl or cycloalkyl group may be optionally replaced by an O heteroatom.

1.29 A compound according to Embodiment 1.1 wherein R⁴ is hydrogen.

1.30 A compound according to Embodiment 1.1 wherein R⁴ is methyl.

1.31 A compound according to Embodiment 1.1 wherein R⁴ is ethyl.

1.32 A compound according to Embodiment 1.1 wherein R⁴ is halo, OH or CN.

1.33 A compound according to Embodiment 1.1 wherein R⁴ is methyl, trifluoromethyl, ethyl, isopropyl or cyclopropyl.

1.34 A compound according to any one of Embodiments 1.1 to 1.33 wherein the ring system formed by X¹ and X² is selected from 8-azabicyclo[3.2.1]octane, 9-azabicyclo[3.3.1]nonane, 6-azabicyclo[3.2.1]octane, 7-azaspiro[3.4]octane, 2-azaspiro[3.4]octane, 6-azaspiro[3.3]heptane, 3-azabicyclo[3.1.1]heptane, 3-oxa-9-azabicyclo[3.3.1]nonane, 2-azabicyclo[2.2.2]octane, piperidine and azepane.

1.35 A compound according to any one of Embodiments 1.1 to 1.33 wherein the ring system formed by X¹ and X² is selected from:

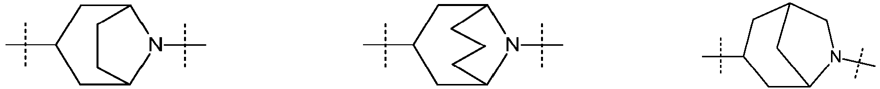

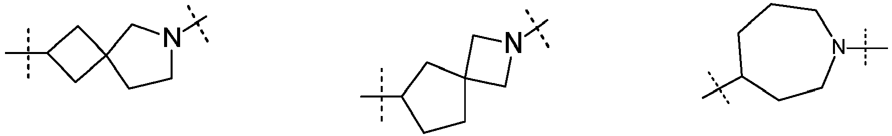

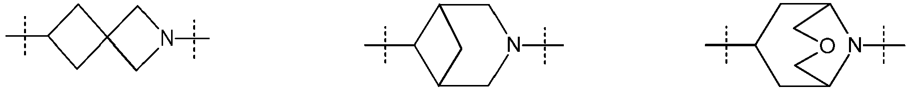

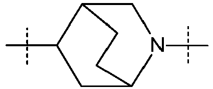

1.36 A compound according to Embodiment 1.1 wherein the ring system formed by X³ and X⁴ is selected from piperidine, azepane and 3-azabicyclo[3.1.0]hexane.

1.37 A compound according to Embodiment 1.1 wherein the ring system formed by X³ and X⁴ is selected from:

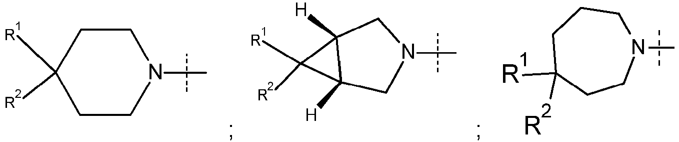

1.38 A compound according to Embodiment 1.36 wherein the ring system formed by X³ and X⁴ is piperidine.

1.39 A compound according to any of Embodiments 1.1 to 1.38 of the formula (2): 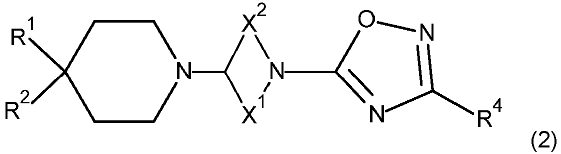 or a salt thereof wherein:
X¹, X², R¹ R² and R⁴ are as defined in any one of Embodiments 1.1 to 1.38.

1.40 A compound according to any of Embodiments 1.1 to 1.38 of the formula (3): 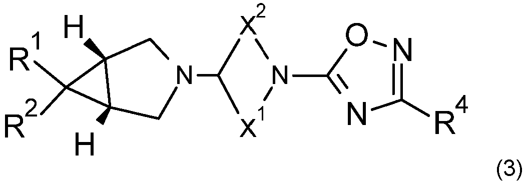 or a salt thereof wherein:
X¹, X², R¹ R² and R⁴ are as defined in any one of Embodiments 1.1 to 1.38.

1.41 A compound according to any of Embodiments 1.1 to 1.38 of the formula (4): 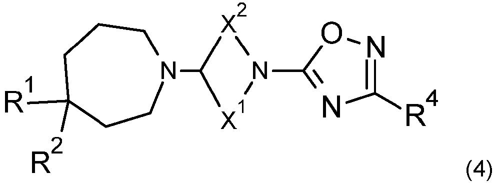 or a salt thereof wherein:
X¹, X², R¹ R² and R⁴ are as defined in any one of Embodiments 1.1 to 1.38.

1.42 A compound according to any of Embodiments 1.1 to 1.41 selected from;
2-(3-Methyl-1,2,4-oxadiazol-5-yl)-6-[4-(1H-pyrazol-1-yl)piperidin-1-yl]-2-azaspiro[3.3]heptane;
6-(3-Methyl-1,2,4-oxadiazol-5-yl)-2-[4-(1H-pyrazol-1-yl)piperidin-1-yl]-6-azaspiro[3.4]octane;
1-[6-(3-Methyl-1,2,4-oxadiazol-5-yl)-6-azaspiro[3.4]oct-2-yl]-4-phenylpiperidine-4-carbonitrile;
Ethyl (1R,5S,6r)-3-[6-(3-methyl-1,2,4-oxadiazol-5-yl)-6-azaspiro[3.4]oct-2-yl]-3-azabicyclo[3.1.0]hexane-6-carboxylate;
2-(3-Methyl-1,2,4-oxadiazol-5-yl)-6-[4-(1H-pyrazol-1-yl)piperidin-1-yl]-2-azaspiro[3.4]octane;
3-(3-Methyl-1,2,4-oxadiazol-5-yl)-6-[4-(1H-pyrazol-1-yl)piperidin-1-yl]-3-azabicyclo[3.1.1]heptane;
8-(3-Methyl-1,2,4-oxadiazol-5-yl)-3-[4-(1H-pyrazol-1-yl)piperidin-1-yl]-8-azabicyclo[3.2.1]octane;
8-[8-(3-Methyl-1,2,4-oxadiazol-5-yl)-8-azabicyclo[3.2.1]oct-3-yl]-2,8-diazaspiro[4.5]decan-3-one;
8-[8-(3-Ethyl-1,2,4-oxadiazol-5-yl)-8-azabicyclo[3.2.1]oct-3-yl]-2,8-diazaspiro[4.5]decan-3-one;
8-[8-(3-Methyl-1,2,4-oxadiazol-5-yl)-8-azabicyclo[3.2.1]octan-3-yl]-1-oxa-3,8-diazaspiro[4.5]decan-2-one;
N-(1-Methylcyclobutyl)-1-[8-(3-methyl-1,2,4-oxadiazol-5-yl)-8-azabicyclo[3.2.1]oct-3-yl]piperidine-4-carboxamide;
2-(3-Methyl-1,2,4-oxadiazol-5-yl)-5-[4-(1H-pyrazol-1-yl)piperidin-1-yl]-2-azabicyclo[2.2.2]octane;
9-(3-Methyl-1,2,4-oxadiazol-5-yl)-3-[4-(1H-pyrazol-1-yl)piperidin-1-yl]-9-azabicyclo[3.3.1]nonane;
8-[9-(3-Methyl-1,2,4-oxadiazol-5-yl)-9-azabicyclo[3.3.1]non-3-yl]-2,8-diazaspiro[4.5]decan-3-one;
8-[9-(3-Methyl-1,2,4-oxadiazol-5-yl)-9-azabicyclo[3.3.1]non-3-yl]-1-oxa-3,8-diazaspiro[4.5]decan-2-one;
N-(1-Methylcyclobutyl)-1-[9-(3-methyl-1,2,4-oxadiazol-5-yl)-9-azabicyclo[3.3.1]non-3-yl]piperidine-4-carboxamide;
9-(3-Methyl-1,2,4-oxadiazol-5-yl)-7-[4-(1H-pyrazol-1-yl)piperidin-1-yl]-3-oxa-9-azabicyclo[3.3.1]nonane;
(1R,5S,6r)-N,N-Diethyl-3-[9-(3-methyl-1,2,4-oxadiazol-5-yl)-3-oxa-9-azabicyclo[3.3.1]non-7-yl]-3-azabicyclo[3.1.0]hexane-6-carboxamide;
1-(3-Methyl-1,2,4-oxadiazol-5-yl)-4-[4-(1H-pyrazol-1-yl)piperidin-1-yl]azepane;
8-[1-(3-Methyl-1,2,4-oxadiazol-5-yl)azepan-4-yl]-2,8-diazaspiro[4.5]decan-3-one;
8-[1-(3-Methyl-1,2,4-oxadiazol-5-yl)azepan-4-yl]-1-oxa-3,8-diazaspiro[4.5]decan-2-one;
4-Ethyl-8-[1-(3-methyl-1,2,4-oxadiazol-5-yl)azepan-4-yl]-1-oxa-3, 8-diazaspiro[4.5]decan-2-one;
4,4-Dimethyl-8-[1-(3-methyl-1,2,4-oxadiazol-5-yl)azepan-4-yl]-1-oxa-3,8-diazaspiro[4.5]decan-2-one;
1'-[1-(3-Methyl-1,2,4-oxadiazol-5-yl)azepan-4-yl]spiro[indole-3,4'-piperidin]-2(1H)-one;
[(2R,4R)-4-Fluoro-1-{1-[1-(3-methyl-1,2,4-oxadiazol-5-yl)azepan-4-yl]piperidin-4-yl} pyrrolidin-2-yl]methanol;
[(2R)-4,4-Difluoro-1-{1-[1-(3-methyl-1,2,4-oxadiazol-5-yl)azepan-4-yl]piperidin-4-yl}pyrrolidin-2-yl]methanol;
(5S)-5- Ethyl-1-{1-[1-(3-methyl-1 ,2,4-oxadiazol-5-yl)azepan-4-yl]piperidin-4-yl}pyrrolidin-2-one;
1-(3-Methyl-1,2,4-oxadiazol-5-yl)-4-{4-[2-(1-methyl-1H-pyrazol-4-yl)pyrrolidin-1-yl]piperidin-1-yl}azepane;
N-Ethyl-N-{1-[1-(3-methyl-1,2,4-oxadiazol-5-yl)azepan-4-yl]piperidin-4-yl}acetamide;
(2S)-N-Methyl-2-{1-[1-(3-methyl-1,2,4-oxadiazol-5-yl)azepan-4-yl]piperidin-4-yl}pyrrolidine-1-carboxamide;
6-(3-Methyl-1,2,4-oxadiazol-5-yl)-3-[4-(1H-pyrazol-1-yl)piperidin-1-yl]-6-azabicyclo[3.2.1]octane;
8-[6-(3-Methyl-1,2,4-oxadiazol-5-yl)-6-azabicyclo[3.2.1]oct-3-yl]-2,8-diazaspiro[4.5]decan-3-one;
8-[6-(3-Methyl-1,2,4-oxadiazol-5-yl)-6-azabicyclo[3.2.1]oct-3-yl]-1-oxa-3,8-diazaspiro[4.5]decan-2-one;
8-(2-(3-methyl-1,2,4-oxadiazol-5-yl)-2-azabicyclo[2.2.2]octan-5-yl)-2,8-diazaspiro[4.5]decan-3-one;
8-(2-(3-methyl-1,2,4-oxadiazol-5-yl)-2-azabicyclo[2.2.2]octan-5-yl)-1-oxa-3,8-diazaspiro[4.5]decan-2-one;
8-(8-(3-(trifluoromethyl)-1,2,4-oxadiazol-5-yl)-8-azabicyclo[3.2.1]octan-3-yl)-2,8-diazaspiro[4.5]decan-3-one;
8-(8-(3-(trifluoromethyl)-1,2,4-oxadiazol-5-yl)-8-azabicyclo[3.2.1]octan-3-yl)-1-oxa-3,8-diazaspiro[4.5]decan-2-one;
(1R,5S,6r)-3-(2-(3-methyl-1,2,4-oxadiazol-5-yl)-2-azaspiro[3.4]octan-6-yl)-N-(1-methylcyclobutyl)-3-azabicyclo[3.1.0]hexane-6-carboxamide;
1-(8-(3-methyl-1,2,4-oxadiazol-5-yl)-8-azabicyclo[3.2.1]octan-3-yl)-N-(1-(trifluoromethyl)cyclobutyl)piperidine-4-carboxamide;
N-(1-ethylcyclobutyl)-1-(8-(3-methyl-1,2,4-oxadiazol-5-yl)-8-azabicyclo[3.2.1]octan-3-yl)piperidine-4-carboxamide;
N-(1-isopropylcyclobutyl)-1-(8-(3-methyl-1,2,4-oxadiazol-5-yl)-8-azabicyclo[3.2.1]octan-3-yl)piperidine-4-carboxamide;
1-(8-(3-methyl-1,2,4-oxadiazol-5-yl)-8-azabicyclo[3.2.1]octan-3-yl)-N-(1-methylcyclopentyl)piperidine-4-carboxamide;
1-(8-(3-methyl-1,2,4-oxadiazol-5-yl)-8-azabicyclo[3.2.1]octan-3-yl)-N-((1-methylcyclobutyl)methyl)piperidine-4-carboxamide;
(1R,5S,6r)-3-(8-(3-methyl-1,2,4-oxadiazol-5-yl)-8-azabicyclo[3.2.1]octan-3-yl)-N-(1-methylcyclobutyl)-3-azabicyclo[3.1.0]hexane-6-carboxamide;
N-(1-methylcyclobutyl)-1-(8-(3-(trifluoromethyl)-1,2,4-oxadiazol-5-yl)-8-azabicyclo[3.2.1]octan-3-yl)piperidine-4-carboxamide;
1-(2-(3-methyl-1,2,4-oxadiazol-5-yl)-2-azabicyclo[2.2.2]octan-5-yl)-N-(1-methylcyclobutyl)piperidine-4-carboxamide;
1-(2-(3-methyl-1,2,4-oxadiazol-5-yl)-2-azabicyclo[2.2.2]octan-5-yl)-N-((1-methylcyclobutyl)methyl)piperidine-4-carboxamide;
N-(1-(9-(3-methyl-1,2,4-oxadiazol-5-yl)-3-oxa-9-azabicyclo[3.3.1]nonan-7-yl)piperidin-4-yl)-N-((1-methylcyclobutyl)methyl)acetamide;
N-(1-(8-(3-methyl-1,2,4-oxadiazol-5-yl)-8-azabicyclo[3.2.1]octan-3-yl)piperidin-4-yl)-N-((1-methylcyclobutyl)methyl)acetamide;
N-(1-(2-(3-methyl-1,2,4-oxadiazol-5-yl)-2-azabicyclo[2.2.2]octan-5-yl)piperidin-4-yl)-N-((1-methylcyclobutyl)methyl)acetamide;
N-benzyl-N-(1-(9-(3-methyl-1,2,4-oxadiazol-5-yl)-3-oxa-9-azabicyclo[3.3.1]nonan-7-yl)piperidin-4-yl)acetamide;
((1R,5S,6r)-3-(9-(3-methyl-1,2,4-oxadiazol-5-yl)-3-oxa-9-azabicyclo[3.3.1]nonan-7-yl)-3-azabicyclo[3.1.0]hexan-6-yl)(1-azaspiro[3.3]heptan-1-yl)methanone;
(1R,5S,6r)-N-isopropyl-N-methyl-3-(9-(3-methyl-1,2,4-oxadiazol-5-yl)-3-oxa-9-azabicyclo[3.3.1]nonan-7-yl)-3-azabicyclo[3.1.0]hexane-6-carboxamide;
(1R,5S,6r)-N,N-diethyl-3-(9-(3-(trifluoromethyl)-1,2,4-oxadiazol-5-yl)-3-oxa-9-azabicyclo[3.3.1]nonan-7-yl)-3-azabicyclo[3.1.0]hexane-6-carboxamide;
(1R,5S,6r)-N-ethyl-N-methyl-3-(8-(3-methyl-1,2,4-oxadiazol-5-yl)-8-azabicyclo[3.2.1]octan-3-yl)-3-azabicyclo[3.1.0]hexane-6-carboxamide;
(1R,5S,6r)-N,N-diethyl-3-(8-(3-methyl-1,2,4-oxadiazol-5-yl)-8-azabicyclo[3.2.1]octan-3-yl)-3-azabicyclo[3.1.0]hexane-6-carboxamide;
(1 R, 5S, 6r)- N, N-diethyl-3-(2-(3-methyl-1 ,2 ,4-oxadiazol-5-yl)-2-azaspiro[3.4]octan-6-yl)-3-azabicyclo[3.1.0]hexane-6-carboxamide;
(1R,5S,6r)-N-ethyl-N-methyl-3-(2-(3-methyl-1,2,4-oxadiazol-5-yl)-2-azaspiro[3.4]octan-6-yl)-3-azabicyclo[3.1.0]hexane-6-carboxamide;
N,N-diethyl-1-(2-(3-methyl-1,2,4-oxadiazol-5-yl)-2-azaspiro[3.4]octan-6-yl)piperidine-4-carboxamide;
8-(8-(3-methyl-1,2,4-oxadiazol-5-yl)-8-azabicyclo[3.2.1]octan-3-yl)-2,8-diazaspiro[4.6]undecan-3-one;
8-(8-(3-methyl-1,2,4-oxadiazol-5-yl)-8-azabicyclo[3.2.1]octan-3-yl)-1-oxa-3,8-diazaspiro[4.6]undecan-2-one;
8-(2-(3-methyl-1,2,4-oxadiazol-5-yl)-2-azabicyclo[2.2.2]octan-5-yl)-2,8-diazaspiro[4.6]undecan-3-one;
8-(2-(3-methyl-1,2,4-oxadiazol-5-yl)-2-azabicyclo[2.2.2]octan-5-yl)-1-oxa-3,8-diazaspiro[4.6]undecan-2-one;
8-(6-(3-methyl-1,2,4-oxadiazol-5-yl)-6-azabicyclo[3.2.1]octan-3-yl)-2,8-diazaspiro[4.6]undecan-3-one;
8-(6-(3-methyl-1,2,4-oxadiazol-5-yl)-6-azabicyclo[3.2.1]octan-3-yl)-1-oxa-3,8-diazaspiro[4.6]undecan-2-one;
((1R,5S,6r)-3-(6-(3-methyl-1,2,4-oxadiazol-5-yl)-6-azabicyclo[3.2.1]octan-3-yl)-3-azabicyclo[3.1.0]hexan-6-yl)(1-azaspiro[3.3]heptan-1-yl)methanone;
(1R,5S,6r)-N,N-diethyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-ethyl-N-isopropyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-isopropyl-N-methyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-ethyl-N-methyl-3-(9-(3-methyl-1,2,4-oxadiazol-5-yl)-9-azabicyclo[3.3.1]nonan-3-yl)-3-azabicyclo[3.1.0]hexane-6-carboxamide;
(1R,5S,6r)-N,N-diethyl-3-(9-(3-methyl-1,2,4-oxadiazol-5-yl)-9-azabicyclo[3.3.1]nonan-3-yl)-3-azabicyclo[3.1.0]hexane-6-carboxamide;
8-(1-(3-(trifluoromethyl)-1,2,4-oxadiazol-5-yl)azepan-4-yl)-2,8-diazaspiro[4.5]decan-3-one;
8-(1-(3-(trifluoromethyl)-1,2,4-oxadiazol-5-yl)azepan-4-yl)-2,8-diazaspiro[4.6]undecan-3-one;
8-(1-(3-methyl-1,2,4-oxadiazol-5-yl)azepan-4-yl)-2,8-diazaspiro[4.6]undecan-3-one;
8-(1-(3-(trifluoromethyl)-1,2,4-oxadiazol-5-yl)azepan-4-yl)-1-oxa-3,8-diazaspiro[4.5]decan-2-one;
8-(8-(3-ethyl-1,2,4-oxadiazol-5-yl)-8-azabicyclo[3.2.1]octan-3-yl)-1-oxa-3,8-diazaspiro[4.5]decan-2-one;
8-(2-(3-methyl-1,2,4-oxadiazol-5-yl)-2-azabicyclo[2.2.2]octan-5-yl)-1-oxa-3,8-diazaspiro[4.5]decan-2-one;
8-(6-(3-(trifluoromethyl)-1,2,4-oxadiazol-5-yl)-6-azabicyclo[3.2.1]octan-3-yl)-1-oxa-3,8-diazaspiro[4.5]decan-2-one;
4-methyl-8-(8-(3-methyl-1,2,4-oxadiazol-5-yl)-8-azabicyclo[3.2.1]octan-3-yl)-1-oxa-3,8-diazaspiro[4.5]decan-2-one;
(1R,5S,6r)-3-(6-(3-methyl-1,2,4-oxadiazol-5-yl)-6-azaspiro[3.4]octan-2-yl)-N-(1-methylcyclobutyl)-3-azabicyclo[3.1.0]hexane-6-carboxamide;
N-(1-(6-(3-methyl-1,2,4-oxadiazol-5-yl)-6-azaspiro[3.4]octan-2-yl)piperidin-4-yl)-N-((1-methylcyclobutyl)methyl)acetamide;
N-(1-(9-(3-methyl-1,2,4-oxadiazol-5-yl)-3-oxa-9-azabicyclo[3.3.1]nonan-7-yl)piperidin-4-yl)-N-((1-methylcyclobutyl)methyl)acetamide;
N-(1-(8-(3-methyl-1,2,4-oxadiazol-5-yl)-8-azabicyclo[3.2.1]octan-3-yl)piperidin-4-yl)-N-((1-methylcyclobutyl)methyl)acetamide;
(1R,5S,6r)-N-isopropyl-N-methyl-3-(8-(3-methyl-1,2,4-oxadiazol-5-yl)-8-azabicyclo[3.2.1]octan-3-yl)-3-azabicyclo[3.1.0]hexane-6-carboxamide;
(1R,5S,6r)-N-ethyl-N-methyl-3-(2-(3-methyl-1,2,4-oxadiazol-5-yl)-2-azabicyclo[2.2.2]octan-5-yl)-3-azabicyclo[3.1.0]hexane-6-carboxamide;
(1R,5S,6r)-N-ethyl-N-methyl-3-(9-(3-methyl-1,2,4-oxadiazol-5-yl)-9-azabicyclo[3.3.1]nonan-3-yl)-3-azabicyclo[3.1.0]hexane-6-carboxamide;
8-(1-(3-methyl-1,2,4-oxadiazol-5-yl)azepan-4-yl)-1-oxa-3,8-diazaspiro[4.5]decan-2-one;
8-(1-(3-(trifluoromethyl)-1,2,4-oxadiazol-5-yl)azepan-4-yl)-2,8-diazaspiro[4.5]decan-3-one;
8-(1-(3-(trifluoromethyl)-1,2,4-oxadiazol-5-yl)azepan-4-yl)-1-oxa-3,8-diazaspiro[4.5]decan-2-one;
8-(8-(3-cyclopropyl-1,2,4-oxadiazol-5-yl)-8-azabicyclo[3.2.1]octan-3-yl)-2,8-diazaspiro[4.5]decan-3-one;
8-(8-(3-isopropyl-1,2,4-oxadiazol-5-yl)-8-azabicyclo[3.2.1]octan-3-yl)-2,8-diazaspiro[4.5]decan-3-one;
8-(8-(3-ethyl-1,2,4-oxadiazol-5-yl)-8-azabicyclo[3.2.1]octan-3-yl)-1-oxa-3,8-diazaspiro[4.5]decan-2-one;
4-benzyl-8-(8-(3-methyl-1,2,4-oxadiazol-5-yl)-8-azabicyclo[3.2.1]octan-3-yl)-1-oxa-3,8-diazaspiro[4.5]decan-2-one;
1-isopropyl-8-(8-(3-methyl-1,2,4-oxadiazol-5-yl)-8-azabicyclo[3.2.1]octan-3-yl)-2,8-diazaspiro[4.5]decan-3-one;
4-isopropyl-8-(8-(3-methyl-1,2,4-oxadiazol-5-yl)-8-azabicyclo[3.2.1]octan-3-yl)-1-oxa-3,8-diazaspiro[4.5]decan-2-one;
1-cyclopropyl-8-(8-(3-methyl-1,2,4-oxadiazol-5-yl)-8-azabicyclo[3.2.1]octan-3-yl)-2,8-diazaspiro[4.5]decan-3-one;
8-(8-(3-cyclopropyl-1,2,4-oxadiazol-5-yl)-8-azabicyclo[3.2.1]octan-3-yl)-1-oxa-3,8-diazaspiro[4.5]decan-2-one;
8-(8-(3-isopropyl-1,2,4-oxadiazol-5-yl)-8-azabicyclo[3.2.1]octan-3-yl)-1-oxa-3,8-diazaspiro[4.5]decan-2-one;
N-benzyl-N-(1-(8-(3-methyl-1,2,4-oxadiazol-5-yl)-8-azabicyclo[3.2.1]octan-3-yl)piperidin-4-yl)acetamide;
(1R,5S,6r)-N,N-diethyl-3-(2-(3-methyl-1,2,4-oxadiazol-5-yl)-2-azaspiro[3.3]heptan-6-yl)-3-azabicyclo[3.1.0]hexane-6-carboxamide;
((1R,58,6r)-3-(6-(3-methyl-1,2,4-oxadiazol-5-yl)-6-azaspiro[3.4]octan-2-yl)-3-azabicyclo[3.1.0]hexan-6-yl)(1-azaspiro[3.3]heptan-1-yl)methanone;
azepan-1-yl(1-(8-(3-methyl-1,2,4-oxadiazol-5-yl)-8-azabicyclo[3.2.1]octan-3-yl)piperidin-4-yl)methanone;
1-(cyclopropylmethyl)-8-(8-(3-methyl-1,2,4-oxadiazol-5-yl)-8-azabicyclo[3.2.1]octan-3-yl)-2,8-diazaspiro[4.5]decan-3-one;
4-(cyclopropylmethyl)-8-(8-(3-methyl-1,2,4-oxadiazol-5-yl)-8-azabicyclo[3.2.1]octan-3-yl)-1-oxa-3,8-diazaspiro[4.5]decan-2-one;
4-cyclopropyl-8-(8-(3-methyl-1,2,4-oxadiazol-5-yl)-8-azabicyclo[3.2.1]octan-3-yl)-1-oxa-3,8-diazaspiro[4.5]decan-2-one;
1-benzyl-8-(6-(3-methyl-1,2,4-oxadiazol-5-yl)-6-azaspiro[3.4]octan-2-yl)-2,8-diazaspiro[4.5]decan-3-one;
4-benzyl-8-(2-(3-methyl-1,2,4-oxadiazol-5-yl)-2-azaspiro[3.3]heptan-6-yl)-1-oxa-3,8-diazaspiro[4.5]decan-2-one;
1-benzyl-8-(1-(3-methyl-1,2,4-oxadiazol-5-yl)azepan-4-yl)-2,8-diazaspiro[4.5]decan-3-one;
8-(8-(3-methyl-1,2,4-oxadiazol-5-yl)-8-azabicyclo[3.2.1]octan-3-yl)-2,8-diazaspiro[4.6]undecan-3-one;
1-benzyl-8-(8-(3-methyl-1,2,4-oxadiazol-5-yl)-8-azabicyclo[3.2.1]octan-3-yl)-2,8-diazaspiro[4.5]decan-3-one;
8-(8-(3-methyl-1,2,4-oxadiazol-5-yl)-8-azabicyclo[3.2.1]octan-3-yl)-1-oxa-3,8-diazaspiro[4.6]undecan-2-one;
(1R,5S,6r)-3-(8-(3-methyl-1,2,4-oxadiazol-5-yl)-8-azabicyclo[3.2.1]octan-3-yl)-N-(1-(trifluoromethyl)cyclobutyl)-3-azabicyclo[3.1.0]hexane-6-carboxamide;
(1R,5S,6r)-3-(2-(3-methyl-1,2,4-oxadiazol-5-yl)-2-azabicyclo[2.2.2]octan-5-yl)-N-(1-(trifluoromethyl)cyclobutyl)-3-azabicyclo[3.1.0]hexane-6-carboxamide;
or a salt thereof
1.43 A compound according to Embodiment 1.1 which is as defined in any one of Examples 1-1 to 18-2.

1.44 A compound according to any one of Embodiments 1.1 to 1.43 having a molecular weight of less than 550, for example less than 500, or less than 450.

1.45 A compound according to any one of Embodiments 1.1 to 1.44 which is in the form of a salt.

1.46 A compound according to Embodiment 1.45 wherein the salt is an acid addition salt.

1.47 A compound according to Embodiment 1.45 or Embodiment 1.46 wherein the salt is a pharmaceutically acceptable salt.

2.38 A compound according to any one of Embodiments 1.1 to 1.65 for use in the treatment of addiction.

2.39 A compound according to any one of Embodiments 1.1 to 1.65 for use in the treatment of movement disorders such as Parkinson's disease, ADHD, Huntingdon's disease, tourette's syndrome and other syndromes associated with dopaminergic dysfunction as an underlying pathogenetic factor driving disease.

2.40 A compound according to any one of Embodiments 1.1 to 1.65 for use in the treatment of behavioural and psychological symptoms of dementia (BPSD; including agitation, verbal aggressiveness, physical aggressiveness, depression, anxiety, abnormal motor behaviour, elated mood, irritablility, apathy, disinhibition, impulsivity. delusions, hallucinations, sleep changes, and appetite changes).

### Methods for the Preparation of Compounds of the Formula (1) or Formula (2)

Compounds of the formula (1), formula (1a), formula (2), formula (3) or formula (4), can be prepared in accordance with synthetic methods well known to the skilled person and as described herein.

Accordingly, in another embodiment (Embodiment 3.1), the invention provides a process for the preparation of a compound as defined in any one of Embodiments 1.1 to 1.65, which process comprises:
(A) the reaction of a compound of the formula (10): 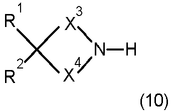 with a compound of the formula (11): 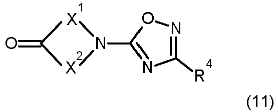 under reductive amination conditions; wherein R¹, R², R⁴, X¹, X², X³ and X⁴ are as defined in any one of Embodiments 1.1 to 1.65; or
(B) when it is required to prepare a compound of formula (1) wherein R¹ comprises CONR⁵R⁶:
   the reaction of a compound of the formula (12): 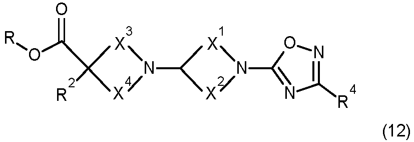
   with an amine of the formula R⁵R⁶NH; wherein R represents a suitable group such as methyl- or ethyl- and R², R⁴, R⁵, R⁶, X¹, X², X³ and X⁴ are as defined in any one of Embodiments 1.1 to 1.65; or
(C) when it is required to prepare a compound of formula (1) wherein R¹ comprises CONR⁵R⁶:
   the reaction of a compound of the formula (13): 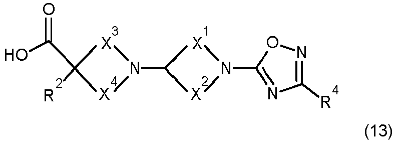
   with an amine of the formula R⁵R⁶NH; wherein R represents a suitable group such as methyl- or ethyl- and R², R⁴, R⁵, R⁶, X¹, X², X³ and X⁴ are as defined in any one of Embodiments 1.1 to 1.65; or
(D) when it is required to prepare a compound of formula (1) wherein R¹ comprises a 5- or 6-membered ring containing a N(C=O)OR or N(C=O)NR₂ group:
   the reaction of an amine of the formula (14): 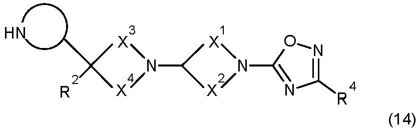
   with a compound of the formula (15) or (16): 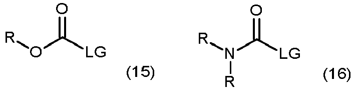
   wherein R², R⁴, X¹, X², X³ and X⁴ are as defined in any one of Embodiments 1.1 to 1.65 and LG represents a suitable leaving group such as Cl, 1-imidazole or 4-nitrophenol; or
   and optionally:
(E) converting one compound of the formula (1) to another compound of the formula (1).

In process variant (A), the ketone (11) is reacted with the amine (10) under reductive amination conditions. The reductive amination reaction is typically carried out at ambient temperature to mild heating (e.g. at a temperature of about 20 °C to about 70 °C) using a borohydride reducing agent such as sodium triacetoxy-borohydride (STAB) in a solvent such as dichloromethane (DCM), dichloroethane (DCE), *N,N*-dimethylformamide (DMF) or methanol (MeOH) containing an acid such as acetic acid (AcOH) or trifluoroacetic acid (TFA), or sodium cyanoborohydride (NaCNBH₃) in combination with zinc chloride (ZnCl₂) in a solvent such as MeOH, or STAB in a solvent such as DCM or DCE containing an acid such as AcOH or TFA in combination with titanium tetraisopropoxide (Ti(OⁱPr)₄). Optionally, the amine (10) may be present in the reaction as an acid salt such as a hydrogen chloride (HCl), hydrogen bromide (HBr) or a TFA salt, optionally in the presence of a tertiary base such as triethylamine (TEA) or *N,N*-diisopropylamine (DIPEA).

Amines of the formula (10) may be sourced commercially or may be prepared by a variety of different methods that exist in the art and that are well known to the skilled person.

Ketones of the formula (11) can be prepared by the sequence of reactions shown in Scheme 1 below. Thus, an *N*-protected amino ketone (17), wherein the protecting group PG represents a suitable protecting group such as BOC or CBZ, can be reacted under reducing conditions suitable to effect formation of *N*-protected amino alcohol (18). Typically, such conditions might be reaction with a borohydride reducing agent such as sodium borohydride (NaBH₄) in a solvent such as MeOH, or lithium borohydide (LiBH₄) in a solvent such as Et₂O or THF, or an aluminium hydride reagent such as lithium aluminium hydride (LAH) in a solvent such as Et₂O or THF, at a temperature of about -20 °C to about 50 °C. Once formed, *N*-protected amino alcohol (18) can be deprotected to give amino alcohol (19). For example, when the protecting group PG is BOC, then suitable conditions to effect its removal might be reaction with an acid such as HCl in a solvent such as 1,4-dioxane or Et₂O, or TFA in a solvent such as DCM. Alternatively, when the protecting group PG is CBZ then suitable conditions to effect its removal might be reaction with H₂ in the presence of a palladium on carbon (Pd/C) catalyst in a solvent such as EtOH at a temperature of about 20 °C to about 80 °C. Once formed, amino alcohol (19) can be reacted with cyanogen bromide in a suitable solvent such as DCM, MeCN, Et₂O or EtOH in the presence of a suitable base such as Et₃N, NaHCO₃, Na₂CO₃, or K₂CO₃, optionally in the presence of water, at a temperature of about 0 °C to about 50 °C, to form cyanamide (20). Once formed, cyanamide (20) can be reacted with a suitable *N*-hydroxy reagent such as *N*-hydroxyamide (22) or the corresponding *N-*hydroxyamidine (23), wherein R⁴ is as defined in any one of Embodiments 1.1 to 1.65, in a suitable solvent such as EtOAc, Et₂O, THF, 1,4-dioxane or EtOH, or a mixture of the aforementioned solvents, in the presence of a lewis acid such as zinc chloride or zinc bromide, at a temperature of about 0 °C to about 25 °C, and then later heated at a temperature of about 50 °C to about 100 °C in a suitable solvent such as EtOH in the presence aqueous acid e.g. aqueous HCl to form alcohol (21). Alternatively, amino alcohol (19) can be reacted directly with an oxadiazole (24), wherein R⁴ is as defined in any one of Embodiments 1.1 to 1.65 and LG represents a suitable leaving group such as halogen (e.g. fluoride, chloride or bromide) or a sulphonic acid ester (e.g. a tosylate, mesylate or triflate), optionally in the presence of a suitable solvent such as THF, 1,4-dioxane, MeCN, EtOH, DMSO, DMF or NMP, optionally in the presence of a suitable base such as Et₃N, DIPEA, K₂CO₃ or DBU, at a temperature suitable to promote reaction, e.g. at a temperature of about 20 °C to about 200 °C, optionally using a sealed vessel under a reaction pressure greater than atmospheric pressure, optionally using conventionally heating or microwave heating. Once formed, alcohol (21) can be oxidized to ketone (11) using a wide variety of reagents and conditions that exist in the art and that are well known to the skilled person, e.g. by using a chromium reagent such as pyridinium chlorochromate in a suitable solvent such a DCM at a temperature of about 0 °C to about 25 °C.

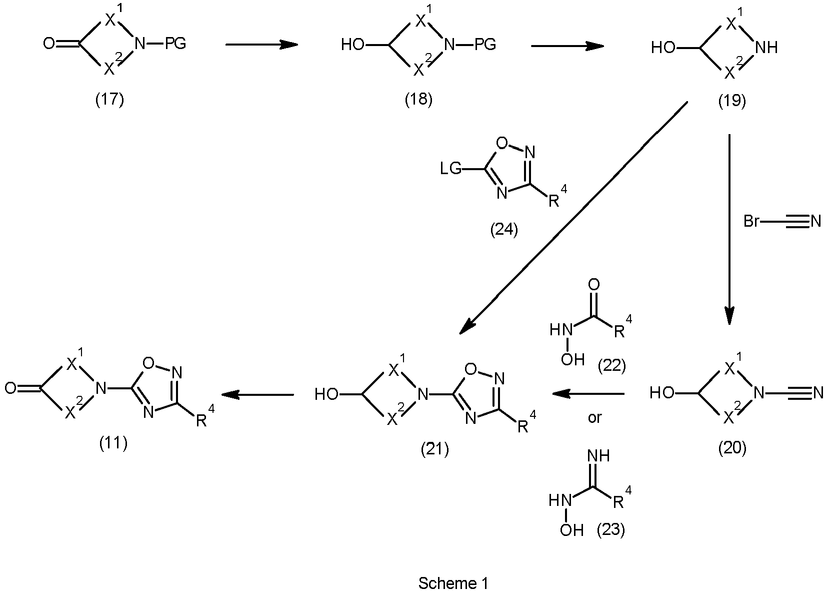

In process variant (B), the ester (12) is reacted with the amine R⁵R⁶NH under conditions suitable to effect formation of an amide. Typically, such conditions are reaction at a temperature between about 0 °C to about 110 °C in a solvent such as toluene in combination with a reagent such as trimethylaluminium (Me₃Al), optionally in the presence of a tertiary base such as TEA or DIPEA. It will be well known to the skilled person that other suitable conditions exist to effect formation of an amide from ester (12) and amine R⁵R⁶NH, such as reaction in the presence of isopropylmagnesium chloride (ⁱPrMgCl) in a suitable solvent, or by direct heating of ester (12) and amine R⁵R⁶NH together, optionally in the presence of a suitable solvent, optionally in the presence of a suitable base such as TEA or DIPEA.

In process variant (C), the carboxylic acid (13) is reacted with the amine R⁵R⁶NH under conditions suitable to effect formation of an amide. It will be well known to the skilled person that many suitable conditions exist in the art to effect formation of an amide from carboxylic acid (13) and amine R⁵R⁶NH, for example reaction with an amide coupling reagent such as diisopropylcarbodiimide (DIC), ethyl-(*N',N*'-dimethylamino)propylcarbodiimide hydrochloride (EDC), (benzotriazol-1-yloxy)tripyrrolidinophosphonium hexafluorophosphate (PyBOP), O-(7-azabenzotriazol-1-yl)-*N*,*N*,*N'*,*N'*-tetramethyluronium hexafluorophosphate (HATU), (1-cyano-2-ethoxy-2-oxoethylidenaminooxy)dimethylamino-morpholino-carbenium hexafluorophosphate (COMU) or 2,4,6-tripropyl-1,3,5,2,4,6-trioxatriphosphorinane-2,4,6-trioxide (T3P), optionally in the presence of a tertiary base such as TEA or DIPEA, optionally in the presence of 1-hydroxybenzotriazole (HOBt), in a solvent such as DCM, THF or DMF, at a temperature between about 0 °C to about 100 °C.

Alternatively, a compound of formula (1) wherein R¹ comprises CONR⁵R⁶ can be prepared from carboxylic acid (13) and an amine R⁵R⁶NH by the sequence of reactions shown in Scheme 2 below:

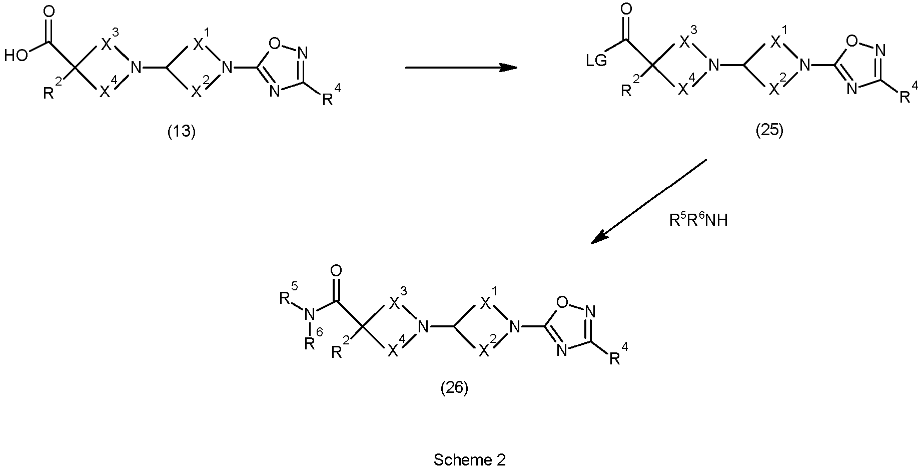

Thus carboxylic acid (13) can be reacted under conditions suitable to effect formation of intermediate (25), wherein R², R⁴, X¹, X², X³ and X⁴ are as defined in any one of Embodiments 1.1 to 1.65 and LG represents a suitable leaving group such as chloride (CI), 1-imidazole, or RO(C=O)O (wherein R represents a group such as ethyl- or isobutyl-). Typically, such conditions are reaction with a reagent such as oxalyl chloride or thionyl chloride (LG = CI), 1,1'-carbonyldiimidazole (CDI) (LG = 1-imidazole) or ethyl- or isobutylchloroformate (LG = RO(C=O)O), optionally in the presence of a tertiary base such as TEA or DIPEA, optionally in the presence of a catalyst such as DMF, in a suitable solvent such as DCM, THF or DMF. Once formed, intermediate (25) is reacted with an amine R⁵R⁶NH under conditions suitable to effect formation of amide (26), wherein R², R⁴, R⁵, R⁶, X¹, X², X³ and X⁴ are as defined in any one of Embodiments 1.1 to 1.65. Typically, such conditions are reaction at a temperature between about 0 °C to about 100 °C in a solvent such as DCM, THF or DMF, optionally in the presence of a tertiary base such as TEA or DIPEA.

In process variant (D), an amine of formula (14) is reacted with a compound of formula (15) or a compound of formula (16) under suitable conditions to form a compound of formula (1) wherein R¹ comprises a 5- or 6-membered ring containing a N(C=O)OR or N(C=O)NR₂ group. Typically, such conditions are reaction at a temperature between about 0 °C to about 100 °C in a solvent such as DCM, THF or DMF, optionally in the presence of a suitable base such as TEA, DIPEA or K₂CO₃.

In process variant (E), one compound of the formula (1) can be converted into another compound of the formula (1) by methods well known to the skilled person. Examples of synthetic procedures for converting one functional group into another functional group are set out in standard texts such as March's Advanced Organic Chemistry: Reactions, Mechanisms, and Structure, 7th Edition, Michael B. Smith, John Wiley, 2013, (ISBN: 978-0-470-46259-1), Organic Syntheses, Online Edition, www.orgsyn.org, (ISSN 2333-3553) and Fiesers' Reagents for Organic Synthesis, Volumes 1-17, John Wiley, edited by Mary Fieser (ISBN: 0-471-58283-2).

In many of the reactions described above, it may be necessary to protect one or more groups to prevent reaction from taking place at an undesirable location on the molecule. Examples of protecting groups, and methods of protecting and deprotecting functional groups, can be found in Greene's Protective Groups in Organic Synthesis, Fifth Edition, Editor: Peter G. M. Wuts, John Wiley, 2014, (ISBN: 9781118057483).

Compounds made by the foregoing methods may be isolated and purified by any of a variety of methods well known to those skilled in the art and examples of such methods include recrystallisation and chromatographic techniques such as column chromatography (e.g. flash chromatography), HPLC and SFC.

### Pharmaceutical Formulations

While it is possible for the active compound to be administered alone, it is preferable to present it as a pharmaceutical composition (e.g. formulation).

Accordingly, in another embodiment (Embodiment 4.1) of the invention, there is provided a pharmaceutical composition comprising at least one compound of the formula (1), formula (1a), formula (2), formula (3) or formula (4), as defined in any one of Embodiments 1.1 to 1.65 together with at least one pharmaceutically acceptable excipient.

In one embodiment (Embodiment 4.2), the composition is a tablet composition.

In another embodiment (Embodiment 4.3), the composition is a capsule composition.

The pharmaceutically acceptable excipient(s) can be selected from, for example, carriers (e.g. a solid, liquid or semi-solid carrier), adjuvants, diluents (e.g solid diluents such as fillers or bulking agents; and liquid diluents such as solvents and co-solvents), granulating agents, binders, flow aids, coating agents, release-controlling agents (e.g. release retarding or delaying polymers or waxes), binding agents, disintegrants, buffering agents, lubricants, preservatives, anti-fungal and antibacterial agents, antioxidants, buffering agents, tonicity-adjusting agents, thickening agents, flavouring agents, sweeteners, pigments, plasticizers, taste masking agents, stabilisers or any other excipients conventionally used in pharmaceutical compositions.

The term "pharmaceutically acceptable" as used herein means compounds, materials, compositions, and/or dosage forms which are, within the scope of sound medical judgment, suitable for use in contact with the tissues of a subject (e.g. a human subject) without excessive toxicity, irritation, allergic response, or other problem or complication, commensurate with a reasonable benefit/risk ratio. Each excipient must also be "acceptable" in the sense of being compatible with the other ingredients of the formulation.

Pharmaceutical compositions containing compounds of the formula (1), formula (1a), formula (2), formula (3) or formula (4) can be formulated in accordance with known techniques, see for example, Remington's Pharmaceutical Sciences, Mack Publishing Company, Easton, PA, USA.

The pharmaceutical compositions can be in any form suitable for oral, parenteral, topical, intranasal, intrabronchial, sublingual, ophthalmic, otic, rectal, intra-vaginal, or transdermal administration.

Pharmaceutical dosage forms suitable for oral administration include tablets (coated or uncoated), capsules (hard or soft shell), caplets, pills, lozenges, syrups, solutions, powders, granules, elixirs and suspensions, sublingual tablets, wafers or patches such as buccal patches.

Tablet compositions can contain a unit dosage of active compound together with an inert diluent or carrier such as a sugar or sugar alcohol, eg; lactose, sucrose, sorbitol or mannitol; and/or a non-sugar derived diluent such as sodium carbonate, calcium phosphate, calcium carbonate, or a cellulose or derivative thereof such as microcrystalline cellulose (MCC), methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Tablets may be designed to release the drug either upon contact with stomach fluids (immediate release tablets) or to release in a controlled manner (controlled release tablets) over a prolonged period of time or with a specific region of the GI tract.

The pharmaceutical compositions typically comprise from approximately 1% (w/w) to approximately 95% (w/w) active ingredient and from 99% (w/w) to 5% (w/w) of a pharmaceutically acceptable excipient (for example as defined above) or combination of such excipients. Preferably, the compositions comprise from approximately 20% (w/w) to approximately 90% (w/w) active ingredient and from 80% (w/w) to 10% of a pharmaceutically acceptable excipient or combination of excipients. The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, pre-filled syringes, dragées, powders, tablets or capsules.

Tablets and capsules may contain, for example, 0-20% disintegrants, 0-5% lubricants, 0-5% flow aids and/or 0-99% (w/w) fillers or bulking agents (depending on drug dose). They may also contain 0-10% (w/w) polymer binders, 0-5% (w/w) antioxidants, 0-5% (w/w) pigments. Slow release tablets would in addition typically contain 0-99% (w/w) release-controlling (e.g. delaying) polymers (depending on dose). The film coats of the tablet or capsule typically contain 0-10% (w/w) polymers, 0-3% (w/w) pigments, and/or 0-2% (w/w) plasticizers.

Parenteral formulations typically contain 0-20% (w/w) buffers, 0-50% (w/w) cosolvents, and/or 0-99% (w/w) Water for Injection (WFI) (depending on dose and if freeze dried). Formulations for intramuscular depots may also contain 0-99% (w/w) oils.

The pharmaceutical formulations may be presented to a patient in "patient packs" containing an entire course of treatment in a single package, usually a blister pack.

The compounds of the formula (1), formula (1a), formula (2), formula (3) or formula (4) will generally be presented in unit dosage form and, as such, will typically contain sufficient compound to provide a desired level of biological activity. For example, a formulation may contain from 1 nanogram to 2 grams of active ingredient, e.g. from 1 nanogram to 2 milligrams of active ingredient. Within these ranges, particular sub-ranges of compound are 0.1 milligrams to 2 grams of active ingredient (more usually from 10 milligrams to 1 gram, e.g. 50 milligrams to 500 milligrams), or 1 microgram to 20 milligrams (for example 1 microgram to 10 milligrams, e.g. 0.1 milligrams to 2 milligrams of active ingredient).

For oral compositions, a unit dosage form may contain from 1 milligram to 2 grams, more typically 10 milligrams to 1 gram, for example 50 milligrams to 1 gram, e.g. 100 milligrams to 1 gram, of active compound.

The active compound will be administered to a patient in need thereof (for example a human or animal patient) in an amount sufficient to achieve the desired therapeutic effect (effective amount). The precise amounts of compound administered may be determined by a supervising physician in accordance with standard procedures.

### EXAMPLES

The invention will now be illustrated, but not limited, by reference to the specific embodiments described in the following examples.

### EXAMPLES 1-1 TO 18-2

The compounds of Examples 1-1 to 18-2 shown in Table 1 below have been prepared. Some of their NMR and LCMS properties and the methods used to prepare them are set out in Table 3. The starting materials and intermediates for some of the Examples are listed in Table 2.

| Table 1 | | |
|---|---|---|
| 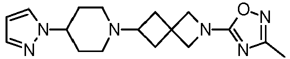 Example 1-1 | 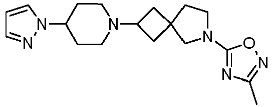 Example 2-1 | 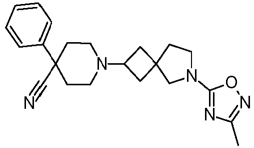 Example 2-2 |
| 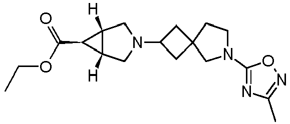 Example 2-3 | 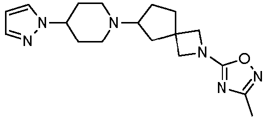 Example 3-1 | 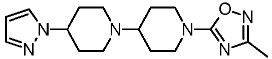 Example 4-1 (*) |
| 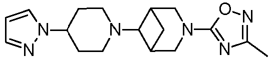 Example 5-1 | 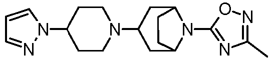 Example 6-1 | 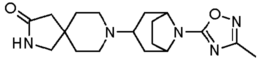 Example 6-2 |
| 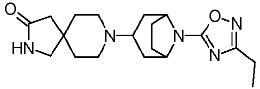 Example 6-3 | 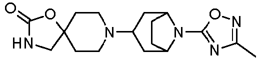 Example 6-4 | 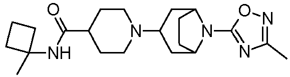 Example 6-5 |
| 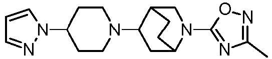 Example 7-1 | 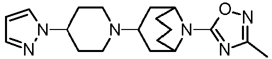 Example 8-1 | 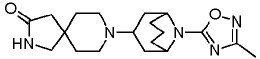 Example 8-2 |
| 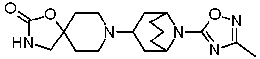 Example 8-3 | 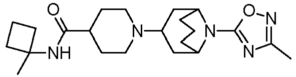 Example 8-4 | 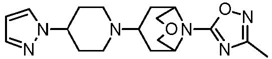 Example 9-1 |
| 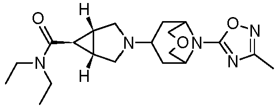 Example 9-2 | 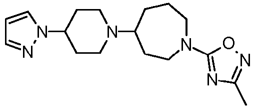 Example 10-1 | 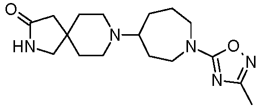 Example 10-2 |
| 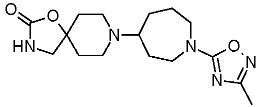 Example 10-3 | 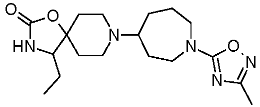 Example 10-4 | 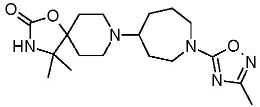 Example 10-5 |
| 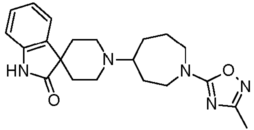 Example 10-6 | 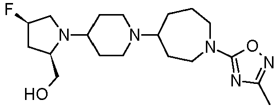 Example 10-7 | 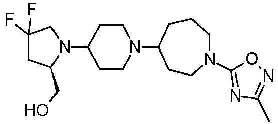 Example 10-8 |
| 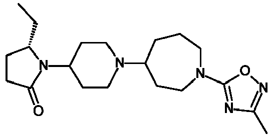 Example 10-9 | 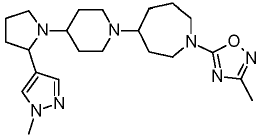 Example 10-10 | 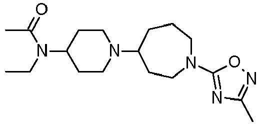 Example 10-11 |
| 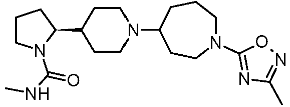 Example 10-12 | 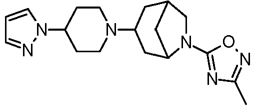 Example 11-1 | 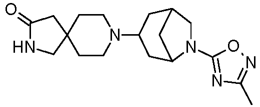 Example 11-2 |
| 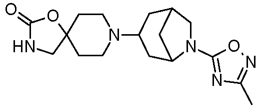 Example 11-3 | 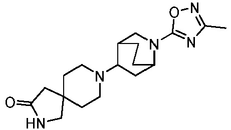 Example 12-1 | 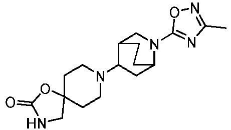 Example 12-2 |
| 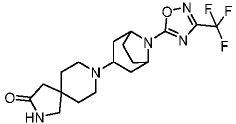 Example 12-3 | 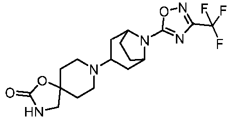 Example 12-4 | 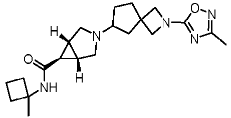 Example 12-6 |
| 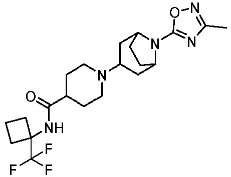 Example 12-7 | 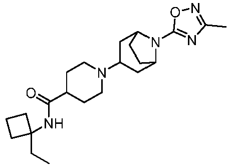 Example 12-8 | 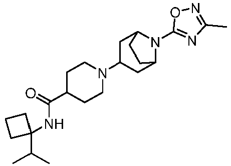 Example 12-9 |
| 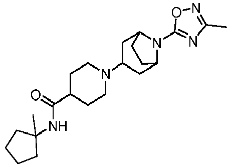 Example 12-11 | 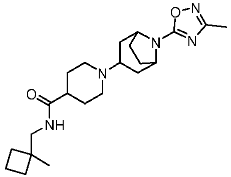 Example 12-12 | 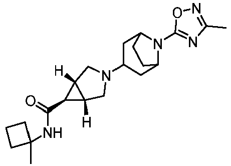 Example 12-13 |
| 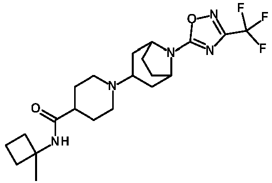 Example 12-14 | 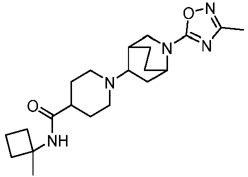 Example 12-15 | 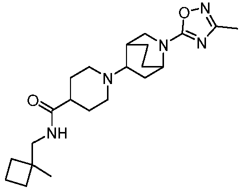 Example 12-16 |
| 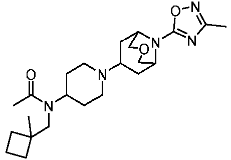 Example 12-17 | 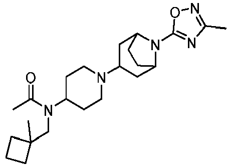 Example 12-18 | 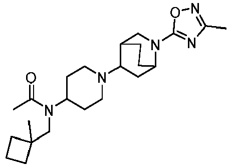 Example 12-19 |
| 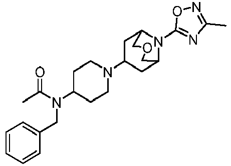 Example 12-20 | 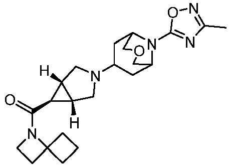 Example 12-21 | 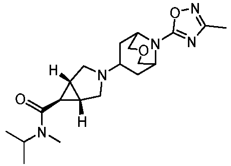 Example 12-22 |
| 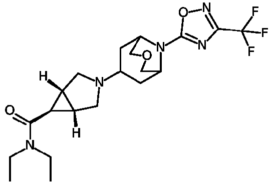 Example 12-23 | 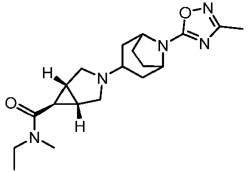 Example 12-24 | 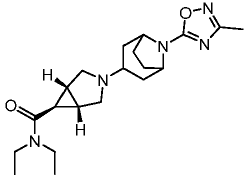 Example 12-25 |
| 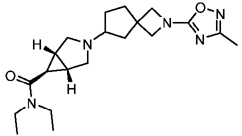 Example 12-26 | 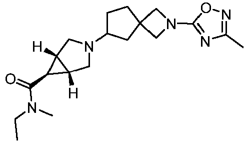 Example 12-27 | 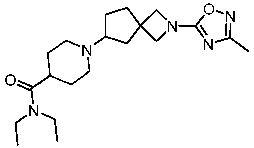 Example 12-28 |
| 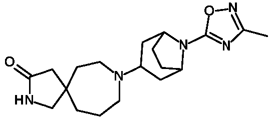 Example 13-1 | 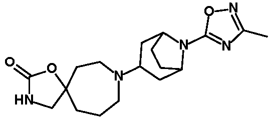 Example 13-2 | 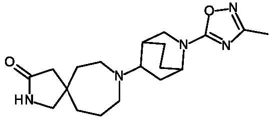 Example 13-3 |
| 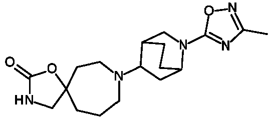 Example 13-4 | 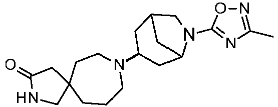 Example 13-5 | 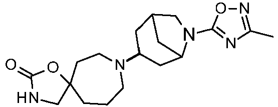 Example 13-6 |
| 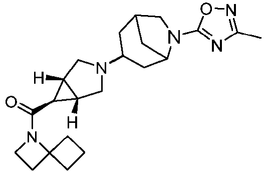 Example 14-1 | 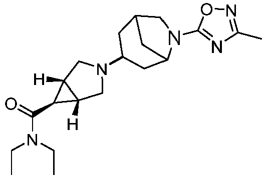 Example 14-2 | 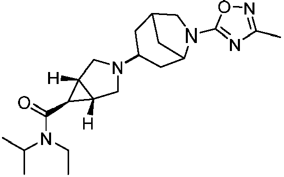 Example 14-3 |
| 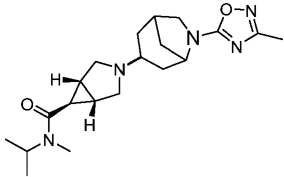 Example 14-4 | 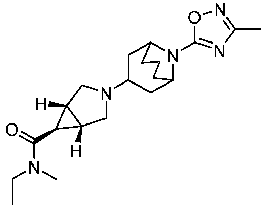 Example 14-5 | 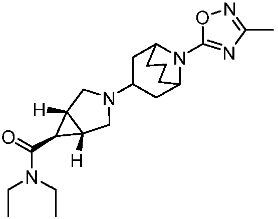 Example 14-6 |
| 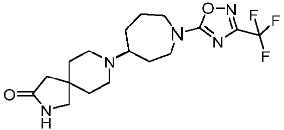 Examnple 15-1 | 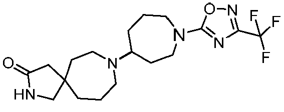 Example 15-2 | 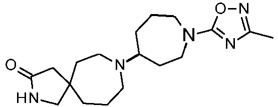 Example 15-3 |
| 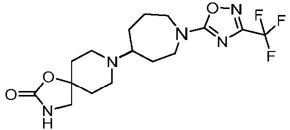 Example 15-4 | 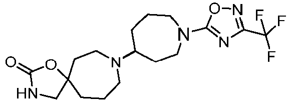 Example 15-5 | 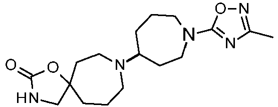 Example 15-6 |
| 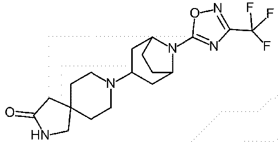 Example 16-1 | 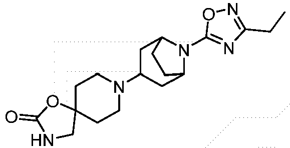 Example 16-2 | 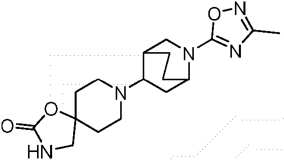 Example 16-3 |
| 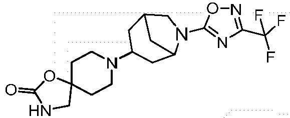 Example 16-4 | 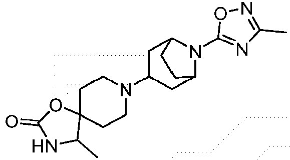 Example 16-5 | 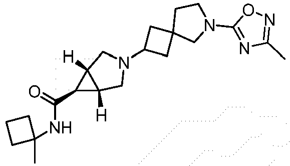 Example 16-10 |
| 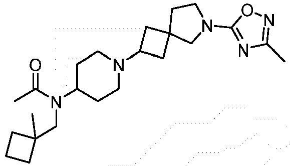 Example 16-11 | 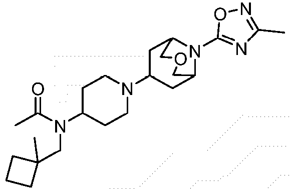 Example 16-12 | 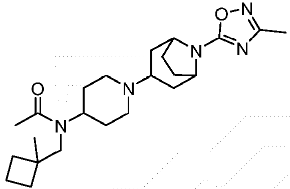 Example 16-13 |
| 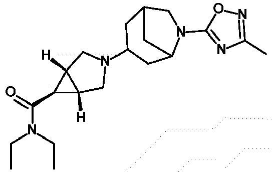 Example 16-14 | 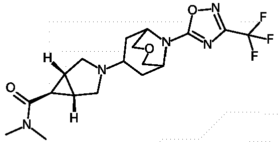 Example 16-15 | 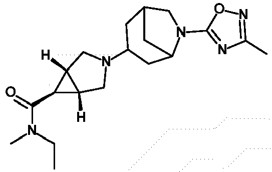 Example 16-16 |
| 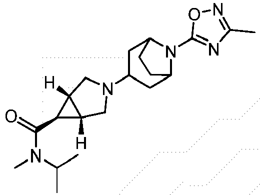 Example 16-17 | 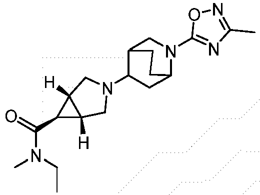 Example 16-18 | 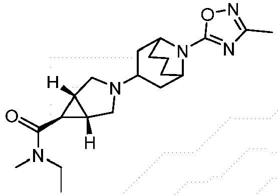 Example 16-19 |
| 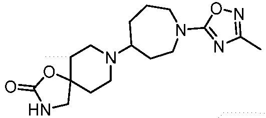 Example 17-1 | 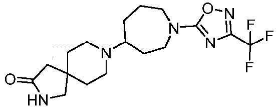 Example 17-2 | 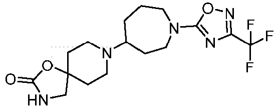 Example 17-3 |
| 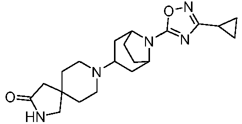 Example 17-4 | 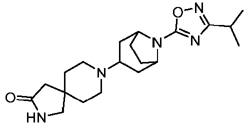 Example 17-5 | 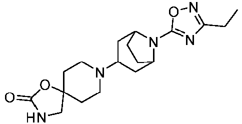 Example 17-6 |
| 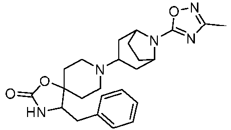 Example 17-7 | 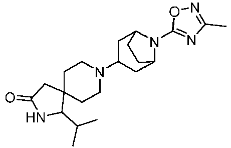 Example 17-8 | 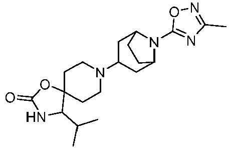 Example 17-9 |
| 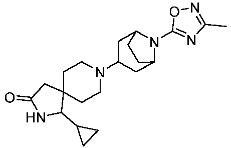 Example 17-10 | 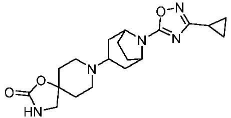 Example 17-11 | 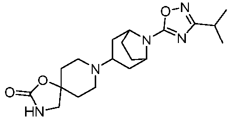 Example 17-12 |
| 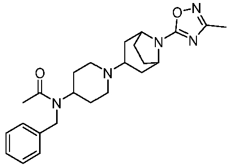 Example 17-13 | 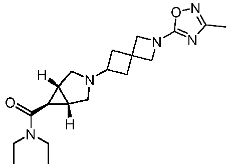 Example 17-14 | 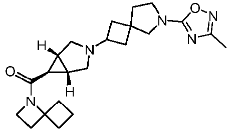 Example 17-15 |
| 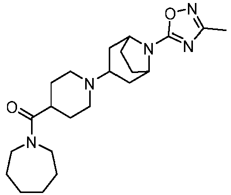 Example 17-16 | 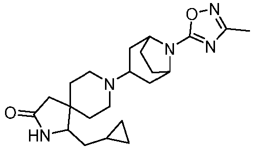 Example 17-17 | 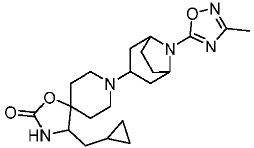 Example 17-18 |
| 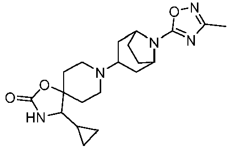 Example 17-19 | 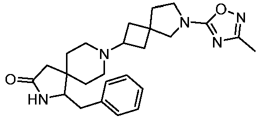 Example 17-20 | 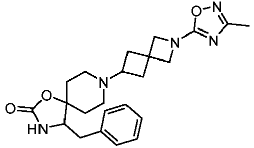 Example 17-21 |
| 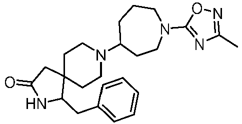 Example 17-22 | 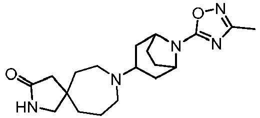 Example 17-23 | 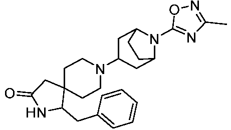 Example 17-24 |
| 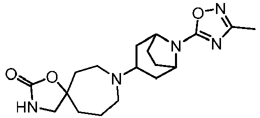 Example 17-25 | 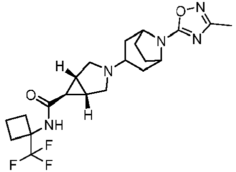 Example 18-1 | 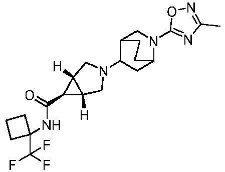 Example 18-2 |

| | | |
|---|---|---|
| (*) Reference compound. | | |

### General procedures

Where no preparative routes are included, the relevant intermediate is commercially available. Commercial reagents were utilized without further purification. Room temperature (rt) refers to approximately 20-27 °C. ¹H NMR spectra were recorded at 400 MHz on either a Bruker or Jeol instrument. Chemical shift values are expressed in parts per million (ppm), i.e. (δ)-values. The following abbreviations are used for the multiplicity of the NMR signals: s=singlet, br=broad, d=doublet, t=triplet, q=quartet, quint=quintet, td=triplet of doublets, tt= triplet of triplets, qd=quartet of doublets, ddd=doublet of doublet of doublets, ddt=doublet of doublet of triplets, m=multiplet. Coupling constants are listed as *J* values, measured in Hz. NMR and mass spectroscopy results were corrected to account for background peaks. Chromatography refers to column chromatography performed using 60 - 120 mesh silica gel and executed under nitrogen pressure (flash chromatography) conditions. TLC for monitoring reactions refers to TLC run using the specified mobile phase and the Silica gel F254 as a stationary phase from Merck. Microwave-mediated reactions were performed in Biotage Initiator or CEM Discover microwave reactors.

### LCMS Analysis

LCMS analysis of compounds was performed under electrospray conditions using the instruments and methods given in the tables below:

| **System** | **Instrument Name** | **LC Detector** | **Mass Detector** |
|---|---|---|---|
| 1 | Waters 2695 | Photo Diode Array | ZQ-2000 Detector |
| 2 | Waters Acquity H Class | Photo Diode Array | SQ Detector |
| 3 | Shimadzu Nexera | Photo Diode Array | LCMS-2020 |
| 4 | Agilent 1290 RRLC | Photo Diode Array | Agilent 6120 |
| 5 | Hewlett Packard HP 1100 | G1315A DAD | Micromass ZQ |

| **Method Name** | **Solvent System** | **Column used** | **Gradient** | **UV Range** | **Mass Range** | **Column Temp.°C** | **Flow Rate ml/min** |
|---|---|---|---|---|---|---|---|
| A | (A) 2 mM ammonium acetate + 0.1% formic acid in water | BEH C18 2.1 x 50 mm, 1.7 µm or equivalent | 98:2 at 0.01 min up to 0.30 min, 50:50 at 0.60 min, 25:75 at 1.10 min, 0:100 at 2.00 min up to 2.70 min, 98:2 at 2.71 min up to 3.00 min | 200 - 400nm | 100-1200 amu | Ambient | 0.55 |
| | (B) 0.1% formic acid in acetonitrile | | | | | | |
| B | (A) 20 mM ammonium acetate in water | X-Bridge C18 4.6 x 150 mm, 5 µm or equivalent | 90:10 at 0.01 min, 10:90 at 5.00 min, 0:100 at 7.00 min up to 11.00 min, 90:10 at 11.01 min up to 12.00 min | 200 - 400 nm | 60-1000 amu | Ambient | 1.00 |
| | (B) methanol | | | | | | |
| C | (A) 10 mM ammonium acetate in water | YMC Triart C18 4.6 x 150 mm, 5 µm or equivalent | 100:0 at 0.01 min, 50:50 at 7.00 min, 0:100 at 9.00 min up to 11.00 min, 100:0 at 11.01 min up to 12.00 min | 200 - 400 nm | 60-1000 amu | Ambient | 1.00 |
| | (B) acetonitrile | | | | | | |
| D | (A) 0.1% ammonia in water | X-Bridge C18 4.6 x 50 mm, 3.5 µm or equivalent | 95:5 at 0.01 min, 10:90 at 5.00 min, 5:95 at 5.80 min up to 7.20 min, 95:5 at 7.21 min up to 10.00 min | 200 - 400 nm | 60-1000 amu | Ambient | 1.00 |
| | (B) 0.1% ammonia in acetonitrile | | | | | | |
| E | (A) 5 mM ammonium acetate + 0.1% formic acid in water | BEH C18 2.1 x 50 mm, 1.7 µm or equivalent | 95:5 at 0.01 min up to 0.40 min, 60:40 at 0.60 min, 40:60 at 1.20 min, 0:100 at 2.30 min up to 3.00 min, 95:5 at 3.01 min up to 3.50 min | 200 - 400 nm | 100-1200 amu | Ambient | 0.55 |
| | (B) 0.1% formic acid in acetonitrile | | | | | | |
| F | (A) 5 mM ammonium bicarbonate in water | X-Bridge C18 4.6 x 50 mm, 3.5 µm or equivalent | 95:5 at 0.01 min, 10:90 at 5.00 min, 5:95 at 5.80 min up to 7.20 min, 95:5 at 7.21 min up to 10.00 min | 200-400 nm | 60-1000 amu | Ambient | 1.00 |
| | (B) acetonitrile | | | | | | |
| G | (A) 2.5 L water + 2.5 mL 28 % ammonia solution in water | Gemini-NX C-18, 2.0 x 30 mm, 3 µm | 98:2 at 0.00 min up to 0.10 min, 5:95 at 8.40 min up to 10.00 min | 230 - 400 nm | 130 - 800 amu | 45 | 1.50 |
| | B) 2.5 L acetonitrile + 135 mL water + 2.5 mL 28% ammonia solution in water | | | | | | |
| H | (A) 2 mM ammonium acetate + 0.1% formic acid in water | BEH C18 2.1 x 50 mm, 1.7 µm or equivalent | 98:2 at 0.01 min up to 0.30 min (flow rate 0.55), 50:50 at 0.60 min (flow rate 0.55), 25:75 at 1.10 min (flow rate 0.55), 0:100 at 2.00 min up to 2.70 min (flow rate 0.60), 98:2 at 2.71 min up to 3.00 min (flow rate 0.55) | 200 - 400 nm | 100-1200 amu | Ambient | 0.55 - 0.60 |
| | (B) 0.1% formic acid in acetonitrile | | | | | | |

LCMS data in the experimental section and Tables 2 and 3 are given in the format: **(Instrument system, Method):** Mass ion, retention time, UV detection wavelength.

### Compound Purification

Final purification of compounds was performed by preparative reversed phase HPLC, chiral HPLC or chiral SFC using the instruments and methods detailed below where data is given in the following format: Purification technique: [phase (column description, column length × internal diameter, particle size), solvent flow-rate, gradient - given as % of mobile phase B in mobile phase A (over time), mobile phase (A), mobile phase (B)].

### Preparative HPLC purification:

Shimadzu LC-20AP binary system with SPD-20A UV detector

### Chiral HPLC purification:

Shimadzu LC-20AP binary system with SPD-20A UV detector

### Chiral SFC purification:

Waters SFC 200
Sepiatec 100
Berger Multigram 2

### Purification Method A

Prep HPLC: [Reversed Phase (X SELECT PHENYL HEXYL, 250 × 19 mm, 5 µm), 15 mL / min, gradient 10 % - 40 % (over 23 min), 100 % (over 2 min), 100 % - 10% (over 4 min), mobile phase (A): 5 mM ammonium bicarbonate in water + 0.1 % ammonia in water, (B): 100 % acetonitrile].

### Purification Method B

Prep HPLC: [Reversed Phase (X-BRIDGE C-8, 250 × 19 mm, 5 µm), 15 mL / min, gradient 10 % - 33 % (over 25 min), 100 % (over 2 min), 10 % (over 3 min), mobile phase (A): 5 mM ammonium bicarbonate in water + 0.1 % ammonia in water, (B): 100 % acetonitrile].

### Purification Method C

Prep HPLC: [Reversed Phase (X-BRIDGE C-18, 250 × 19 mm, 5 µm), 15 mL / min, gradient 37 % - 40 % (over 23 min), 100 % (over 2 min), 37 % (over 2 min), mobile phase (A): 5 mM ammonium bicarbonate in water + 0.1 % ammonia in water, (B): 100 % acetonitrile].

### Purification Method D

Prep HPLC: [Reversed Phase (X-BRIDGE C-8, 250 × 19 mm, 5 µm), 15 mL / min, gradient 30 % - 40 % (over 20 min), 100 % (over 2 min), 100 - 30 % (over 3 min), mobile phase (A): 5 mM ammonium bicarbonate in water + 0.1 % ammonia in water, (B): 100 % acetonitrile].

### Purification Method E

Prep HPLC: [Reversed Phase (X-BRIDGE C-8, 250 × 19 mm, 5 µm), 15 mL / min, gradient 5 % - 40 % (over 20 min), 40 % (over 3 min), 100 % (over 2 min), 100 - 5 % (over 2 min), mobile phase (A): 5 mM ammonium bicarbonate in water + 0.1 % ammonia in water, (B): 100 % acetonitrile].

### Purification Method F

Prep HPLC: [Reversed Phase (X SELECT PHENYL HEXYL, 250 × 19 mm, 5 µm), 5 mL / min, gradient 15 % - 47 % (over 15 min), 100 % (over 1 min), 10 % (over 3 min), mobile phase (A): 5 mM ammonium bicarbonate in water + 0.1 % ammonia in water, (B): 100 % acetonitrile].

### Purification Method G

Prep HPLC: [Reversed Phase (X-BRIDGE C-8, 250 × 19 mm, 5 µm), 15 mL / min, gradient 5 % - 50 % (over 17 min), 50 - 70 % (over 2 min), 100 % (over 3 min), 100 - 5 % (over 3 min), mobile phase (A): 5 mM ammonium bicarbonate in water + 0.1 % ammonia in water, (B): 100 % acetonitrile].

### Purification Method H

Prep HPLC: [Reversed Phase (X-BRIDGE C-8, 250 × 19 mm, 5 µm), 16 mL / min, gradient 15 % - 42 % (over 20 min), 100 % (over 2 min), 100 % - 15 % (over 3 min), mobile phase (A): 5 mM ammonium bicarbonate in water + 0.1 % ammonia in water, (B): 100 % acetonitrile].

### Purification Method I

Prep HPLC: [Reversed Phase (X-BRIDGE C-18, 250 × 19 mm, 5 µm), 16 mL / min, gradient 5 % - 30 % (over 19 min), 100 % (over 3 min), 100 % - 5 % (over 4 min), mobile phase (A): 5 mM ammonium bicarbonate in water + 0.1 % ammonia in water, (B): 100 % acetonitrile].

### Purification Method J

Prep HPLC: [Reversed Phase (X-BRIDGE C-18, 250 × 19 mm, 5 µm), 10 mL / min, gradient 0 % - 30 % (over 45 min), 30 - 30 % (over 48 min), 100 % (over 2 min), 100 % - 0 % (over 3 min), mobile phase (A): 5 mM ammonium bicarbonate in water + 0.1 % ammonia in water, (B): 100 % acetonitrile].

### Purification Method K

Prep HPLC: [Reversed Phase (X-BRIDGE C-8, 250 × 19 mm, 5 µm), 15 mL / min, gradient 5 % - 38 % (over 25 min), 100 % (over 2 min), 100 - 5 % (over 5 min), mobile phase (A): 5 mM ammonium bicarbonate in water + 0.1 % ammonia in water, (B): 100 % acetonitrile].

### Purification Method L

Prep HPLC: [Reversed Phase (X-BRIDGE C-18, 250 × 19 mm, 5 µm), 16 mL / min, gradient 20 % - 30 % (over 25 min), 30 - 30 % (over 5 min), 100 % (over 3 min), 100 % - 20 % (over 5 min), mobile phase (A): 5 mM ammonium bicarbonate in water + 0.1 % ammonia in water, (B): 100 % acetonitrile].

### Purification Method M

Prep HPLC: [Reversed Phase (X SELECT PHENYL HEXYL, 250 × 19 mm, 5 µm), 15 mL / min, gradient 15 % - 30 % (over 30 min), 30 - 30% (over 6 min), 100 % (over 2 min), 100 - 15 % (over 4 min), mobile phase (A): 5 mM ammonium bicarbonate in water + 0.1 % ammonia in water, (B): 100 % acetonitrile].

### Purification Method N

Prep HPLC: [Reversed Phase (X SELECT PHENYL HEXYL, 250 × 19 mm, 5 µm), 15 mL / min, gradient 25 % - 35 % (over 23 min), 100 % (over 1 min), 100 - 25 % (over 4 min), mobile phase (A): 5 mM ammonium bicarbonate in water + 0.1 % ammonia in water, (B): 100 % acetonitrile].

### Purification Method O

Chiral HPLC: [Normal Phase (CHIRALPAK AD-H, 250 × 21 mm, 5 µm), 18 mL / min, Isochratic (A : B) 45 : 55 (over 45 min), mobile phase (A): 0.1 % diethylamine in hexane, (B): 0.1 % diethylamine in isopropanol : methanol (30 : 70)].

### Purification Method P

Prep HPLC: [Reversed Phase (X-BRIDGE C-18, 250 × 19 mm, 5 µm), 13 mL / min, gradient 15 % - 20 % (over 30 min), 20 - 20 % (over 6 min), 100 % (over 1 min), 100 % - 15 % (over 6 min), mobile phase (A): 5 mM ammonium bicarbonate in water + 0.1 % ammonia in water, (B): 100 % acetonitrile].

### Purification Method Q

Prep HPLC: [Reversed Phase (X-BRIDGE C-18, 250 × 19 mm, 5 µm), 15 mL / min, gradient 5 % - 17 % (over 32 min), 17 - 17 % (over 58 min), 100 % (over 3 min), 100 % - 5 % (over 4 min), mobile phase (A): 5 mM ammonium bicarbonate in water + 0.1 % ammonia in water, (B): 100 % acetonitrile].

### Purification Method R

Prep HPLC: [Reversed Phase (X-BRIDGE C-8, 250 × 19 mm, 5 µm), 15 mL / min, gradient 20 % - 35 % (over 25 min), 35 - 35 % (over 3 min), 100 % (over 2 min), 100 - 20 % (over 3 min), mobile phase (A): 5 mM ammonium bicarbonate in water + 0.1 % ammonia in water, (B): 100 % acetonitrile].

### Purification Method S

SFC: [(CHIRALCEL OX-H, 250 × 21 mm, 5 µm), 70 mL / min, Isochratic (A : B) 70 : 30 (over 8.5 min), mobile phase (A): 100 % liquid CO₂, (B): 0.1 % diethylamine in isopropanol : methanol (50 : 50)].

### Purification Method T

Prep HPLC: [Reversed Phase (X-BRIDGE C-8, 250 × 19 mm, 5 µm), 15 mL / min, gradient 18 % - 30 % (over 18 min), 100 % (over 2 min), 100 - 18 % (over 2 min), mobile phase (A): 5 mM ammonium bicarbonate in water + 0.1 % ammonia in water, (B): 100 % acetonitrile].

### Purification Method U

Chiral HPLC: [Normal Phase (CHIRALCEL OX-H, 250 × 21 mm, 5 µm), 18 mL / min, Isochratic (A : B) 90 : 10 (over 32 min), mobile phase (A): 0.1 % diethylamine in hexane, (B): 0.1 % diethylamine in isopropanol : acetonitrile (70 : 30)].

### Purification Method V

Prep HPLC: [Reversed Phase (X-BRIDGE C-18, 250 × 19 mm, 5 µm), 15 mL / min, gradient 10 % - 25 % (over 25 min), 25 - 25 % (over 7 min), 100 % (over 2 min), 100 % - 10 % (over 2 min), mobile phase (A): 5 mM ammonium bicarbonate in water + 0.1 % ammonia in water, (B): 100 % acetonitrile].

### Purification Method W

Prep HPLC: [Reversed Phase (X SELECT PHENYL HEXYL, 250 × 19 mm, 5 µm), 15 mL / min, gradient 25 % - 40 % (over 15 min), 100 % (over 3 min), 100 % - 25 % (over 5 min), mobile phase (A): 0.1 % ammonia in water, (B): 100 % acetonitrile].

### Purification Method X

SFC: [(LUX A1, 250 × 21.2 mm, 5 µm), 50 mL / min, Isochratic (A : B) 65 : 35, mobile phase (A): 100 % liquid CO₂, (B): 0.1 % ammonia in methanol].

### Purification Method Y

Prep HPLC: [Reversed Phase (X-BRIDGE C-8, 250 × 19 mm, 5 µm), 15 mL / min, gradient 8 % - 20 % (over 23 min), 20 % - 20 % (over 2 min), 100 % (over 2 min), 100 % - 8 % (over 5 min), mobile phase (A): 5 mM ammonium bicarbonate in water + 0.1 % ammonia in water, (B): 100 % acetonitrile].

### Purification Method Z

SFC: [(CHIRALPAK AD-H, 250 × 21 mm, 5 µm), 75 mL / min, Isochratic (A : B) 82 : 18 (over 20 min), mobile phase (A): 100 % liquid CO₂, (B): 0.1 % diethylamine in methanol].

### Purification Method AA

Prep HPLC: [Reversed Phase (X-BRIDGE C-8, 250 × 19 mm, 5 µm), 15 mL / min, gradient 8 % - 23 % (over 17 min), 23 % - 23 % (over 2 min), 100 % (over 2 min), 100 % - 8 % (over 4 min), mobile phase (A): 5 mM ammonium bicarbonate in water + 0.1 % ammonia in water, (B): 100 % acetonitrile].

### Purification Method AB

Prep HPLC: [Reversed Phase (YMC ACTUS TRIART C-18, 250 × 20 mm, 5 µm), 15 mL / min, gradient 15 % - 30 % (over 28 min), 100 % (over 2 min), 100 % - 15 % (over 2 min), mobile phase (A): 5 mM ammonium bicarbonate in water + 0.1 % ammonia in water, (B): 100 % acetonitrile].

### Purification Method AC

SFC: [(CHIRALPAK AD-H, 250 × 21 mm, 5 µm), 75 mL / min, Isochratic (A : B) 75 : 25 (over 18 min), mobile phase (A): 100 % liquid CO₂, (B): 0.1 % diethylamine in methanol].

### Purification Method AD

Prep HPLC: [Reversed Phase (X-BRIDGE C-18, 250 × 50 mm, 5 µm), 90 mL / min, gradient 5 % - 35 % (over 20 min), 100 % (over 2 min), 100 % - 5 % (over 3 min), mobile phase (A): 5 mM ammonium bicarbonate in water + 0.1 % ammonia in water, (B): 100 % acetonitrile].

### Purification Method AE

Chiral HPLC: [Normal Phase (CHIRALPAK AD-H, 250 × 21 mm, 5 µm), 18 mL / min, Isochratic (A : B) 70 : 30 (over 35 min), mobile phase (A): 0.1 % diethylamine in hexane, (B): 0.1 % diethylamine in isopropanol : methanol (50 : 50)].

### Purification Method AF

Prep HPLC: [Reversed Phase (X SELECT PHENYL HEXYL, 250 × 19 mm, 5 µm), 13 mL / min, gradient 15 % - 30 % (over 30 min), 30 % - 30 % (over 10 min), 100 % (over 2 min), 100 % - 15 % (over 3 min), mobile phase (A): 5 mM ammonium bicarbonate in water + 0.1 % ammonia in water, (B): 100 % acetonitrile].

### Purification Method AG

SFC: [(CHIRALPAK AD-H, 250 × 21 mm, 5 µm), 80 mL / min, Isochratic (A : B) 75 : 25 (over 13 min), mobile phase (A): 100 % liquid CO₂, (B): 0.1 % diethylamine in isopropanol : methanol (50 : 50)].

### Purification Method AH

Prep HPLC: [Reversed Phase (X-BRIDGE C-18, 250 × 50 mm, 5 µm), 80 mL / min, gradient 5 % - 18 % (over 50 min), 18 % - 18 % (over 10 min), 100 % (over 3 min), 100 % - 5 % (over 7 min), mobile phase (A): 5 mM ammonium bicarbonate in water + 0.1 % ammonia in water, (B): 100 % acetonitrile].

### Purification Method Al

SFC: [(CHIRALPAK AD-H, 250 × 21 mm, 5 µm), 80 mL / min, Isochratic (A : B) 70 : 30 (over 20 min), mobile phase (A): 100 % liquid CO₂, (B): 0.1 % diethylamine in methanol].

### Purification Method AJ

Prep HPLC: [Reversed Phase (X-BRIDGE C-8, 250 × 19 mm, 5 µm), 15 mL / min, gradient 0 % - 27 % (over 28 min), 27 % - 27 % (over 6 min), 100 % (over 4 min), 100 % - 0 % (over 2 min), mobile phase (A): 5 mM ammonium bicarbonate in water + 0.1 % ammonia in water, (B): 100 % acetonitrile].

### Purification Method AK

Chiral HPLC: [Normal Phase (CHIRALPAK AD-H, 250 × 21 mm, 5 µm), 18 mL / min, Isochratic (A : B) 90 : 10 (over 27 min), mobile phase (A): 0.1 % diethylamine in hexane, (B): 0.1 % diethylamine in isopropanol].

### Purification Method AL

Prep HPLC: [Reversed Phase (X-BRIDGE C-8, 250 × 19 mm, 5 µm), 15 mL / min, gradient 15 % - 35 % (over 20 min), 35 % - 35 % (over 3 min), 100 % (over 2 min), 100 % - 15 % (over 3 min), mobile phase (A): 5 mM ammonium bicarbonate in water + 0.1 % ammonia in water, (B): 100 % acetonitrile].

### Purification Method AM

SFC: [(CHIRALPAK AD-H, 250 × 21 mm, 5 µm), 80 mL / min, Isochratic (A : B) 65 : 35 (over 16.5 min), mobile phase (A): 100 % liquid CO₂, (B): 0.1 % diethylamine in methanol].

### Purification Method AN

Prep HPLC: [Reversed Phase (X-BRIDGE C-18, 250 × 19 mm, 5 µm), 15 mL / min, gradient 10 % - 30 % (over 20 min), 30 % - 30 % (over 5 min), 100 % (over 2 min), 100 % - 10 % (over 3 min), mobile phase (A): 5 mM ammonium bicarbonate in water + 0.1 % ammonia in water, (B): 100 % acetonitrile].

### Purification Method AO

Chiral HPLC: [Normal Phase (CHIRALPAK AD-H, 250 × 21 mm, 5 µm), 18 mL / min, Isochratic (A : B) 85 : 15 (over 30 min), mobile phase (A): 0.1 % diethylamine in hexane, (B): 0.1 % diethylamine in isopropanol].

### Purification Method AP

Prep HPLC: [Reversed Phase (X-BRIDGE C-18, 250 × 19 mm, 5 µm), 16 mL / min, gradient 10 % - 32 % (over 18 min), 100 % (over 2 min), 100 % - 10 % (over 2 min), mobile phase (A): 5 mM ammonium bicarbonate in water + 0.1 % ammonia in water, (B): 100 % acetonitrile].

### Purification Method AQ

Prep HPLC: [Reversed Phase (X-BRIDGE C-18, 250 × 19 mm, 5 µm), 13 mL / min, gradient 20 % - 25 % (over 17 min), 25 % - 25 % (over 3 min), 100 % (over 2 min), 100 % - 20 % (over 3 min), mobile phase (A): 5 mM ammonium bicarbonate in water + 0.1 % ammonia in water, (B): 100 % acetonitrile].

### Purification Method AR

Chiral HPLC: [Normal Phase (CHIRALCEL OX-H, 250 × 21 mm, 5 µm), 18 mL / min, Isochratic (A : B) 78 : 22 (over 37 min), mobile phase (A): 0.1 % diethylamine in hexane, (B): 0.1 % diethylamine in isopropanol : methanol (50 : 50)].

### Purification Method AS

Prep HPLC: [Reversed Phase (X SELECT PHENYL HEXYL, 250 × 19 mm, 5 µm), 15 mL / min, gradient 25 % - 38 % (over 16 min), 100 % (over 2 min), 100 % - 25 % (over 5 min), mobile phase (A): 5 mM ammonium bicarbonate in water + 0.1 % ammonia in water, (B): 100 % acetonitrile].

### Purification Method AT

Prep HPLC: [Reversed Phase (X SELECT PHENYL HEXYL, 250 × 19 mm, 5 µm), 15 mL / min, gradient 5 % - 35 % (over 20 min), 100 % (over 4 min), 100 % - 5 % (over 4 min), mobile phase (A): 0.1 % ammonia in water, (B): 100 % acetonitrile].

### Purification Method AU

Prep HPLC: [Reversed Phase (X-BRIDGE C-8, 250 × 19 mm, 5 µm), 15 mL / min, gradient 5 % - 28 % (over 18 min), 28 % - 28 % (over 2 min), 100 % (over 2 min), 100 % - 5 % (over 2 min), mobile phase (A): 5 mM ammonium bicarbonate in water + 0.1 % ammonia in water, (B): 100 % acetonitrile].

### Purification Method AV

Prep HPLC: [Reversed Phase (X-BRIDGE C-8, 250 × 19 mm, 5 µm), 15 mL / min, gradient 10 % - 20 % (over 25 min), 20 % - 20 % (over 8 min), 100 % (over 2 min), 100 % - 10 % (over 5 min), mobile phase (A): 5 mM ammonium bicarbonate in water + 0.1 % ammonia in water, (B): 100 % acetonitrile].

### Purification Method AW

Prep HPLC: [Reversed Phase (X-BRIDGE C-8, 250 × 19 mm, 5 µm), 15 mL / min, gradient 15 % - 20 % (over 22 min), 20 % - 20 % (over 5 min), 100 % (over 2 min), 100 % - 15 % (over 6 min), mobile phase (A): 5 mM ammonium bicarbonate in water + 0.1 % ammonia in water, (B): 100 % acetonitrile].

### Purification Method AX

Prep HPLC: [Reversed Phase (X-BRIDGE C-8, 250 × 19 mm, 5 µm), 10 mL / min, gradient 5 % - 55 % (over 18 min), 55 % - 55 % (over 6 min), 100 % (over 2 min), 100 % - 5 % (over 6 min), mobile phase (A): 5 mM ammonium bicarbonate in water + 0.1 % ammonia in water, (B): 100 % acetonitrile].

### Purification Method AY

Prep HPLC: [Reversed Phase (X-BRIDGE C-18, 250 × 19 mm, 5 µm), 15 mL / min, gradient 20 % - 45 % (over 22 min), 100 % (over 2 min), 100 % - 20 % (over 5 min), mobile phase (A): 5 mM ammonium bicarbonate in water + 0.1 % ammonia in water, (B): 100 % acetonitrile].

### Purification Method AZ

Prep HPLC: [Reversed Phase (X-BRIDGE C-18, 250 × 19 mm, 5 µm), 16 mL / min, gradient 30 % - 39 % (over 14 min), 100 % (over 2 min), 100 % - 30 % (over 4 min), mobile phase (A): 5 mM ammonium bicarbonate in water + 0.1 % ammonia in water, (B): 100 % acetonitrile].

### Purification Method BA

SFC: [(CHIRALPAK AD-H, 250 × 21 mm, 5 µm), 80 mL / min, Isochratic (A : B) 85 : 15 (over 7 min), mobile phase (A): 100 % liquid CO₂, (B): 0.1 % diethylamine in methanol].

### Purification Method BB

Prep HPLC: [Reversed Phase (X-BRIDGE C-8, 250 × 19 mm, 5 µm), 13 mL / min, gradient 25 % - 35 % (over 28 min), 35 % - 35 % (over 2 min), 100 % (over 2 min), 100 % - 25 % (over 5 min), mobile phase (A): 5 mM ammonium bicarbonate in water + 0.1 % ammonia in water, (B): 100 % acetonitrile].

### Purification Method BC

Prep HPLC: [Reversed Phase (X-BRIDGE C-8, 250 × 19 mm, 5 µm), 13 mL / min, gradient 25 % - 40 % (over 30 min), 40 % - 40 % (over 2 min), 100 % (over 2 min), 100 % - 25 % (over 6 min), mobile phase (A): 5 mM ammonium bicarbonate in water + 0.1 % ammonia in water, (B): 100 % acetonitrile].

### Purification Method BD

Prep HPLC: [Reversed Phase (X-BRIDGE C-8, 250 × 19 mm, 5 µm), 14 mL / min, gradient 20 % - 50 % (over 20 min), 50 % - 50 % (over 5 min), 100 % (over 2 min), 100 % - 20 % (over 6 min), mobile phase (A): 5 mM ammonium bicarbonate in water + 0.1 % ammonia in water, (B): 100 % acetonitrile].

### Purification Method BE

Prep HPLC: [Reversed Phase (X-BRIDGE C-8, 250 × 19 mm, 5 µm), 12 mL / min, gradient 5 % - 50 % (over 23 min), 50 % - 50 % (over 5 min), 100 % (over 2 min), 100 % - 5 % (over 3 min), mobile phase (A): 5 mM ammonium bicarbonate in water + 0.1 % ammonia in water, (B): 100 % acetonitrile].

### Purification Method BF

Prep HPLC: [Reversed Phase (X-BRIDGE C-8, 250 × 19 mm, 5 µm), 13 mL / min, gradient 5 % - 55 % (over 22 min), 55 % - 55 % (over 6 min), 100 % (over 2 min), 100 % - 5 % (over 3 min), mobile phase (A): 5 mM ammonium bicarbonate in water + 0.1 % ammonia in water, (B): 100 % acetonitrile].

### Purification Method BG

Prep HPLC: [Reversed Phase (X-BRIDGE C-18, 250 × 19 mm, 5 µm), 14 mL / min, gradient 25 % - 52 % (over 25 min), 52 % - 52 % (over 9 min), 100 % (over 2 min), 100 % - 25 % (over 4 min), mobile phase (A): 5 mM ammonium bicarbonate in water + 0.1 % ammonia in water, (B): 100 % acetonitrile].

### Abbreviations

- AcOH =: acetic acid
- aq. =: aqueous
- DCM =: dichloromethane
- DIPEA =: diisopropylethylamine
- DMF =: dimethylformamide
- DMSO =: dimethylsulfoxide
- ES(I) =: electro spray ionization
- Et₂O =: diethyl ether
- EtOAc =: ethyl acetate
- EtOH =: ethanol
- h =: hour(s)
- H₂O =: water
- HATU =: 1-[bis(dimethylamino)methylene]-1H-1,2,3-triazolo[4,5-b]pyridinium 3-oxid hexafluorophosphate
- HCl =: hydrogen chloride, hydrochloric acid
- HPLC =: high performance liquid chromatography
- LC =: liquid chromatography
- MeCN =: acetonitrile
- MeOH =: Methanol
- min(s) =: minute(s)
- MS =: mass spectrometry
- nm =: nanometre(s)
- NMR =: nuclear magnetic resonance
- sat. =: saturated
- SFC =: supercritical fluid chromatography
- STAB =: sodium triacetoxyborohydride
- TFA =: trifluoroacetic acid
- THF =: tetrahydrofuran
- TLC =: thin layer chromatography

Prefixes *n-, s-, i-, t-* and *tert-* have their usual meanings: normal, secondary, *iso,* and tertiary.

### Synthesis of Intermediates:

### Route 1

### Typical procedure for the preparation of ketones, as exemplified by the preparation of Intermediate 3, 2-(3-methyl-1,2,4-oxadiazol-5-yl)-2-azaspiro[3.3]heptan-6-one

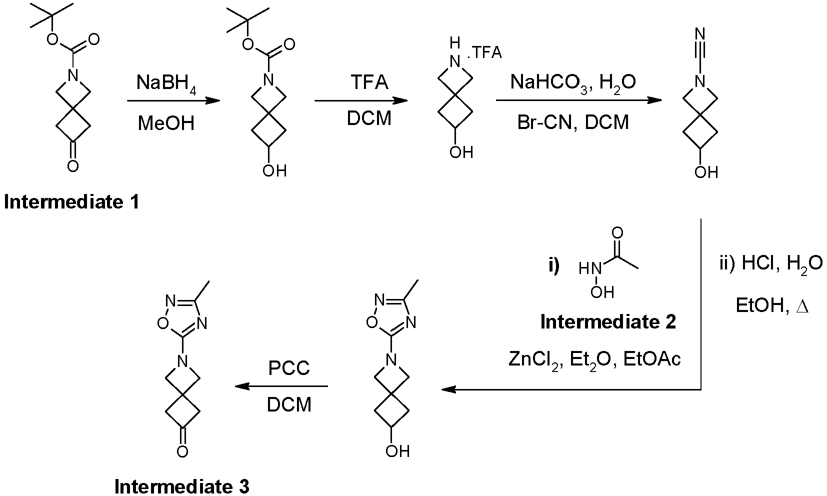

*tert*-Butyl 6-oxo-2-azaspiro[3.3]heptane-2-carboxylate **(Intermediate 1)** (500 mg, 2.37 mmol) was dissolved in MeOH (10 mL) and the resulting solution was cooled to 0 °C. Sodium borohydride (270 mg, 7.10 mmol) was added portion wise and the resulting reaction mixture was stirred at 25 °C for 2 h. The solvents were removed *in-vacuo* and the residue was partitioned between H₂O (80 mL) and EtOAc (60 mL). The aqueous layer was further extracted with EtOAc (2 × 60 mL), and the organic layers were combined, dried (Na₂SO₄), and the solvent was removed *in-vacuo.* The residue was triturated with pentane to give *tert*-butyl 6-hydroxy-2-azaspiro[3.3]heptane-2-carboxylate (500 mg, 99 %) as a solid.

**LCMS (System 2, Method E):** m/z 214 (M+H)⁺ (ESI +ve), at 1.54 min, 202 nm.

*tert*-Butyl 6-hydroxy-2-azaspiro[3.3]heptane-2-carboxylate (500 mg, 2.34 mmol) was dissolved in DCM (10 mL) and the resulting solution was cooled to 0 °C. Trifluoroacetic acid (1 mL) was added dropwise and the resulting reaction mixture was stirred at 25 °C for 6 h. The solvents were removed *in-vacuo* and the residue was triturated with pentane (3 x 1 mL) to give 2-azaspiro[3.3]heptan-6-ol trifluoroacetic acid salt (260 mg, 98 %) as a gum.

**LCMS (System 2, Method E):** m/z 114 (M+H)⁺ (ESI +ve), at 0.22 min, 202 nm.

2-Azaspiro[3.3]heptan-6-ol trifluoroacetic acid salt (250 mg, 2.21 mmol) was dissolved in DCM (10 mL), treated with sat. aq. NaHCO₃ (1 mL) and cooled to 0 °C for 10 min. Cyanogen bromide (255 mg, 2.43 mmol) was added portion wise and the resulting reaction mixture was stirred at 25 °C for 5 h. The solvents were removed *in-vacuo* and the residue was basified with sat. aq. NaHCO₃ solution. The precipitated solids were removed by filtration and washed with DCM 5 to 6 times. The combined filtrate and washings were then dried (Na₂SO₄), the solvent was removed *in-vacuo* and the crude product was purified by trituration with pentane to give 6-hydroxy-2-azaspiro[3.3]heptane-2-carbonitrile (230 mg, 75 %) as a gum.

**LCMS (System 2, Method E):** m/z 139 (M+H)⁺ (ESI +ve), at 0.76 min, 202 nm.

6-Hydroxy-2-azaspiro[3.3]heptane-2-carbonitrile (180 mg, 1.30 mmol) was dissolved in a mixture of EtOAc:Et₂O (5:1) (5 mL) and *N*-hydroxyacetamide **(Intermediate 2)** (125 mg, 1.69 mmol) was added. The resulting mixture was stirred at 25 °C for 5 min, then a solution of ZnCl₂ in diethyl ether (1 M, 1 mL, 1 mmol) was added drop wise. The reaction mixture was stirred at 25 °C for 20 min, during which time a white precipitate formed. The reaction mixture was diluted with Et₂O (3 x 10 mL) and the solvent was decanted off each time. The remaining solid was dissolved in EtOH (3 mL), treated with aq. HCl (4 M, 2 mL) and heated at reflux for 6 h. The solvents were removed *in-vacuo* and the residue was basified with aq. NaHCO₃ and then partitioned between H₂O (80 mL) and DCM (60 mL). The aqueous layer was further extracted with DCM (2 × 60 mL) and the combined organic layers were dried (Na₂SO₄) and the solvent was removed *in-vacuo.* The residue was purified by trituration with pentane to give 2-(3-methyl-1,2,4-oxadiazol-5-yl)-2-azaspiro[3.3]heptan-6-ol (60 mg, 24 %) as a gum. **LCMS (System 2, Method E):** m/z 196 (M+H)⁺ (ESI +ve), at 1.34 min, 202 nm.

2-(3-Methyl-1,2,4-oxadiazol-5-yl)-2-azaspiro[3.3]heptan-6-ol (80 mg, 0.41 mmol) was dissolved in DCM (5 mL) and the solution was cooled to 0 °C. Pyridinium chlorochromate (132 mg, 0.62 mmol) was added portion wise and the resulting reaction mixture was stirred at 25 °C for 3 h. The solvents were then removed *in-vacuo* and the residue was basified with NaHCO₃ solution and partitioned between H₂O (60 mL) and EtOAc (45 mL). The aqueous layer was further extracted with EtOAc (2 × 45 mL), and the combined organic layers were dried (Na₂SO₄), and the solvent was removed *in-vacuo.* The residue was triturated with pentane to give 2-(3-methyl-1,2,4-oxadiazol-5-yl)-2-azaspiro[3.3]heptan-6-one **(Intermediate 3)** (60 mg, 76 %) as a gum.

The data for **Intermediate 3** are in Table 2.

### Route 2

### Typical procedure for the preparation of alcohols, as exemplified by the preparation of Intermediate 6, 6-azaspiro[3.4]octan-2-ol hydrochloride salt

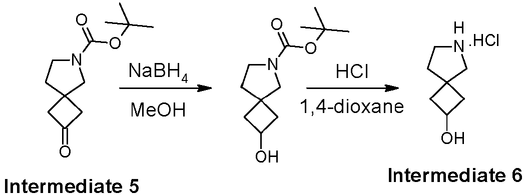

*tert*-Butyl 2-oxo-6-azaspiro[3.4]octane-6-carboxylate **(Intermediate 5)** (10 g, 44.3 mmol), was dissolved in MeOH (1000 mL) and the resulting solution was cooled to 0 °C. Sodium borohydride (2.0 g, 53.2 mmol) was added portion wise and the resulting reaction mixture was stirred at 25 °C for 1 h. The solvents were removed *in-vacuo* and the residue was partitioned between H₂O (250 mL) and EtOAc (250 mL). The aqueous layer was further extracted with EtOAc (2 × 150 mL), and the organic layers were combined, dried (Na₂SO₄), and the solvent was removed *in-vacuo* to give *tert-butyl* 2-hydroxy-6-azaspiro[3.4]octane-6-carboxylate (9.2 g, 91 %) as a gum.

**LCMS (System 3, Method F):** m/z 228 (M+H)⁺ (ESI +ve), at 3.09 min, 202 nm.

*tert*-Butyl 2-hydroxy-6-azaspiro[3.4]octane-6-carboxylate (9 g, 39.6 mmol) was slowly dissolved in HCl solution in 1,4-dioxane (4 M, 90 mL) and the resulting reaction mixture was stirred at 25 °C for 16 h. The solvent was removed *in-vacuo* and the residue was purified by trituration with diethyl ether (3 x 50 mL) to give 6-azaspiro[3.4]octan-2-ol hydrochloride salt **(Intermediate 6)** (7 g, 90 %) as a gum.

The data for **Intermediate 6** are in Table 2.

### Route 3

### Typical procedure for the preparation of alcohols, as exemplified by the preparation of Intermediate 27, 2-azabicyclo[2.2.2]octan-5-ol hydrochloride salt

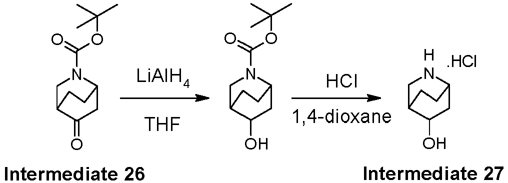

*tert*-Butyl 5-oxo-2-azabicyclo[2.2.2]octane-2-carboxylate **(Intermediate 26)** (500 mg, 2.2 mmol) was dissolved in THF (10 mL) and the solution was cooled to - 20 °C under an atmosphere of nitrogen. Lithium aluminium hydride solution in THF (1 M, 3.3 mL, 3.3 mmol) was added at - 20 °C and the resulting mixture was stirred at 0 °C for 2 h. The reaction mixture was partitioned between cold H₂O (25 mL) and EtOAc (15 mL) and the aqueous layer was further extracted with EtOAc (2 x 15 mL). The combined organic layers were dried (Na₂SO₄) and the solvent was removed *in-vacuo* to give *tert-*butyl 5-hydroxy-2-azabicyclo[2.2.2]octane-2-carboxylate (500 mg, 99 %) as a gum.

**LCMS (System 2, Method E):** m/z 172 (M+H-56)⁺ (ESI +ve), at 1.60 min, 202 nm.

*tert*-Butyl 5-hydroxy-2-azabicyclo[2.2.2]octane-2-carboxylate (500 mg, 2.2 mmol) was dissolved in HCl solution in 1,4-dioxane (4 M, 10 mL) at 0 °C and then stirred at room temperature for 2 h. The solvent was removed *in-vacuo* and the residue was triturated with n-pentane (2 x 10 mL) to give 2-azabicyclo[2.2.2]octan-5-ol hydrochloride salt **(Intermediate 27)** (280 mg, 100 %) as a solid.

The data for **Intermediate 27** are in Table 2.

### Route 4

### Typical procedure for the preparation of alcohols, as exemplified by the preparation of Intermediate 30, 9-azabicyclo[3.3.1]nonan-3-ol trifluoroacetic acid salt

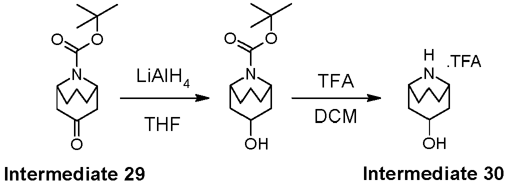

Lithium aluminium hydride solution in THF (1 M, 37.6 mL, 37.6 mmol) was added dropwise at 0 °C to a solution of *tert*-butyl 3-oxo-9-azabicyclo[3.3.1]nonane-9-carboxylate **(Intermediate 29)** (6 g, 25.1 mmol) in THF (60 mL), and the reaction mixture was stirred at 0 °C for 4 h. The reaction was then quenched by the addition of ice-cold water (20 mL), and the mixture was extracted with ethyl acetate (2 × 50 mL). The organic layers were combined, dried (Na₂SO₄), and the solvent was removed *in-vacuo* to give *tert*-butyl 3-hydroxy-9-azabicyclo[3.3.1]nonane-9-carboxylate (6 g, 99 %) as a solid. The product was used crude in the next step without further purification or characterisation.

*tert*-Butyl 3-hydroxy-9-azabicyclo[3.3.1]nonane-9-carboxylate (6 g, 24.1 mmol) was dissolved in DCM (30 mL) and the resulting solution cooled to 0 °C. TFA (15 mL) was added and the mixture was stirred at room temperature for 4 h. The solvent was removed *in-vacuo* and the residue was triturated with toluene (2 x 15 mL) to give 9-azabicyclo[3.3.1]nonan-3-ol trifluoroacetic acid salt **(Intermediate 30)** (8.5 g, 100%) as a gum.

The data for **Intermediate 30** are in Table 2.

### Route 5

### Typical procedure for the preparation of amines, as exemplified by the preparation of Intermediate 47, [(2R,4R)-4-fluoro-1-(piperidin-4-yl)pyrrolidin-2-yl]methanol trifluoroacetic acid salt

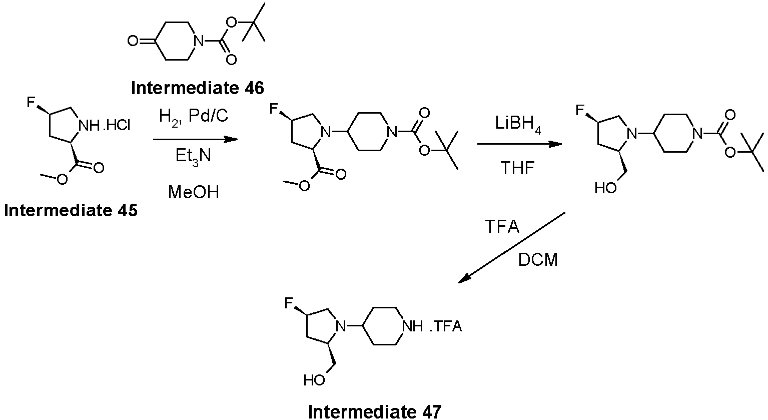

Methyl (2*R*,4*R*)-4-fluoropyrrolidine-2-carboxylate hydrochloride salt **(Intermediate 45)** (11.3 g, 61.7 mmol) was dissolved in methanol (280 mL) and *tert*-butyl 4-oxopiperidine-1-carboxylate **(Intermediate 46)** (12.2 g, 61.7 mmol), triethylamine (13 mL, 92.6 mmol) and 10 % palladium on carbon catalyst (~ 50 % wet with H₂O, 6 g) was added. The reaction mixture was stirred under hydrogen at one atmosphere pressure at 25 °C for 18 h, then filtered through Celite and the filtrate was concentrated *in-vacuo.* The resulting crude product was triturated with pentane to give *tert*-butyl 4-[(2*R*,4*R*)-4-fluoro-2-(methoxycarbonyl)pyrrolidin-1-yl]piperidine-1-carboxylate (18 g, 89 %) as a solid.

**LCMS (System 1, Method D):** m/z 331 (M+H)⁺ (ESI +ve), at 4.23 min, 210 nm.

*tert*-Butyl 4-[(2*R*,4*R*)-4-fluoro-2-(methoxycarbonyl)pyrrolidin-1-yl]piperidine-1-carboxylate (18 g, 54.5 mmol) was dissolved in THF (200 mL) and the resulting solution was cooled to 0 °C. Lithium borohydride solution in THF (3 M, 54 mL, 164 mmol) was then added dropwise, and the mixture was stirred at 25 °C for 17 h. The solvents were removed *in-vacuo* and the residue was basified with saturated NaHCO₃ solution and partitioned between H₂O (600 mL) and DCM (400 mL). The aqueous layer was further extracted with EtOAc (2 × 400 mL), then all the organic layers were combined, dried (Na₂SO₄) and the solvent was removed *in-vacuo.* The residue was purified by trituration with pentane (3 x 10 mL) to give *tert*-butyl 4-[(2*R*,4*R*)-4-fluoro-2-(hydroxymethyl)pyrrolidin-1-yl]piperidine-1-carboxylate (14.5 g, 88 %) as a gum.

**LCMS (System 1, Method D):** m/z 303 (M+H)⁺ (ESI +ve), at 3.57 min, 210 nm.

*tert*-Butyl 4-[(2*R*,4*R*)-4-fluoro-2-(hydroxymethyl)pyrrolidin-1-yl]piperidine-1-carboxylate (14.5 g, 0.42 mmol) was dissolved in DCM (100 mL) and the resulting solution was cooled to 0 °C. Trifluoroacetic acid (30 mL) was added dropwise and the reaction mixture was stirred at 25 °C for 8 h. The solvents were removed *in-vacuo* and the residue was purified by trituration with pentane (3 x 10 mL) to give [(2*R*,4*R*)-4-fluoro-1-(piperidin-4-yl)pyrrolidin-2-yl]methanol trifluoroacetic acid salt **(Intermediate 47)** (9.60 g, 100 %) as a colourless gum.

The data for **Intermediate 47** are in Table 2.

### Route 6

### Typical procedure for the preparation of amines, as exemplified by the preparation of Intermediate 51, 4-[2-(1-methyl-1H-pyrazol-4-yl)pyrrolidin-1-yl]piperidine trifluoroacetic acid salt

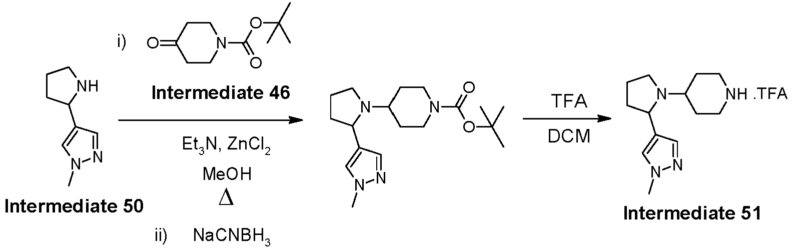

1-Methyl-4-(pyrrolidin-2-yl)-1*H*-pyrazole **(Intermediate** 50) (0.25 g, 1.66 mmol), *tert-*butyl 4-oxopiperidine-1-carboxylate **(Intermediate 46)** (0.32 g, 1.66 mmoL), ZnCl₂ solution in diethyl ether (1 M, 0.083 mL, 0.083 mmol) and triethylamine (0.72 mL, 4.97 mmol) were dissolved in MeOH (10 mL) and the resulting mixture was stirred at 70 °C for 4 h. The mixture was then cooled to 0 °C and NaBH₃CN (0.21 g, 3.31 mmoL) was added portionwise. The reaction mixture was stirred at room temperature for 16 h and then the solvents were removed *in-vacuo.* The residue was partitioned between H₂O (50 mL) and EtOAc (100 mL), and the aqueous layer was further extracted with EtOAc (2 × 100 mL). The combined organic layers were dried (Na₂SO₄) and the solvent was removed *in-vacuo* to give the crude product which was purified by column chromatography using alumina and MeOH/DCM as solvents to give *tert*-butyl 4-(2-(1-methyl-1*H*-pyrazol-4-yl)pyrrolidin-1-yl)piperidine-1-carboxylate (0.33 g, 59 %) as a gum.

**LCMS (System 3, Method F):** m/z 335 (M+H)⁺ (ESI +ve), at 3.23 min, 215 nm.

To a stirred solution of *tert*-butyl 4-(2-(1-methyl-1*H*-pyrazol-4-yl)pyrrolidin-1-yl)piperidine-1-carboxylate (0.30 g, 0.90 mmol) in DCM (3 mL) at 0 °C was added TFA (3 mL) dropwise and the resulting mixture was stirred at room temperature for 3 h. The solvent was evaporated *in-vacuo* and the residue co-evaporated from toluene (3 x 5 mL) to give 4-[2-(1-methyl-1*H*-pyrazol-4-yl)pyrrolidin-1-yl] piperidine trifluoroacetic acid salt **(Intermediate 51)** (0.20 g, 95 %) as a gum.

The data for **Intermediate 51** are in Table 2.

### Route 7

### Typical procedure for the preparation of ketones, as exemplified by the preparation of Intermediate 60, 8-(3-(trifluoromethyl)-1,2,4-oxadiazol-5-yl)-8-azabicyclo[3.2.1]octan-3-one

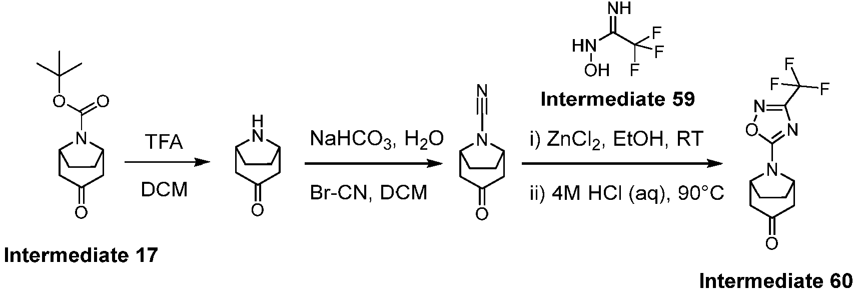

Trifluoroacetic acid (25 mL) was added dropwise at 0 °C to a solution of *tert*-butyl 3-oxo-8-azabicyclo[3.2.1]octane-8-carboxylate **(Intermediate 17)** (10 g, 44.4 mmol) in DCM (100 mL). The resulting mixture was stirred at room temperature for 2 h, then the solvent was evaporated under reduced pressure and the residue was triturated with toluene (3 ×15 mL) to give the crude 8-azabicyclo[3.2.1]octan-3-one trifluoroacetic acid salt (16 g, 100 %), which was carried forward to the next reaction without further purification.

**LCMS (System 4, Method B):** m/z 126 (M+H)⁺ (ESI +ve), at 2.20 min, 214 nm.

A solution of 8-azabicyclo[3.2.1]octan-3-one trifluoroacetic acid salt (16 g, 0.067 mol) in DCM (150 mL) was added dropwise to a solution of sodium bicarbonate (26 g, 0.32 mol) in water (100 mL) with vigorous stirring. A solution of cyanogen bromide (16.3 g, 0.153 mol) in DCM (25 mL) was then added at 0 °C, the ice bath was removed and the resulting reaction mixture was stirred at room temperature for 16 h. Solid sodium carbonate was added until the pH was adjusted to 7, then the mixture was extracted with DCM (3 x 25 mL). The organic phases were dried over anhydrous Na₂SO₄ and concentrated under reduced pressure. The residue was purified by column chromatography (Normal phase, Silica gel 60-120 mesh, 0 to 3 % MeOH in DCM) to afford 3-oxo-8-azabicyclo[3.2.1]octane-8-carbonitrile (6.5 g, 65 %).

**LCMS (System 4, Method B):** m/z 151 (M+H)⁺ (ESI +ve), at 4.13 min, 202 nm.

3-Oxo-8-azabicyclo[3.2.1]octane-8-carbonitrile (1 g, 6.65 mmol) and 2,2,2-trifluoro-*N-*hydroxyacetimidamide **(Intermediate 59)** (1 g, 7.99 mmol) were dissolved in ethanol (50 mL), ZnCl₂ solution in diethyl ether (1 M, 7.99 mL, 7.99 mmol) was added and the resulting mixture was stirred at room temperature for 16 h. Aqueous HCl (4 M, 6 mL) was then added and the mixture was heated at reflux at 90 °C overnight. Saturated sodium bicarbonate solution (50 mL) was added and the mixture was extracted with ethyl acetate (3 ×10 mL). The combined organic layers were dried over anhydrous Na₂SO₄, concentrated under reduced pressure and the residue was purified by column chromatography (Normal phase, Silica gel 60-120 mesh, 0 to 7 % EtOAC in hexanes) to give 8-(3-(trifluoromethyl)-1 ,2,4-oxadiazol-5-yl)-8-azabicyclo[3.2.1]octan-3-one **(Intermediate 60)** (517 mg, 30 %).

The data for **Intermediate 60** are in Table 2.

### Route 8

### Typical procedure for the preparation of amines, as exemplified by the preparation of Intermediate 62, (1R,5S,6r)-N-(-methylcyclobutyl)-3-azabicyclo[3.1.0]hexane-6-carboxamide hydrochloride salt

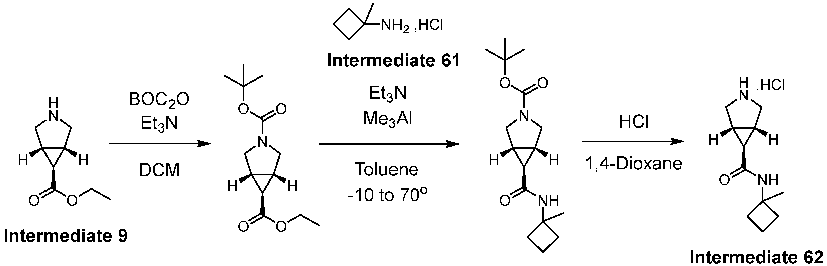

Ethyl (1*R*,5*S*,6*r*)-3-azabicyclo[3.1.0]hexane-6-carboxylate **(Intermediate 9)** (2.0 g, 8.16 mmol) was dissolved in DCM (25 mL) and triethylamine (3.45 mL, 24.5 mmol) was added. The mixture was cooled to 0 °C, stirred for 20 mins and di-*tert*-butyl dicarbonate (2.65 g, 12.2 mmol) was added. The resulting reaction mixture was then stirred at 25 °C for 18 h. The solvents were removed *in-vacuo* and the residue was partitioned between H₂O (80 mL) and ethyl acetate (60 mL). The aqueous layer was further extracted with ethyl acetate (2 × 60 mL), the combined organic layers were dried (Na₂SO₄) and the solvent was removed *in- vacuo.* The residue was purified by column chromatography (Normal-Phase activated Al₂O₃, 0 % to 40 % ethyl acetate in hexanes) to give 3-(*tert*-butyl) 6-ethyl (1*R,*5*S,*6*r*)-3-azabicyclo[3.1.0]hexane-3,6-dicarboxylate (1.70 g, 82 %) as a liquid.

**LCMS (System 3, Method F):** m/z 241 (M-15+H)⁺ (ESI +ve), at 4.06 min, 202 nm.

1-Methylcyclobutan-1-amine hydrochloride **(Intermediate 61)** (948 mg, 7.84 mmol) was suspended in toluene (10 mL) and triethylamine (1.70 mL, 11.8 mmol) was added. The mixture was cooled to -10 °C, trimethylaluminum solution in toluene (2 M, 5.9 mL, 11.8 mmol) was added dropwise and stirring was continued for 20 min. 3-(*tert*-Butyl) 6-ethyl (1*R*,5*S*,6*r*)-3-azabicyclo[3.1.0]hexane-3,6-dicarboxylate (1.0 g, 3.92 mmol) was added at -10 °C, then the reaction mixture was stirred at 70 °C for 40 h. The solvents were removed *in-vacuo* and the residue was partitioned between H₂O (80 mL) and ethyl acetate (60 mL). The aqueous layer was further extracted with ethyl acetate (2 × 80 mL) and the combined organic layers were dried (Na₂SO₄) and the solvent was removed *in-vacuo.* The residue was purified by column chromatography (Normal-Phase activated Al₂O₃, 0 % to 60 % ethyl acetate in hexanes) to give *tert-butyl* (1*R*,5*S*,6*r*)-6-((1-methylcyclobutyl)carbamoyl)-3-azabicyclo[3.1.0]hexane-3-carboxylate (560 mg, 49 %) as a solid.

**LCMS (System 3, Method F):** m/z 293 (M-H)- (ESI -ve), at 3.80 min, 202 nm.

*tert*-Butyl (1*R*,5*S*,6*r*)-6-((1-methylcyclobutyl)carbamoyl)-3-azabicyclo[3.1.0]hexane-3-carboxylate (550 mg, 1.87 mmol) was dissolved in 1,4-dioxane (6 mL) and the solution was cooled to 0 °C. HCl solution in 1,4-dioxane (4 M, 10 mL) was added and the reaction mixture was stirred at 25 °C for 5 h. The solvents were removed *in-vacuo* and the residue was purified by trituration with pentane (3 x 3 mL) to give (1*R*,5*S*,6*r*)-N-(1-methylcyclobutyl)-3-azabicyclo[3.1.0]hexane-6-carboxamide hydrochloride salt **(Intermediate 62)** (408 mg, 95 %) as a solid.

The data for **Intermediate 62** are in Table 2.

### Route 9

### Typical procedure for the preparation of amines, as exemplified by the preparation of Intermediate 68, N-((1-methylcyclobutyl)methyl)piperidine-4-carboxamide trifluoroacetic acid salt

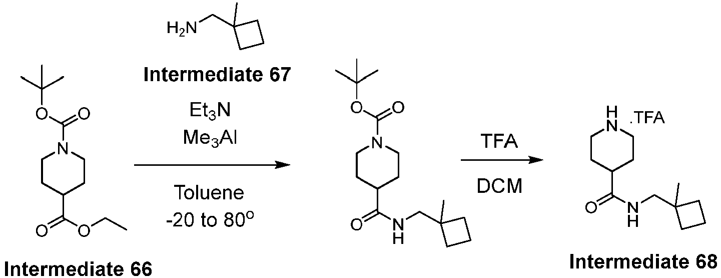

1-(*tert*-Butyl) 4-ethyl piperidine-1,4-dicarboxylate **(Intermediate 64)** (700 mg, 0.0027 mole) and (1-methylcyclobutyl)methanamine **(Intermediate 65)** (434 mg, 0.0032 mole) were dissolved in toluene (3.0 mL) and triethylamine (1.13 mL, 0.008 mole) was added, followed by trimethylaluminum solution in toluene (2 M, 5.44 mL, 0.0108 mole) added dropwise at -20 °C. The mixture was stirred for 30 min at -20 °C and then heated at 80 °C for 48 hrs. The reaction mixture was concentrated under reduced pressure and the residue was partitioned between cold H₂O (200 mL) and DCM (100 mL). The aqueous layer was further extracted with DCM (2 x 100 mL) and the combined organic layers were dried (Na₂SO₄) and the solvent was removed *in-vacuo.* The crude product was purified by column chromatography (Normal phase, 100-120 mesh silica gel, 0 to 39 % ethyl acetate in hexanes) to give *tert*-butyl 4-(((1-methylcyclobutyl)methyl)carbamoyl)piperidine-1-carboxylate (320 mg, 38 %) as a solid.

**LCMS (System 2, Method H):** m/z 311 (M+H)⁺ (ESI +ve), at 1.71 min, 202 nm.

A solution of *tert*-butyl 4-(((1-methylcyclobutyl)methyl)carbamoyl)piperidine-1-carboxylate (320 mg, 0.00103 mol) in DCM (2 mL) was cooled to 0°C and TFA (1 mL) was added dropwise, then the resulting reaction mixture was stirred at RT for 2 h. The solvent was removed *in-vacuo* and the residue was purified by trituration with toluene (3 x 5 mL) to give the crude *N-*((1-methylcyclobutyl)methyl)piperidine-4-carboxamide trifluoroacetic acid salt **(Intermediate 66)** (410 mg, 100 %), which was used without further purification.

The data for **Intermediate 66** are in Table 2.

### General Synthetic Procedures:

### Route A

### Typical procedure for the preparation of amines as exemplified by the preparation of Example 1-1, 2-(3-methyl-1,2,4-oxadiazol-5-yl)-6-[4-(1H-pyrazol-1-yl)piperidin-1-yl]-2-azaspiro[3.3]heptane

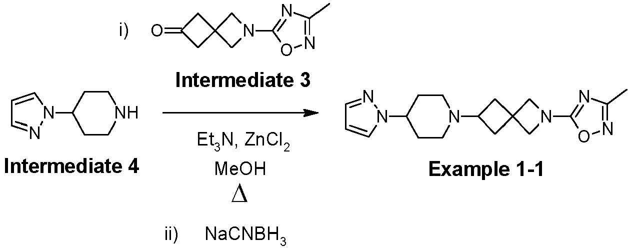

4-(1*H*-Pyrazol-1-yl)piperidine **(Intermediate 4)** (47 mg, 0.31 mmol), 2-(3-methyl-1,2,4-oxadiazol-5-yl)-2-azaspiro[3.3]heptan-6-one **(Intermediate 3)** (60 mg, 0.31 mmol), triethylamine (0.1 mL, 0.93 mmol) and ZnCl₂ (4 mg, 0.03 mmol) were dissolved in MeOH (10 mL) and the resulting mixture was stirred at 65 °C for 8 h. The mixture was then cooled to 0 °C and NaBH₃CN (58 mg, 0.93 mmol) was added portion wise and the resulting reaction mixture was stirred at 25 °C for 16 h. The solvents were removed *in-vacuo,* the residue was partitioned between H₂O (50 mL) and EtOAc (40 mL), and the aqueous layer was further extracted with EtOAc (2 × 40 mL). The combined organic layers were dried (Na₂SO₄), the solvent was removed *in-vacuo,* and the residue was purified using Purification Method A to give 2-(3-methyl-1,2,4-oxadiazol-5-yl)-6-[4-(1*H*-pyrazol-1-yl)piperidin-1-yl]-2-azaspiro[3.3]heptane **(Example 1-1)** (17 mg, 17 %) as a solid.

The data for **Example 1-1** are in Table 3.

### Route B

### Typical procedure for the preparation of amines as exemplified by the preparation of Example 6-5, N-(1-methylcyclobutyl)-1-[8-(3-methyl-1,2,4-oxadiazol-5-yl)-8-azabicyclo[3.2.1]oct-3-yl]piperidine-4-carboxamide

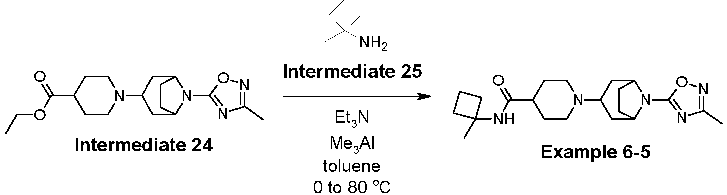

Triethylamine (0.12 ml, 0.86 mmol) was added dropwise to a solution of 1-methylcyclobutan-1-amine **(Intermediate 25)** (27 mg, 0.3 mmol) in toluene (10 mL) and the mixture was stirred at room temperature for 15 min. Ethyl 1-[8-(3-methyl-1,2,4-oxadiazol-5-yl)-8-azabicyclo[3.2.1]oct-3-yl]piperidine-4-carboxylate **(Intermediate 24)** (100 mg, 0.29 mmol) was added and the reaction mixture was stirred at room temperature for 15 min and then cooled to 0 °C. Trimethylaluminium solution in toluene (2 M, 0.43 mL, 0.86 mmol) was added at 0 °C, and then the reaction mixture was stirred at 80 °C for 16 h. The mixture was partitioned between H₂O (10 mL) and ethyl acetate (20 mL), and the aqueous layer was further extracted with ethyl acetate (2 x 20 mL). The combined organic layers were dried (Na₂SO₄) and the solvent was removed *in-vacuo* to give the crude product, which was purified by Purification Method L to give *N*-(1-methylcyclobutyl)-1-[8-(3-methyl-1,2,4-oxadiazol-5-yl)-8-azabicyclo[3.2.1]oct-3-yl]piperidine-4-carboxamide, **Example 6-5 Isomer 1** (40 mg, 24 %) as a gum and *N*-(1-methylcyclobutyl)-1-[8-(3-methyl-1,2,4-oxadiazol-5-yl)-8-azabicyclo[3.2.1]oct-3-yl]piperidine-4-carboxamide, **Example 6-5 Isomer 2** (5 mg, 3 %) as a solid.

The data for **Example 6-5 Isomer 2** are in Table 3.

### Route C

### Typical procedure for the preparation of amines as exemplified by the preparation of Example 10-12, (2S)-N-methyl-2-{1-[1-(3-methyl-1,2,4-oxadiazol-5-yl)azepan-4-yl]piperidin-4-yl}pyrrolidine-1-carboxamide

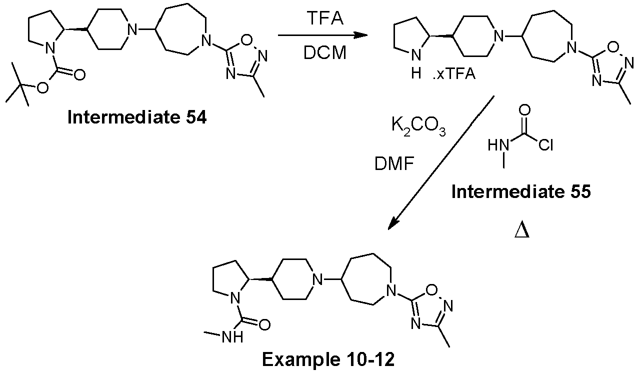

To a stirred solution of *tert-butyl* (2S)-2-{1-[1-(3-methyl-1,2,4-oxadiazol-5-yl)azepan-4-yl]piperidin-4-yl}pyrrolidine-1-carboxylate **(Intermediate 54)** (260 mg, 0.60 mmol) in DCM (3 mL) was added TFA (3 mL) dropwise at 0 °C. The reaction mixture was stirred at room temperature for 3 h, then the solvent was evaporated *in-vacuo* and the residue co-evaporated from toluene (3 x 5 mL) to give 1-(3-methyl-1,2,4-oxadiazol-5-yl)-4-{4-[(2*S*)-pyrrolidin-2-yl]piperidin-1-yl}azepane trifluoroacetic acid salt (190 mg, 95 %) as a gum.

**LCMS (System 3, Method F):** m/z 334 (M+H)⁺ (ESI +ve), at 1.95 min, 225 nm.

To a stirred solution of 1-(3-methyl-1,2,4-oxadiazol-5-yl)-4-{4-[(2S)-pyrrolidin-2-yl]piperidin-1-yl}azepane trifluoroacetic acid salt (300 mg, 1.29 mmol) in DMF (3 mL) was added K₂CO₃ (533 mg, 3.86 mmol) and the resulting mixture was stirred at room temperature for 1 h. Methylcarbamyl chloride **(Intermediate 55)** (240 mg, 2.58 mmol) was added and the reaction mixture was stirred at 80 °C for 16 h. The reaction mixture was then diluted with ice-cold water (10 mL) and extracted with EtOAc (20 mL). The aqueous layer was further extracted with EtOAc (2 × 10 mL), then all the organic layers were combined, dried (Na₂SO₄) and evaporated under reduced pressure to give the crude product, which was purified by Purification Method AQ followed by Purification Method AR to give (2*S*)-*N-*methyl-2-{1-[1-(3-methyl-1,2,4-oxadiazol-5-yl)azepan-4-yl]piperidin-4-yl}pyrrolidine-1-carboxamide, **Example 10-12 Isomer 1** (11 mg, 2 %) as a gum and (2S)-N-methyl-2-{1-[1-(3-methyl-1,2,4-oxadiazol-5-yl)azepan-4-yl]piperidin-4-yl}pyrrolidine-1-carboxamide, **Example 10-12 Isomer 2** (10 mg, 2 %) as a gum.

The data for **Example 10-12 Isomer 1** and **Example 10-12 Isomer 2** are in Table 3.

**Table 2 - Starting Materials and Intermediates**

| **Table 2** | | | | |
|---|---|---|---|---|
| **Intermediate Number** | **Name** | **Synthetic Route** | **Intermediates Used** | **Data** |
| **1** | *tert*-Butyl 6-oxo-2-azaspiro[3.3]heptane-2-carboxylate | - | - | Commercially available, CAS: 1181816-12-5 |
| **2** | *N*-Hydroxyacetamide | - | - | Commercially available, CAS: 546-88-3 |
| **3** | 2-(3-Methyl-1,2,4-oxadiazol-5-yl)-2-azaspiro[3.3]heptan-6-one | **1** | **1 and 2** | **LCMS (System 2, Method E):** m/z 194 (M+H)⁺ (ES⁺), at 1.36 min, 226 nm |
| **4** | 4-(1*H*-Pyrazol-1-yl)piperidine | - | - | Commercially available, CAS: 762240-09-5 |
| **5** | *tert*-Butyl 2-oxo-6-azaspiro[3.4]octane-6-carboxylate | - | - | Commercially available, CAS: 203661-71-6 |
| **6** | 6-Azaspiro[3.4]octan-2-ol hydrochloride salt | **2** | **5** | **¹H NMR** (400 MHz, DMSO-*d*₆) δ 9.42 (br. s, 1H), 9.31 (br. s, 1H), 4.13 - 4.00 (m, 1H), 3.14 - 2.97 (m, 4H), 2.36 - 2.25 (m, 1H), 2.23 - 2.12 (m, 1H), 1.93 - 1.77 (m, 4H). |
| | | | | One exchangeable proton not observed. |
| **7** | 6-(3-Methyl-1,2,4-oxadiazol-5-yl)-6-azaspiro[3.4]octan-2-one | **1 (from Step 3)** | **6 and 2** | **LCMS (System 3, Method F):** m/z 208 (M+H)⁺ (ES⁺), at 2.25 min, 202 nm |
| **8** | 4-Phenylpiperidine-4-carbonitrile | - | - | Commercially available, CAS: 40481-13-8 |
| **9** | Ethyl (1*R*,5*S*,6*r*)-3-azabicyclo[3.1.0]hexane-6-carboxylate | - | - | Commercially available, CAS: 174456-77-0 |
| **10** | *tert*-Butyl 6-oxo-2-azaspiro[3.4]octane-2-carboxylate | - | - | Commercially available, CAS: 1363382-39-1 |
| **11** | 2-Azaspiro[3.4]octan-6-ol hydrochloride salt | **2** | **10** | **¹H NMR** (400 MHz, DMSO-*d*₆) δ 9.07 (br. s, 1H), 8.12 (br. s, 1H), 4.12 - 4.03 (m, 1H), 3.93 - 3.65 (m, 4H), 3.00 - 2.87 (m, 1H), 2.10 - 1.94 (m, 1H), 1.84 - 1.66 (m, 3H), 1.64 - 1.40 (m, 2H). |
| **12** | 2-(3-Methyl-1,2,4-oxadiazol-5-yl)-2-azaspiro[3.4]octan-6-one | **1 (from Step 3)** | **11 and 2** | **LCMS (System 2, Method E):** m/z 208 (M+H)⁺ (ES⁺), at 1.39 min, 230 nm |
| **13** | Piperidin-4-ol | - | - | Commercially available, CAS: 5382-16-1 |
| **14** | 1-(3-Methyl-1,2,4-oxadiazol-5-yl)piperidin-4-one | **1 (from Step 3)** | **13 and 2** | **LCMS (System 2, Method E):** m/z 182 (M+H)⁺ (ES⁺), at 1.31 min, 228 nm |
| **15** | *tert*-Butyl 6-oxo-3-azabicyclo[3.1.1]heptane-3-carboxylate | - | - | Commercially available, CAS: 1251013-26-9 |
| **16** | 3-(3-Methyl-1,2,4-oxadiazol-5-yl)-3-azabicyclo[3.1.1]heptan-6-one | **1** | **15 and 2** | **LCMS (System 4, Method B):** m/z 194 (M+H)⁺ (ES⁺), at 3.91 min, 202 nm |
| **17** | *tert*-Butyl 3-oxo-8-azabicyclo[3.2.1]octane-8-carboxylate | - | - | Commercially available, CAS: 185099-67-6 |
| **18** | 8-(3-Methyl-1,2,4-oxadiazol-5-yl)-8-azabicyclo[3.2.1]octan-3-one | **1** | **17 and 2** | **LCMS (System 3, Method F):** m/z 208 (M+H)⁺ (ES⁺), at 2.33 min, 202 nm |
| **19** | 2,8-Diazaspiro[4.5]decan-3-one | - | - | Commercially available, CAS: 561314-57-6 |
| **20** | *N*-Hydroxypropanamide | - | - | Commercially available, CAS: 2580-63-4 |
| **21** | 8-(3-Ethyl-1,2,4-oxadiazol-5-yl)-8-azabicyclo[3.2.1]octan-3-one | **1** | **17 and 20** | **LCMS (System 3, Method F):** m/z 222 (M+H)⁺ (ES⁺), at 2.67 min, 202 nm |
| **22** | 1-Oxa-3,8-diazaspiro[4.5]decan-2-one | - | - | Commercially available, CAS: 5052-95-9 |
| **23** | Ethyl piperidine-4-carboxylate | - | - | Commercially available, CAS: 1126-09-6 |
| **24** | Ethyl 1-[8-(3-methyl-1,2,4-oxadiazol-5-yl)-8-azabicyclo[3.2.1]oct-3-yl]piperidine-4-carboxylate | **A** | **23 and 18** | **LCMS (System 3, Method F):** m/z 349 (M+H)⁺ (ES⁺), at 3.21 min, 202 nm |
| **25** | 1-Methylcyclobutan-1-amine | - | - | Commercially available, CAS: 40571-47-9 |
| **26** | *tert*-Butyl 5-oxo-2-azabicyclo[2.2.2]octane-2-carboxylate | - | - | Commercially available, CAS: 617714-22-4 |
| **27** | 2-Azabicyclo[2.2.2]octan-5-ol hydrochloride salt | **3** | **26** | **LCMS (System 4, Method C):** m/z 128 (M+H)⁺ (ES⁺), at 2.56 and 3.66 min, no UV absorption at 202 nm |
| **28** | 2-(3-Methyl-1,2,4-oxadiazol-5-yl)-2-azabicyclo[2.2.2]octan-5-one | **1 (from Step 3)** | **27 and 2** | **LCMS (System 3, Method F):** m/z 208 (M+H)⁺ (ES⁺), at 2.27 min, 202 nm |
| **29** | *tert*-Butyl 3-oxo-9-azabicyclo[3.3.1]nonane-9-carboxylate | - | - | Commercially available, CAS: 512822-27-4 |
| **30** | 9-Azabicyclo[3.3.1] non an-3-ol trifluoroacetic acid salt | **4** | **29** | **LCMS (System 4, Method B):** m/z 142 (M+H)⁺ (ES⁺), at 1.82 min, 202 nm |
| **31** | 9-(3-Methyl-1,2,4-oxadiazol-5-yl)-9-azabicyclo[3.3.1]nonan-3-one | **1 (from Step 3)** | **30 and 2** | **LCMS (System 3, Method F):** m/z 222 (M+H)⁺ (ES⁺), at 2.46 min, 202 nm |
| **32** | Ethyl 1-[9-(3-methyl-1,2,4-oxadiazol-5-yl)-9-azabicyclo[3.3.1]non-3-yl]piperidine-4-carboxylate | **A** | **23 and 31** | **LCMS (System 3, Method F):** m/z 363 (M+H)⁺ (ES⁺), at 3.08 min, 202 nm |
| **33** | *tert*-Butyl 7-oxo-3-oxa-9-azabicyclo[3.3.1]nonane-9-carboxylate | - | - | Commercially available, CAS: 280761-97-9 |
| **34** | 3-Oxa-9-azabicyclo[3.3.1]nonan-7-ol hydrochloride salt | **3** | **33** | **¹H NMR** (400 MHz, DMSO-*d*₆) δ 9.40 (br. s, 1H), 9.08 (br. s, 1H), 4.98 - 4.86 (m, 1H), 4.02 - 3.79 (m, 5H), 3.61 - 3.52 (m, 2H), 2.37 - 2.25 (m, 2H), 1.85 - 1.75 (m, 2H). |
| **35** | 9-(3-Methyl-1,2,4-oxadiazol-5-yl)-3-oxa-9-azabicyclo[3.3.1]nonan-7-one | **1 (from Step 3)** | **34 and 2** | **LCMS (System 3, Method F):** m/z 224 (M+H)⁺ (ES⁺), at 1.84 min, 202 nm |
| **36** | Methyl (1*R*,5*S*,6*r*)-3-azabicyclo[3.1.0]hexane-6-carboxylate | - | - | Commercially available, CAS: 1024038-72-9 |
| **37** | Methyl (1*R*,5*S*,6*r*)-3-[9-(3-methyl-1,2,4-oxadiazol-5-yl)-3-oxa-9-azabicyclo[3.3.1] nonan-7-yl]-3-azabicyclo [3.1.0]hexane-6-carboxylate | **A** | **36 and 35** | **LCMS (System 3, Method F):** m/z 349 (M+H)⁺ (ES⁺), at 2.90 min, 202 nm |
| **38** | *N*-Ethylethanamine | - | - | Commercially available, CAS: 109-89-7 |
| **39** | *tert*-Butyl 4-oxoazepane-1-carboxylate | - | - | Commercially available, CAS: 188975-88-4 |
| **40** | Azepan-4-ol hydrochloride salt | **2** | **39** | **¹H NMR** (400 MHz, DMSO-*d*₆) δ 9.18 - 8.88 (m, 2H), 3.90 - 3.57 (m, 2H), 3.21 - 3.07 (m, 1H), 3.07 - 2.87 (m, 3H), 1.98 - 1.87 (m, 1H), 1.87 - 1.68 (m, 3H), 1.68 - 1.53 (m, 2H). |
| **41** | 1-(3-Methyl-1,2,4-oxadiazol-5-yl)azepan-4-one | **1 (from Step 3)** | **40 and 2** | **LCMS (System 4, Method B):** m/z 196 (M+H)⁺ (ES⁺), at 3.34 min, 202 nm |
| **42** | 4-Ethyl-1-oxa-3,8-diazaspiro[4.5]decan-2-one | - | - | **See** WO2016147011 |
| **43** | 4,4-Dimethyl-1-oxa-3,8-diazaspiro[4.5]decan-2-one | - | - | **See** WO2016147011 |
| **44** | Spiro[indole-3,4'-piperidin]-2(1*H*)-one | - | - | Commercially available, CAS: 252882-61-4 |
| **45** | Methyl (2*R*,4*R*)-4-fluoropyrrolidine-2-carboxylate hydrochloride salt | - | - | Commercially available, CAS: 1445948-46-8 |
| **46** | *tert*-Butyl 4-oxopiperidine-1-carboxylate | - | - | Commercially available, CAS: 79099-07-3 |
| **47** | [(2*R*,4*R*)-4-Fluoro-1-(piperidin-4-yl)pyrrolidin-2-yl]methanol trifluoroacetic acid salt | **5** | **45 and 46** | **LCMS (System 4, Method B):** m/z 203 (M+H)⁺ (ES⁺), at 2.10 min, 202 nm |
| **48** | [(2*R*)-4,4-Difluoro-1-(piperidin-4-yl)pyrrolidin-2-yl]methanol | - | - | **See** WO2017021728 |
| **49** | (5*S*)-5-Ethyl-1-(piperidin-4-yl)pyrrolidin-2-one | - | - | **See** WO2017021728 |
| **50** | 1-Methyl-4-(pyrrolidin-2-yl)-1*H*-pyrazole | - | - | Commercially available, CAS: 1170640-87-5 |
| **51** | 4-[2-(1-Methyl-1*H*-pyrazol-4-yl)pyrrolidin-1-yl]piperidine | **6** | **50 and 46** | **LCMS (System 2, Method A):** m/z 235 (M+H)⁺ (ES⁺), at 0.27 min, 202 nm |
| **52** | *N*-Ethyl-*N*-(piperidin-4-yl)acetamide | - | - | Commercially available, CAS: 139062-99-0 **Also see** WO2017021730 |
| **53** | *tert*-Butyl (2*S*)-2-(piperidin-4-yl)pyrrolidine-1-carboxylate | - | - | Commercially available, CAS: 1449131-15-0 |
| **54** | *tert*-Butyl (2*S*)-2-{1-[1-(3-methyl-1,2,4-oxadiazol-5-yl)azepan-4-yl]piperidin-4-yl}pyrrolidine-1-carboxylate | **A** | **54 and 41** | **LCMS (System 3, Method F):** m/z 434 (M+H)⁺ (ES⁺), at 3.69 min, 202 nm |
| **55** | Methylcarbamyl chloride | - | - | Commercially available, CAS: 6452-47-7 |
| **56** | *tert*-Butyl 3-oxo-6-azabicyclo[3.2.1]octane-6-carboxylate | - | - | Commercially available, CAS: 359779-74-1 |
| **57** | 6-Azabicyclo[3.2.1]octan-3-ol trifluoroacetic acid salt | **4** | **56** | **LCMS (System 4, Method B):** m/z 128 (M+H)⁺ (ES⁺), at 1.46 min, 202 nm |
| **58** | 6-(3-Methyl-1,2,4-oxadiazol-5-yl)-6-azabicyclo[3.2.1]octan-3-one | **1 (from Step 3)** | **56 and 2** | **LCMS (System 4, Method B):** m/z 208 (M+H)⁺ (ES⁺), at 3.88 min, 202 nm |
| **59** | 2,2,2-Trifluoro-*N-*hydroxyacetimidamide | - | - | Commercially available, CAS: 4314-35-6 |
| **60** | 8-(3-(Trifluoromethyl)-1,2,4-oxadiazol-5-yl)-8-azabicyclo[3.2.1]octan-3-one | **7** | **17 and 59** | **1H NMR** (400 MHz, DMSO-*d*₆) δ 4.72 - 4.62 (m, 2H), 2.89 - 2.77 (m, 2H), 2.39 - 2.28 (m, 2H), 2.23 - 2.09 (m, 2H), 1.79 - 1.67 (m, 2H). |
| **61** | 1-Methylcyclobutan-1-amine hydrochloride | - | - | Commercially available, CAS: 174886-05-6 |
| **62** | (1*R,*5*S*,6*r*)-*N*-(1-methylcyclobutyl)-3-azabicyclo[3.1.0]hexane-6-carboxamide hydrochloride salt | **8** | **9 and 61** | **LCMS (System 3, Method F):** m/z 193 (M-H)⁻ (ES), at 1.92 min, 202 nm |
| **63** | 1-(Trifluoromethyl)cyclobutan -1-amine hydrochloride | - | - | Commercially available, CAS: 1260768-75-9 |
| **64** | 1-Ethylcyclobutan-1-amine hydrochloride | - | - | Commercially available, CAS: 279215-56-4 |
| **65** | 1-Methylcyclopentan-1-amine hydrochloride | - | - | Commercially available, CAS: 102014-58-4 |
| **66** | 1-(*tert*-Butyl) 4-ethyl piperidine-1,4-dicarboxylate | - | - | Commercially available, CAS: 142851-03-4 |
| **67** | (1-Methylcyclobutyl)methana mine | - | - | Commercially available, CAS: 933722-69-1 |
| **68** | *N-((1-*Methylcyclobutyl)methyl)pip eridine-4-carboxamide trifluoroacetic acid salt | **9** | **66 and 67** | **LCMS (System 3, Method F):** m/z 211 (M+H)⁺ (ES⁺), at 2.39 min, 202 nm |
| **69** | Ethyl 1-(8-(3-(trifluoromethyl)-1,2,4-oxadiazol-5-yl)-8-azabicyclo[3.2.1]octan-3-yl)piperidine-4-carboxylate | **A** | **23 and 60** | **LCMS (System 3, Method F):** m/z 403 (M+H)⁺ (ES⁺), at 4.06 min, 239 nm |

**Table 3 - NMR and LCMS properties and the methods used to prepare and purify compounds represented by Examples 1 - 12-14**

| **Table 3** | | | | | | |
|---|---|---|---|---|---|---|
| **Ex. No.** | **Name** | **Synthetic Method and Intermediates Used** | **Purification Method** | **¹H NMR** | **LCMS System and Method** | **LCMS data** |
| **1-1** | 2-(3-Methyl-1,2,4-oxadiazol-5-yl)-6-[4-(1*H-*pyrazol-1-yl)piperidin-1-yl]-2-azaspiro[3.3]heptane | **A** | **A** | ¹H NMR (400 MHz, Methanol-*d*₄) δ 7.67 (d, *J* = 2 Hz, 1H), 7.48 (d, *J* = 2 Hz, 1H), 6.29 (t, *J* = 2 Hz, 1H), 4.25 (s, 2H), 4.23 - 4.15 (m, 1H), 4.14 (s, 2H), 3.03 - 2.93 (m, 2H), 2.81 - 2.68 (m, 1H), 2.51 - 2.41 (m, 2H), 2.16 (s, 3H), 2.21 - 1.94 (m, 8H). | **System 3** | m/z 329 (M+H)⁺ (ES⁺), at 2.76 min, 202 nm |
| | | **3 and 4** | | | **Method F** | |
| **2-1** | Isomer 2: 6-(3-Methyl-1,2,4-oxadiazol-5-yl)-2-[4-(1*H*-pyrazol-1-yl)piperidin-1-yl]-6-azaspiro[3.4]octane | **A** | **B** | ¹H NMR (400 MHz, Methanol-*d*₄) δ 7.68 (d, *J* = 2 Hz, 1H), 7.49 (d, *J* = 2 Hz, 1H), 6.29 (t, *J* = 2 Hz, 1H), 4.27 - 4.15 (m, 1H), 3.59 (t, *J* = 6.8 Hz, 2H), 3.47 (s, 2H), 3.06 - 2.99 (m, 2H), 2.94 - 2.83 (m, 1H), 2.24 - 2.18 (m, 2H), 2.17 (s, 3H), 2.14 - 2.07 (m, 4H), 2.06 - 1.96 (m, 6H). | **System 3** | m/z 343 (M+H)⁺ (ES⁺), at 2.82 min, 202 nm |
| | | **4 and 7** | | | **Method F** | |
| **2-2** | Isomer 2: 1-[6-(3-Methyl-1,2,4-oxadiazol-5-yl)-6-azaspiro[3.4]oct-2-yl]-4-phenylpiperidine-4-carbonitrile | **A** | **C** | ¹H NMR (400 MHz, Methanol-*d*₄) δ 7.58 - 7.50 (m, 2H), 7.48 - 7.39 (m, 2H), 7.40 - 7.31 (m, 1H), 3.60 (t, *J* = 6.8 Hz, 2H), 3.47 (s, 2H), 3.06 (d, *J* = 12.2 Hz, 2H), 3.03 - 2.90 (m, 1H), 2.36 - 2.19 (m, 4H), 2.17 (s, 3H), 2.19 - 2.06 (m, 6H), 2.06 - 1.97 (m, 2H). | **System 3** | m/z 378 (M+H)⁺ (ES⁺), at 3.84 min, 202 nm |
| | | **7 and 8** | | | **Method F** | |
| **2-3** | Isomer 2: Ethyl (1*R*,5*S*,6*r*)-3-[6-(3-methyl-1,2,4-oxadiazol-5-yl)-6-azaspiro[3.4]oct-2-yl]-3-azabicyclo[3.1.0]hexane-6-carboxylate | **A** | **D** | ¹H NMR (400 MHz, Methanol-*d*₄) δ 4.10 (q, *J* = 7.1 Hz, 2H), 3.59 - 3.50 (m, 2H), 3.44 (s, 2H), 3.09 - 3.00 (m, 3H), 2.44 - 2.36 (m, 2H), 2.16 (s, 3H), 2.13 - 2.02 (m, 3H), 2.02 - 1.92 (m, 6H), 1.24 (t, *J* = 7.1 Hz, 3H). | **System 3** | m/z 347 (M+H)⁺ (ES⁺), at 3.52 min, 202 nm |
| | | **7 and 9** | | | **Method F** | |
| **3-1** | Isomer 2: 2-(3-Methyl-1,2,4-oxadiazol-5-yl)-6-[4-(1*H*-pyrazol-1-yl)piperidin-1-yl]-2-azaspiro[3.4]octane | **A** | **E** | ¹H NMR (400 MHz, Methanol-*d*₄) δ 7.68 (d, *J* = 2 Hz, 1H), 7.48 (d, *J* = 2 Hz, 1H), 6.29 (t, *J* = 2 Hz, 1H), 4.26 - 4.12 (m, 2H), 4.12 - 3.99 (m, 3H), 3.15 (d, *J* = 11.5 Hz, 2H), 2.77 - 2.68 (m, 1H), 2.33 - 2.18 (m, 2H), 2.16 (s, 3H), 2.15 - 1.91 (m, 8H), 1.87 - 1.76 (m, 1H), 1.66 - 1.53 (m, 1H). | **System 3** | m/z 343 (M+H)⁺ (ES⁺), at 2.72 min, 202 nm |
| | | **4 and 12** | | | **Method F** | |
| **4-1 (*)** | 1'-(3-Methyl-1,2,4-oxadiazol-5-yl)-4-(1*H-*pyrazol-1-yl)-1,4'-bipiperidine | **A** | **F** | ¹H NMR (400 MHz, Methanol-*d*₄) δ 7.68 (d, *J* = 2 Hz, 1H), 7.48 (d, *J* = 2 Hz, 1H), 6.29 (t, *J* = 2 Hz, 1H), 4.21 - 4.10 (m, 3H), 3.22 - 3.08 (m, 4H), 2.72 - 2.67 (m, 1H), 2.53 - 2.42 (m, 2H), 2.17 (s, 3H), 2.16 - 2.09 (m, 2H), 2.08 - 1.95 (m, 4H), 1.68 - 1.55 (m, 2H). | **System 3** | m/z 317 (M+H)⁺ (ES⁺), at 2.60 min, 202 nm |
| | | **4 and 14** | | | **Method F** | |
| **5-1** | Isomer 1: 3-(3-Methyl-1,2,4-oxadiazol-5-yl)-6-[4-(1*H*-pyrazol-1-yl)piperidin-1-yl]-3-azabicyclo[3.1.1]heptane | **A** | **G** | ¹H NMR (400 MHz, Methanol-*d*₄) δ 7.70 (d, *J* = 2 Hz, 1H), 7.49 (d, *J =* 2 Hz, 1H), 6.29 (t, *J* = 2 Hz, 1H), 4.28 - 4.16 (m, 1H), 3.89 (d, *J* = 10.9 Hz, 2H), 3.72 (d, *J* = 10.9 Hz, 2H), 3.38 - 3.21 (m, 2H), 2.72 - 2.63 (m, 1H), 2.57 (d, *J* = 6.4 Hz, 2H), 2.41 (d, *J* = 5.2 Hz, 1H), 2.20 (s, 3H), 2.16 - 2.07 (m, 4H), 2.04 (d, *J* = 10.9 Hz, 1H), 1.56 - 1.49 (m, 1H), 1.31 - 1.26 (m, 1H). | **System 3** | m/z 329 (M+H)⁺ (ES⁺), at 2.88 min, 202 nm |
| | | **4 and 16** | | | **Method F** | |
| **6-1** | Isomer 1: 8-(3-Methyl-1,2,4-oxadiazol-5-yl)-3-[4-(1*H*-pyrazol-1-yl)piperidin-1-yl]-8-azabicyclo[3.2.1]octane | **A** | **H** | ¹H NMR (400 MHz, Methanol-*d*₄) δ 7.65 (d, *J* = 2 Hz, 1H), 7.47 (d, *J* = 2 Hz, 1H), 6.27 (t, *J* = 2 Hz, 1H), 4.46 - 4.40 (m, 2H), 4.20 - 4.10 (m, 1H), 3.08 (d, *J* = 11.8 Hz, 2H), 3.00 - 2.89 (m, 1H), 2.34 - 2.23 (m, 2H), 2.20 (s, 3H), 2.15 - 2.04 (m, 4H), 2.02 - 1.93 (m, 4H), 1.93 - 1.86 (m, 2H), 1.76 - 1.66 (m, 2H). | **System 3** | m/z 343 (M+H)⁺ (ES⁺), at 2.75 min, 202 nm |
| | | **4 and 18** | | | **Method F** | |
| **6-2** | Isomer 1: 8-[8-(3-Methyl-1,2,4-oxadiazol-5-yl)-8-azabicyclo[3.2.1]oct-3-yl]-2,8-diazaspiro[4.5]decan-3-one | **A** | **I** | ¹H NMR (400 MHz, Methanol-*d*₄) δ 4.47-4.35 (m, 2H), 3.17 (s, 2H), 2.91 - 2.78 (m, 1H), 2.66 - 2.34 (m, 4H), 2.24 - 2.15 (m, 6H), 2.14 - 2.04 (m, 2H), 2.01 - 1.91 (m, 2H), 1.91 - 1.80 (m, 2H), 1.76 - 1.53 (m, 5H). One exchangeable proton not observed. | **System 3** | m/z 346 (M+H)⁺ (ES⁺), at 2.14 min, 202 nm |
| | | **18 and 19** | | | **Method F** | |
| **6-2** | Isomer 2: 8-[8-(3-Methyl-1,2,4-oxadiazol-5-yl)-8-azabicyclo[3.2.1]oct-3-yl]-2,8-diazaspiro[4.5]decan-3-one | **A** | **I** | ¹H NMR (400 MHz, Methanol-*d*₄) δ 4.40 - 4.27 (m, 2H), 3.19 (s, 2H), 2.74 - 2.41 (m, 4H), 2.40 - 2.29 (m, 1H), 2.28 - 2.13 (m, 7H), 2.11 - 1.98 (m, 4H), 1.92 - 1.80 (m, 2H), 1.78 - 1.62 (m, 4H). One exchangeable proton not observed. | **System 3** | m/z 346 (M+H)⁺ (ES⁺), at 2.25 min, 202 nm |
| | | **18 and 19** | | | **Method F** | |
| **6-3** | Isomer 2: 8-[8-(3-Ethyl-1,2,4-oxadiazol-5-yl)-8-azabicyclo[3.2.1]oct-3-yl]-2,8-diazaspiro[4.5]decan-3-one | **A** | **J** | ¹H NMR (400 MHz, Methanol-*d*₄) δ 4.38 - 4.31 (m, 2H), 3.18 (s, 2H), 2.56 (q, J = 7.6 Hz, 2H), 2.57 - 2.39 (m, 4H), 2.32 - 2.22 (m, 1H), 2.24 - 2.13 (m, 5H), 2.13 - 1.98 (m, 2H), 1.88 (dd, J = 14.5, 5.6 Hz, 2H), 1.74 - 1.59 (m, 5H), 1.25 (t, J = 7.6 Hz, 3H). One exchangeable proton not observed. | **System 3** | m/z 360 (M+H)⁺ (ES⁺), at 2.56 min, 202 nm |
| | | **19 and 21** | | | **Method F** | |
| **6-4** | Isomer 1: 8-[8-(3-Methyl-1,2,4-oxadiazol-5-yl)-8-azabicyclo[3.2.1]octan-3-yl]-1-oxa-3,8-diazaspiro[4.5]decan-2-one | **A** | **K** | ¹H NMR (400 MHz, Methanol-*d*₄) δ 4.48 - 4.37 (m, 2H), 3.33 (s, 2H), 2.98 - 2.84 (m, 1H), 2.74 - 2.62 (m, 2H), 2.56 (t, J = 10.9 Hz, 2H), 2.19 (s, 3H), 2.15 - 2.03 (m, 2H), 2.00 - 1.83 (m, 6H), 1.82 - 1.62 (m, 4H). One exchangeable proton not observed. | **System 3** | m/z 348 (M+H)⁺ (ES⁺), at 2.28 min, 202 nm |
| | | **18 and 22** | | | **Method F** | |
| **6-4** | Isomer 2: 8-[8-(3-Methyl-1,2,4-oxadiazol-5-yl)-8-azabicyclo[3.2.1]octan-3-yl]-1-oxa-3,8-diazaspiro[4.5]decan-2-one | **A** | **K** | ¹H NMR (400 MHz, Methanol-*d*₄) δ 4.41 - 4.26 (m, 2H), 3.35 (s, 2H), 2.79 - 2.44 (m, 4H), 2.39 - 2.29 (m, 1H), 2.18 (s, 3H), 2.17 - 2.08 (m, 4H), 2.08 - 2.01 (m, 2H), 2.00 - 1.89 (m, 4H), 1.89 - 1.79 (m, 2H). One exchangeable proton not observed. | **System 3** | m/z 348 (M+H)⁺ (ES⁺), at 2.40 min, 202 nm |
| | | **18 and 22** | | | **Method F** | |
| **6-5** | Isomer 2: *N*-(1-Methylcyclobutyl)-1-[8-(3-methyl-1,2,4-oxadiazol-5-yl)-8-azabicyclo[3.2.1]oct-3-yl]piperidine-4-carboxamide | **B** | **L** | ¹H NMR (400 MHz, Methanol-*d*₄) δ 4.37 - 4.29 (m, 2H), 3.21 (d, *J = 11.2* Hz, 2H), 2.30 - 2.20 (m, 2H), 2.20 - 1.93 (m, 10H), 2.18 (s, 3H), 1.93 - 1.78 (m, 6H), 1.78 - 1.63 (m, 4H), 1.41 (s, 3H). One exchangeable proton not observed. | **System 3** | m/z 388 (M+H)⁺ (ES⁺), at 3.06 min, 202 nm |
| | | **24 and 25** | | | **Method F** | |
| **7-1** | Isomer 1: 2-(3-Methyl-1,2,4-oxadiazol-5-yl)-5-[4-(1*H*-pyrazol-1-yl)piperidin-1-yl]-2-azabicyclo[2.2.2]octane | **A** | **M** | ¹H NMR (400 MHz, Methanol-*d*₄) δ 7.68 (d, *J* = 2 Hz, 1H), 7.49 (d, *J* = 2 Hz, 1H), 6.29 (t, *J* = 2 Hz, 1H), 4.28 - 4.14 (m, 1H), 4.06 - 3.99 (m, 1H), 3.65 - 3.58 (m, 1H), 3.52 - 3.44 (m, 1H), 3.38 - 3.15 (m, 2H), 2.46 - 2.38 (m, 1H), 2.34 - 2.29 (m, 1H), 2.29 - 2.19 (m, 1H), 2.17 (s, 3H), 2.15 - 1.99 (m, 7H), 1.95 - 1.78 (m, 2H), 1.66 - 1.53 (m, 2H). | **System 3** | m/z 343 (M+H)⁺ (ES⁺), at 2.87 min, 202 nm |
| | | **4 and 28** | | | **Method F** | |
| **7-1** | Isomer 2: 2-(3-Methyl-1,2,4-oxadiazol-5-yl)-5-[4-(1*H*-pyrazol-1-yl)piperidin-1-yl]-2-azabicyclo[2.2.2]octane | **A** | **M** | ¹H NMR (400 MHz, Methanol-*d*₄) δ 7.67 (d, *J* = 2 Hz, 1H), 7.47 (d, *J* = 2 Hz, 1H), 6.28 (t, *J* = 2 Hz, 1H), 4.27 - 4.14 (m, 1H), 4.08 - 4.01 (m, 1H), 3.81 - 3.71 (m, 1H), 3.44 - 3.36 (m, 1H), 3.31 - 3.18 (m, 2H), 2.46 - 2.30 (m, 2H), 2.17 (s, 3H), 2.14 - 1.98 (m, 7H), 1.97 - 1.74 (m, 3H), 1.71 (d, *J* = 8.2 Hz, 2H). | **System 3** | m/z 343 (M+H)⁺ (ES⁺), at 2.99 min, 202 nm |
| | | **4 and 28** | | | **Method F** | |
| **8-1** | Isomer 2: 9-(3-Methyl-1,2,4-oxadiazol-5-yl)-3-[4-(1*H*-pyrazol-1-yl)piperidin-1-yl]-9-azabicyclo[3.3.1]nonane | **A** | **N then O** | ¹H NMR (400 MHz, Methanol-*d*₄) δ 7.66 (d, *J* = 2 Hz, 1H), 7.48 (d, *J* = 2 Hz, 1H), 6.28 (t, *J* = 2 Hz, 1H), 4.42 - 4.33 (m, 2H), 4.24 - 4.13 (m, 1H), 3.44 - 3.34 (m, 1H), 3.20 (d, *J =* 11.8 Hz, 2H), 2.40 - 2.28 (m, 2H), 2.18 (s, 3H), 2.16 - 2.07 (m, 4H), 2.06 - 1.89 (m, 4H), 1.89 - 1.77 (m, 3H), 1.76 - 1.67 (m, 1H), 1.40 - 1.19 (m, 1H), 1.15 (d, *J* = 6.2 Hz, 0.3H), 0.95 - 0.82 (m, 0.7H). | **System 3** | m/z 357 (M+H)⁺ (ES⁺), at 3.01 min, 215 nm |
| | | **4 and 31** | | | **Method F** | |
| **8-2** | Isomer 1: 8-[9-(3-Methyl-1,2,4-oxadiazol-5-yl)-9-azabicyclo[3.3.1]non-3-yl]-2,8-diazaspiro[4.5]decan-3-one | **A** | **P** | ¹H NMR (400 MHz, Methanol-*d*₄) δ 4.45 (d, *J* = 10.7 Hz, 2H), 3.19 (s, 2H), 2.65 - 2.55 (m, 4H), 2.44 - 2.29 (m, 3H), 2.21 (s, 2H), 2.19 - 1.99 (m, 2H), 2.16 (s, 3H), 1.79 - 1.66 (m, 6H), 1.62 - 1.49 (m, 4H). One exchangeable proton not observed. | **System 3** | m/z 360 (M+H)⁺ (ES⁺), at 2.45 min, 202 nm |
| | | **19 and 31** | | | **Method F** | |
| **8-3** | Isomer 1: 8-[9-(3-Methyl-1,2,4-oxadiazol-5-yl)-9-azabicyclo[3.3.1]non-3-yl]-1-oxa-3,8-diazaspiro[4.5]decan-2-one | **A** | **Q** | ¹H NMR (400 MHz, Methanol-*d*₄) δ 4.46 (d, *J* = 10.3 Hz, 2H), 3.35 (s, 2H), 2.77 - 2.63 (m, 4H), 2.48 - 2.31 (m, 3H), 2.19 - 2.02 (m, 1H), 2.16 (s, 3H), 2.00 - 1.90 (m, 2H), 1.87 - 1.67 (m, 4H), 1.64 - 1.49 (m, 5H). One exchangeable proton not observed. | **System 3** | m/z 362 (M+H)⁺ (ES⁺), at 2.43 min, 202 nm |
| | | **22 and 31** | | | **Method F** | |
| **8-4** | Isomer 1: *N*-(1-Methylcyclobutyl)-1-[9-(3-methyl-1,2,4-oxadiazol-5-yl)-9-azabicyclo[3.3.1]non-3-yl]piperidine-4-carboxamide | **B** | **R then S** | ¹H NMR (400 MHz, Methanol-*d*₄) δ 4.58 - 4.36 (m, 2H), 3.11 (d, *J* = 11.7 Hz, 2H), 2.49 - 2.20 (m, 7H), 2.16 (s, 3H), 2.13 - 2.04 (m, 1H), 2.02 - 1.90 (m, 3H), 1.88 - 1.67 (m, 8H), 1.65 - 1.48 (m, 5H), 1.41 (s, 3H). One exchangeable proton not observed. | **System 3** | m/z 402 (M+H)⁺ (ES⁺), at 3.16 min, 202 nm |
| | | **32 and 25** | | | **Method F** | |
| **9-1** | Isomer 1: 9-(3-Methyl-1,2,4-oxadiazol-5-yl)-7-[4-(1*H*-pyrazol-1-yl)piperidin-1-yl]-3-oxa-9-azabicyclo[3.3.1]nonane | **A** | **T then U** | ¹H NMR (400 MHz, Methanol-*d*₄) δ 7.66 (d, *J* = 2 Hz, 1H), 7.47 (d, *J* = 2 Hz, 1H), 6.28 (t, *J* = 2 Hz, 1H), 4.24 - 4.10 (m, 3H), 3.95 (d, *J* = 11.7 Hz, 2H), 3.87 - 3.72 (m, 3H), 3.18 - 3.09 (m, 2H), 2.41 - 2.29 (m, 2H), 2.23 - 2.15 (m, 2H), 2.20 (s, 3H), 2.15 - 2.07 (m, 2H), 2.04 - 1.92 (m, 2H), 1.91 - 1.79 (m, 2H). | **System 3** | m/z 359 (M+H)⁺ (ES⁺), at 2.43 min, 220 nm |
| | | **4 and 35** | | | **Method F** | |
| **9-2** | Isomer 2: (1*R,*5*S*,6*r*)-*N,N-*Diethyl-3-[9-(3-methyl-1,2,4-oxadiazol-5-yl)-3-oxa-9-azabicyclo[3.3.1]non-7-yl]-3-azabicyclo[3.1.0] hexane-6-carboxamide | **B** | **V** | ¹H NMR (400 MHz, Methanol-*d*₄) δ 4.13 - 4.03 (m, 2H), 3.96 - 3.86 (m, 2H), 3.86 - 3.76 (m, 2H), 3.67 - 3.61 (m, 1H), 3.52 - 3.39 (m, 2H), 3.39 - 3.31 (m, 2H), 3.20 - 3.08 (m, 2H), 2.58 - 2.32 (m, 3H), 2.24 - 2.07 (m, 2H), 2.19 (s, 3H), 1.98 - 1.88 (m, 2H), 1.79 - 1.65 (m, 2H), 1.22 (t, *J* = 7.1 Hz, 3H), 1.12 - 1.01 (m, 3H). | **System 3** | m/z 390 (M+H)⁺ (ES⁺), at 2.75 min, 202 nm |
| | | **37 and 38** | | | **Method F** | |
| **10-1** | Isomer 1: 1-(3-Methyl-1,2,4-oxadiazol-5-yl)-4-[4-(1*H*-pyrazol-1-yl)piperidin-1-yl]azepane | **A** | **W then X** | ¹H NMR (400 MHz, Chloroform-d) δ 7.50 - 7.48 (m, 1H), 7.43 - 7.41 (m, 1H), 6.26 - 6.22 (m, 1H), 4.17 - 4.06 (m, 1H), 3.81 - 3.67 (m, 2H), 3.56 - 3.40 (m, 2H), 2.98 - 2.86 (m, 2H), 2.64 - 2.52 (m, 1H), 2.48 - 2.32 (m, 2H), 2.21 (t, *J* = 0.5 Hz, 3H), 2.20 - 2.11 (m, 2H), 2.10 - 1.88 (m, 5H), 1.85 - 1.73 (m, 1H), 1.72 - 1.59 (m, 1H), 1.58 - 1.45 (m, 1H). | **System 5** | m/z 331 (M+H)⁺ (ES⁺), at 2.94 min, 254 nm |
| | | **4 and 41** | | | **Method G** | |
| **10-1** | Isomer 2: 1-(3-Methyl-1,2,4-oxadiazol-5-yl)-4-[4-(1*H*-pyrazol-1-yl)piperidin-1-yl]azepane | **A** | **W then X** | ¹H NMR (400 MHz, Chloroform-d) δ 7.50 - 7.48 (m, 1H), 7.42 (d, *J* = 2.3 Hz, 1H), 6.25 - 6.23 (m, 1H), 4.15 - 4.05 (m, 1H), 3.80 - 3.67 (m, 2H), 3.55 - 3.40 (m, 2H), 2.95 - 2.86 (m, 2H), 2.61 - 2.52 (m, 1H), 2.45 - 2.33 (m, 2H), 2.21 (s, 3H), 2.18 - 2.10 (m, 2H), 2.08 - 1.87 (m, 5H), 1.84 - 1.72 (m, 1H), 1.72 - 1.59 (m, 1H), 1.55 - 1.44 (m, 1H). | **System 5** | m/z 331 (M+H)⁺ (ES⁺), at 2.95 min, 254 nm |
| | | **4 and 41** | | | **Method G** | |
| **10-2** | Isomer 1: 8-[1-(3-Methyl-1,2,4-oxadiazol-5-yl)azepan-4-yl]-2,8-diazaspiro[4.5]decan-3-one | **A** | **Y then Z** | ¹H NMR (400 MHz, Methanol-*d*₄) δ 3.82 - 3.63 (m, 2H), 3.59 - 3.44 (m, 2H), 3.18 (s, 2H), 2.62 - 2.44 (m, 6H), 2.20 (s, 2H), 2.17 (s, 3H), 2.13 - 2.02 (m, 1H), 2.03 - 1.91 (m, 1H), 1.86 - 1.74 (m, 1H), 1.73 - 1.60 (m, 5H), 1.58 - 1.45 (m, 1H). One exchangeable proton not observed. | **System 3** | m/z 334 (M+H)⁺ (ES⁺), at 1.98 min, 202 nm |
| | | **19 and 41** | | | **Method F** | |
| **10-2** | Isomer 2: 8-[1-(3-Methyl-1,2,4-oxadiazol-5-yl)azepan-4-yl]-2,8-diazaspiro[4.5]decan-3-one | **A** | **Y then Z** | ¹H NMR (400 MHz, Methanol-*d*₄) δ 3.82 - 3.64 (m, 2H), 3.57 - 3.44 (m, 2H), 3.18 (s, 2H), 2.63 - 2.44 (m, 6H), 2.20 (s, 2H), 2.17 (s, 3H), 2.12 - 2.04 (m, 1H), 2.04 - 1.93 (m, 1H), 1.86 - 1.74 (m, 1H), 1.73 - 1.60 (m, 5H), 1.59 - 1.47 (m, 1H). One exchangeable proton not observed. | **System 3** | m/z 334 (M+H)⁺ (ES⁺), at 1.99 min, 202 nm |
| | | **19 and 41** | | | **Method F** | |
| **10-3** | Racemic mixture: 8-[1-(3-Methyl-1,2,4-oxadiazol-5-yl)azepan-4-yl]-1-oxa-3,8-diazaspiro[4.5]decan-2-one | **A** | **AA** | ¹H NMR (400 MHz, DMSO-*d*₆) δ 7.46 (s, 1H), 3.70 - 3.55 (m, 2H), 3.47 - 3.36 (m, 2H), 3.18 (s, 2H), 2.57 - 2.44 (m, 3H), 2.44 - 2.36 (m, 2H), 2.08 (s, 3H), 1.93 - 1.81 (m, 2H), 1.80 - 1.60 (m, 6H), 1.59 - 1.48 (m, 1H), 1.48 - 1.37 (m, 1H). | **System 3** | m/z 336 (M+H)⁺ (ES⁺), at 2.13 min, 202 nm |
| | | **22 and 41** | | | **Method F** | |
| **10-4** | Isomer 1: 4-Ethyl-8-[1-(3-methyl-1,2,4-oxadiazol-5-yl)azepan-4-yl]-1-oxa-3,8-diazaspiro[4.5]decan-2-one | **A** | **AB then AC** | ¹H NMR (400 MHz, Methanol-*d*₄) δ 3.83 - 3.66 (m, 2H), 3.59 - 3.46 (m, 2H), 3.40 - 3.34 (m, 1H), 2.89 - 2.59 (m, 5H), 2.17 (s, 3H), 2.16 - 2.07 (m, 1H), 2.06 - 1.97 (m, 2H), 1.97 - 1.76 (m, 5H), 1.75 - 1.66 (m, 1H), 1.65 - 1.51 (m, 2H), 1.50 - 1.38 (m, 1H), 0.99 (t, *J* = 7.4 Hz, 3H). One exchangeable proton not observed. | **System 3** | m/z 364 (M+H)⁺ (ES⁺), at 2.42 min, 228 nm |
| | | **42 and 41** | | | **Method F** | |
| **10-4** | Isomer 2: 4-Ethyl-8-[1-(3-methyl-1,2,4-oxadiazol-5-yl)azepan-4-yl]-1-oxa-3,8-diazaspiro[4.5]decan-2-one | **A** | **AB then AC** | ¹H NMR (400 MHz, Methanol-*d*₄) δ 3.81 - 3.67 (m, 2H), 3.58 - 3.46 (m, 2H), 3.39 - 3.33 (m, 1H), 2.83 - 2.58 (m, 5H), 2.17 (s, 3H), 2.14 - 2.06 (m, 1H), 2.05 - 1.95 (m, 2H), 1.95 - 1.73 (m, 5H), 1.72 - 1.65 (m, 1H), 1.65 - 1.52 (m, 2H), 1.51 - 1.39 (m, 1H), 0.99 (t, *J* = 7.4 Hz, 3H). One exchangeable proton not observed. | **System 3** | m/z 364 (M+H)⁺ (ES⁺), at 2.42 min, 230 nm |
| | | **42 and 41** | | | **Method F** | |
| **10-4** | Isomer 3: 4-Ethyl-8-[1-(3-methyl-1,2,4-oxadiazol-5-yl)azepan-4-yl]-1-oxa-3,8-diazaspiro[4.5]decan-2-one | **A** | **AB then AC** | ¹H NMR (400 MHz, Methanol-*d*₄) δ 3.82 - 3.63 (m, 2H), 3.60 - 3.44 (m, 2H), 3.39 - 3.33 (m, 1H), 2.77 - 2.52 (m, 5H), 2.17 (s, 3H), 2.13 - 2.05 (m, 1H), 2.04 - 1.92 (m, 2H), 1.92 - 1.71 (m, 5H), 1.70 - 1.65 (m, 1H), 1.64 - 1.50 (m, 2H), 1.50 - 1.38 (m, 1H), 0.98 (t, *J* = 7.4 Hz, 3H). One exchangeable proton not observed. | **System 3** | m/z 364 (M+H)⁺ (ES⁺), at 2.40 min, 202 nm |
| | | **42 and 41** | | | **Method F** | |
| **10-4** | Isomer 4: 4-Ethyl-8-[1-(3-methyl-1,2,4-oxadiazol-5-yl)azepan-4-yl]-1-oxa-3,8-diazaspiro[4.5]decan-2-one | **A** | **AB then AC** | ¹H NMR (400 MHz, Methanol-*d*₄) δ 3.81 - 3.62 (m, 2H), 3.60 - 3.46 (m, 2H), 3.38 - 3.32 (m, 1H), 2.77 - 2.52 (m, 5H), 2.17 (s, 3H), 2.13 - 2.04 (m, 1H), 2.04 - 1.93 (m, 2H), 1.92 - 1.71 (m, 5H), 1.71 - 1.63 (m, 1H), 1.62 - 1.50 (m, 2H), 1.49 - 1.37 (m, 1H), 0.98 (t, *J* = 7.4 Hz, 3H). One exchangeable proton not observed. | **System 3** | m/z 364 (M+H)⁺ (ES⁺), at 2.40 min, 228 nm |
| | | **42 and 41** | | | **Method F** | |
| **10-5** | Isomer 1: 4,4-Dimethyl-8-[1-(3-methyl-1,2,4-oxadiazol-5-yl)azepan-4-yl]-1-oxa-3,8-diazaspiro[4.5]decan-2-one | **A** | **AD then AE** | ¹H NMR (400 MHz, Methanol-*d*₄) δ 3.81 - 3.62 (m, 2H), 3.58 - 3.43 (m, 2H), 2.82 - 2.69 (m, 2H), 2.65 - 2.48 (m, 3H), 2.17 (s, 3H), 2.12 - 2.04 (m, 1H), 2.04 - 1.78 (m, 5H), 1.77 - 1.50 (m, 4H), 1.20 (s, 6H). One exchangeable proton not observed. | **System 3** | m/z 364 (M+H)⁺ (ES⁺), at 2.32 min, 202 nm |
| | | **43 and 41** | | | **Method F** | |
| **10-6** | Isomer 1: 1'-[1-(3-Methyl-1,2,4-oxadiazol-5-yl)azepan-4-yl]spiro[indole-3,4'-piperidin]-2(1*H*)-one | **A** | **AF then AG** | ¹H NMR (400 MHz, Methanol-*d*₄) δ 7.38 (d, *J* = 7.5 Hz, 1H), 7.24 - 7.17 (m, 1H), 7.06 - 6.98 (m, 1H), 6.90 (d, *J* = 7.7 Hz, 1H), 3.97 - 3.88 (m, 1H), 3.84 - 3.70 (m, 2H), 3.62 - 3.51 (m, 2H), 3.16 - 3.05 (m, 2H), 2.86 - 2.76 (m, 2H), 2.75 - 2.66 (m, 1H), 2.26 - 2.15 (m, 1H), 2.18 (s, 3H), 2.14 - 2.00 (m, 2H), 1.96 - 1.79 (m, 4H), 1.77 - 1.58 (m, 2H). One exchangeable proton not observed. | **System 3** | m/z 382 (M+H)⁺ (ES⁺), at 3.03 min, 202 nm |
| | | **44 and 41** | | | **Method F** | |
| **10-7** | Isomer 1: [(2*R*,4*R*)-4-Fluoro-1-{1-[1-(3-methyl-1,2,4-oxadiazol-5-yl)azepan-4-yl]piperidin-4-yl}pyrrolidin-2-yl]methanol | **A** | **AH then Al** | ¹H NMR (400 MHz, Methanol-*d*₄) δ 5.12 (dt, *J* = 54.2, 4.4 Hz, 1H), 3.79 - 3.64 (m, 2H), 3.61 - 3.45 (m, 3H), 3.45 - 3.38 (m, 1H), 3.24 - 3.11 (m, 1H), 3.08 - 2.97 (m, 1H), 2.91 - 2.83 (m, 2H), 2.84 - 2.68 (m, 1H), 2.60 - 2.48 (m, 2H), 2.38 - 2.22 (m, 2H), 2.17 (s, 3H), 2.10 - 1.91 (m, 5H), 1.91 - 1.72 (m, 3H), 1.72 - 1.59 (m, 1H), 1.59 - 1.42 (m, 3H). One exchangeable proton not observed. | **System 3** | m/z 382 (M+H)⁺ (ES⁺), at 2.37 min, 220 nm |
| | | **47 and 41** | | | **Method F** | |
| **10-7** | Isomer 2: [(2*R*,4*R*)-4-Fluoro-1-{1-[1-(3-methyl-1,2,4-oxadiazol-5-yl)azepan-4-yl]piperidin-4-yl}pyrrolidin-2-yl]methanol | **A** | **AH then Al** | ¹H NMR (400 MHz, Methanol-*d*₄) δ 5.12 (dt, *J* = 54.1, 4.4 Hz, 1H), 3.80 - 3.65 (m, 2H), 3.61 - 3.45 (m, 3H), 3.45 - 3.37 (m, 1H), 3.23 - 3.11 (m, 1H), 3.07 - 2.98 (m, 1H), 2.91 - 2.84 (m, 2H), 2.84 - 2.68 (m, 1H), 2.60 - 2.48 (m, 2H), 2.38 - 2.26 (m, 2H), 2.17 (s, 3H), 2.13 - 1.91 (m, 5H), 1.91 - 1.72 (m, 3H), 1.72 - 1.57 (m, 1H), 1.57 - 1.44 (m, 3H). One exchangeable proton not observed. | **System 3** | m/z 382 (M+H)⁺ (ES⁺), at 2.37 min, 220 nm |
| | | **47 and 41** | | | **Method F** | |
| **10-8** | Isomer 1: [(2*R*)-4,4-Difluoro-1-{1-[1-(3-methyl-1,2,4-oxadiazol-5-yl)azepan-4-yl]piperidin-4-yl}pyrrolidin-2-yl]methanol | **A** | **AJ then AK** | ¹H NMR (400 MHz, Methanol-*d*₄) δ 3.81 - 3.64 (m, 2H), 3.61 - 3.44 (m, 4H), 3.26 - 3.11 (m, 2H), 3.10 - 2.97 (m, 1H), 2.95 - 2.82 (m, 2H), 2.76 - 2.64 (m, 1H), 2.64 - 2.52 (m, 1H), 2.41 - 2.23 (m, 3H), 2.22 - 2.14 (m, 1H), 2.17 (s, 3H), 2.12 - 2.03 (m, 1H), 2.03 - 1.92 (m, 2H), 1.87 - 1.73 (m, 3H), 1.71 - 1.60 (m, 1H), 1.59 - 1.41 (m, 3H). One exchangeable proton not observed. | **System 4** | m/z 400 (M+H)⁺ (ES⁺), at 2.48 min, 228 nm |
| | | **48 and 41** | | | **Method D** | |
| **10-8** | Isomer 2: [(2*R*)-4,4-Difluoro-1-{1-[1-(3-methyl-1,2,4-oxadiazol-5-yl)azepan-4-yl]piperidin-4-yl}pyrrolidin-2-yl]methanol | **A** | **AJ then AK** | ¹H NMR (400 MHz, Methanol-*d*₄) δ 3.81 - 3.65 (m, 2H), 3.61 - 3.44 (m, 4H), 3.26 - 3.11 (m, 2H), 3.11 - 2.98 (m, 1H), 2.95 - 2.83 (m, 2H), 2.76 - 2.65 (m, 1H), 2.65 - 2.53 (m, 1H), 2.41 - 2.26 (m, 3H), 2.24 - 2.13 (m, 1H), 2.17 (s, 3H), 2.13 - 2.04 (m, 1H), 2.03 - 1.91 (m, 2H), 1.87 - 1.75 (m, 3H), 1.74 - 1.59 (m, 1H), 1.58 - 1.44 (m, 3H). One exchangeable proton not observed. | **System 4** | m/z 400 (M+H)⁺ (ES⁺), at 2.47 min, 202 nm |
| | | **48 and 41** | | | **Method D** | |
| **10-9** | Isomer 2: (5S)-5-Ethyl-1-{1-[1-(3-methyl-1,2,4-oxadiazol-5-yl)azepan-4-yl]piperidin-4-yl}pyrrolidin-2-one | **A** | **AL then AM** | ¹H NMR (400 MHz, Methanol-*d*₄) δ 3.79 - 3.66 (m, 3H), 3.66 - 3.56 (m, 1H), 3.56 - 3.45 (m, 2H), 2.95 - 2.81 (m, 2H), 2.61 - 2.52 (m, 1H), 2.50 - 2.41 (m, 1H), 2.41 - 2.30 (m, 2H), 2.30 - 2.20 (m, 1H), 2.17 (s, 3H), 2.15 - 1.92 (m, 5H), 1.91 - 1.74 (m, 5H), 1.73 - 1.60 (m, 2H), 1.60 - 1.44 (m, 2H), 0.92 (t, *J* = 7.4 Hz, 3H). | **System 3** | m/z 376 (M+H)⁺ (ES⁺), at 2.77 min, 202 nm |
| | | **49 and 41** | | | **Method F** | |
| **10-10** | Isomer 1b: 1-(3-Methyl-1,2,4-oxadiazol-5-yl)-4-{4-[2-(1-methyl-1*H*-pyrazol-4-yl)pyrrolidin-1-yl]piperidin-1-yl}azepane | **A** | **AN to give Isomers 1 and 2 then AO to give Isomers 1a and 1b from Isomer 1** | ¹H NMR (400 MHz, Methanol-*d*₄) δ 7.52 (s, 1H), 7.41 (s, 1H), 3.92 - 3.81 (m, 1H), 3.85 (s, 3H), 3.77 - 3.64 (m, 2H), 3.54 - 3.42 (m, 2H), 2.98 - 2.89 (m, 1H), 2.88 - 2.77 (m, 2H), 2.76 - 2.68 (m, 1H), 2.56 - 2.46 (m, 1H), 2.38 - 2.29 (m, 1H), 2.26 - 2.09 (m, 3H), 2.16 (s, 3H), 2.07 - 1.82 (m, 6H), 1.81 - 1.68 (m, 3H), 1.67 - 1.59 (m, 1H), 1.59 - 1.41 (m, 3H). | **System 3** | m/z 414 (M+H)⁺ (ES⁺), at 2.36 min, 225 nm |
| | | **51 and 41** | | | **Method F** | |
| **10-10** | Isomer 2a: 1-(3-Methyl-1,2,4-oxadiazol-5-yl)-4-{4-[2-(1-methyl-1*H*-pyrazol-4-yl)pyrrolidin-1-yl]piperidin-1-yl}azepane | **A** | **AN to give Isomers 1 and 2 then AO to give Isomers 2a and 2b from Isomer 2** | ¹H NMR (400 MHz, Methanol-*d*₄) δ 7.54 (s, 1H), 7.42 (s, 1H), 3.97 - 3.89 (m, 1H), 3.86 (s, 3H), 3.77 - 3.64 (m, 2H), 3.55 - 3.43 (m, 2H), 3.01 - 2.91 (m, 1H), 2.89 - 2.71 (m, 3H), 2.59 - 2.48 (m, 1H), 2.44 - 2.33 (m, 1H), 2.28 - 2.10 (m, 3H), 2.16 (s, 3H), 2.09 - 1.84 (m, 6H), 1.83 - 1.68 (m, 3H), 1.67 - 1.41 (m, 4H). | **System 3** | m/z 414 (M+H)⁺ (ES⁺), at 2.34 min, 226 nm |
| | | **51 and 41** | | | **Method F** | |
| **10-10** | Isomer 2b: 1-(3-Methyl-1,2,4-oxadiazol-5-yl)-4-{4-[2-(1-methyl-1*H*-pyrazol-4-yl)pyrrolidin-1-yl]piperidin-1-yl}azepane | **A** | **AN to give Isomers 1 and 2 then AO to give Isomers 2a and 2b from Isomer 2** | ¹H NMR (400 MHz, Methanol-*d*₄) δ 7.52 (s, 1H), 7.41 (s, 1H), 3.92 - 3.82 (m, 1H), 3.85 (s, 3H), 3.76 - 3.64 (m, 2H), 3.54 - 3.42 (m, 2H), 2.98 - 2.89 (m, 1H), 2.88 - 2.76 (m, 2H), 2.76 - 2.68 (m, 1H), 2.56 - 2.47 (m, 1H), 2.39 - 2.29 (m, 1H), 2.27 - 2.09 (m, 3H), 2.16 (s, 3H), 2.08 - 1.81 (m, 6H), 1.82 - 1.68 (m, 3H), 1.67 - 1.54 (m, 2H), 1.54 - 1.41 (m, 2H). | **System 3** | m/z 414 (M+H)⁺ (ES⁺), at 2.34 min, 226 nm |
| | | **51 and 41** | | | **Method F** | |
| **10-11** | Racemic mixture: *N*-Ethyl-*N*-{1-[1-(3-methyl-1,2,4-oxadiazol-5-yl)azepan-4-yl]piperidin-4-yl}acetamide | **A** | **AP** | ¹H NMR (400 MHz, DMSO-*d*₆) δ 4.17 - 4.01 (m, 1H), 3.75 - 3.52 (m, 2H), 3.52 - 3.34 (m, 2H), 3.27 - 3.09 (m, 4H), 2.83 - 2.70 (m, 2H), 2.24 - 2.12 (m, 2H), 2.09 (s, 3H), 1.98 (d, *J* = 5.5 Hz, 3H), 1.93 - 1.81 (m, 2H), 1.80 - 1.65 (m, 2H), 1.65 - 1.36 (m, 5H), 1.03 (dt, J = 40.8, 6.9 Hz, 3H). | **System 3** | m/z 350 (M+H)⁺ (ES⁺), at 2.37 min, 202 nm |
| | | **52 and 41** | | | **Method F** | |
| **10-12** | Isomer 1: (2*S*)-*N*-Methyl-2-{1-[1-(3-methyl-1,2,4-oxadiazol-5-yl)azepan-4-yl]piperidin-4-yl}pyrrolidine-1-carboxamide | **C** | **AQ then AR** | ¹H NMR (400 MHz, Methanol-*d*₄) δ 3.90 - 3.80 (m, 1H), 3.79 - 3.63 (m, 3H), 3.60 - 3.43 (m, 3H), 2.93 - 2.80 (m, 2H), 2.71 (s, 3H), 2.61 - 2.50 (m, 1H), 2.35 - 2.21 (m, 2H), 2.16 (s, 3H), 2.13 - 2.05 (m, 1H), 2.03 - 1.94 (m, 2H), 1.94 - 1.69 (m, 6H), 1.68 - 1.45 (m, 4H), 1.43 - 1.31 (m, 2H). One exchangeable proton not observed. | **System 3** | m/z 391 (M+H)⁺ (ES⁺), at 2.10 min, 225 nm |
| | | **54 and 55** | | | **Method F** | |
| **10-12** | Isomer 2: (2*S*)-*N*-Methyl-2-{1-[1-(3-methyl-1,2,4-oxadiazol-5-yl)azepan-4-yl]piperidin-4-yl}pyrrolidine-1-carboxamide | **C** | **AQ then AR** | ¹H NMR (400 MHz, Methanol-*d*₄) δ 3.90 - 3.81 (m, 1H), 3.80 - 3.63 (m, 3H), 3.59 - 3.44 (m, 3H), 3.01 - 2.86 (m, 2H), 2.76 - 2.58 (m, 1H), 2.71 (s, 3H), 2.46 - 2.27 (m, 2H), 2.21 - 2.07 (m, 1H), 2.17 (s, 3H), 2.07 - 1.96 (m, 2H), 1.95 - 1.87 (m, 2H), 1.87 - 1.71 (m, 4H), 1.71 - 1.49 (m, 4H), 1.48 - 1.32 (m, 2H). One exchangeable proton not observed. | **System 3** | m/z 391 (M+H)⁺ (ES⁺), at 2.08 min, 225 nm |
| | | **54 and 55** | | | **Method F** | |
| **11-1** | Isomer 2: 6-(3-Methyl-1,2,4-oxadiazol-5-yl)-3-[4-(1*H*-pyrazol-1-yl)piperidin-1-yl]-6-azabicyclo[3.2.1]octane | **A** | **AS** | ¹H NMR (400 MHz, Methanol-*d*₄) δ 7.61 (d, *J* = 2 Hz, 1H), 7.45 (d, *J* = 2 Hz, 1H), 6.28 (t, *J* = 2 Hz, 1H), 4.23 - 4.16 (m, 1H), 4.13 - 4.02 (m, 1H), 3.63 (d, *J* = 9.7 Hz, 1H), 3.52 - 3.44 (m, 1H), 3.15 - 3.05 (m, 1H), 2.61 - 2.50 (m, 2H), 2.33 - 2.21 (m, 1H), 2.17 (s, 3H), 2.11 - 1.80 (m, 11H), 1.63 - 1.49 (m, 1H). | **System 3** | m/z 343 (M+H)⁺ (ES⁺), at 2.89 min, 202 nm |
| | | **4 and 58** | | | **Method F** | |
| **11-2** | Isomer 2: 8-[6-(3-Methyl-1,2,4-oxadiazol-5-yl)-6-azabicyclo[3.2.1]oct-3-yl]-2,8-diazaspiro[4.5]decan-3-one | **A** | **AT** | ¹H NMR (400 MHz, Methanol-*d*₄) δ 4.21 - 4.15 (m, 1H), 3.58 (d, *J* = 9.6 Hz, 1H), 3.49 - 3.43 (m, 1H), 3.12 (s, 2H), 2.60 - 2.52 (m, 2H), 2.52 - 2.40 (m, 3H), 2.38 - 2.27 (m, 2H), 2.18 (s, 3H), 2.14 (s, 2H), 2.10 - 2.01 (m, 2H), 1.97 - 1.92 (m, 1H), 1.91 - 1.81 (m, 4H), 1.63 - 1.52 (m, 1H), 1.52 - 1.39 (m, 1H). One exchangeable proton not observed. | **System 3** | m/z 346 (M+H)⁺ (ES⁺), at 2.10 min, 202 nm |
| | | **19 and 58** | | | **Method F** | |
| **11-3** | Isomer 2: 8-[6-(3-Methyl-1,2,4-oxadiazol-5-yl)-6-azabicyclo[3.2.1]oct-3-yl]-1-oxa-3,8-diazaspiro[4.5]decan-2-one | **A** | **AU** | ¹H NMR (400 MHz, Methanol-*d*₄) δ 4.24 - 4.13 (m, 1H), 3.60 (d, *J* = 9.6 Hz, 1H), 3.52 - 3.42 (m, 1H), 2.77 - 2.31 (m, 6H), 2.30 - 2.13 (m, 2H), 2.18 (s, 3H), 2.09 - 1.98 (m, 2H), 1.97 - 1.80 (m, 5H), 1.79 - 1.68 (m, 2H), 1.57 - 1.42 (m, 1H). One exchangeable proton not observed. | **System 3** | m/z 348 (M+H)⁺ (ES⁺), at 2.32 min, 210 nm |
| | | **22 and 58** | | | **Method F** | |
| **12-1** | Isomer 1: 8-(2-(3-Methyl-1,2,4-oxadiazol-5-yl)-2-azabicyclo[2.2.2]octan-5-yl)-2,8-diazaspiro[4.5]decan-3-one | **A** | **AV** | ¹H NMR (400 MHz, Methanol-*d*₄) δ 4.05 - 3.97 (m, 1H), 3.64 - 3.55 (m, 1H), 3.50 - 3.42 (m, 1H), 3.21 (s, 2H), 2.73 - 2.22 (m, 4H), 2.38 - 2.28 (m, 2H), 2.27 - 2.20 (m, 3H), 2.16 (s, 3H), 2.07 - 1.94 (m, 1H), 1.92 - 1.79 (m, 2H), 1.77 - 1.65 (m, 4H), 1.63 - 1.49 (m, 2H). One exchangeable proton not observed. | **System 3** | m/z 346 (M+H)⁺ (ES⁺), at 2.17 min, 229 nm |
| | | **19 and 28** | | | **Method F** | |
| **12-1** | Isomer 2: 8-(2-(3-Methyl-1,2,4-oxadiazol-5-yl)-2-azabicyclo[2.2.2]octan-5-yl)-2,8-diazaspiro[4.5]decan-3-one | **A** | **AV** | ¹H NMR (400 MHz, Methanol-*d*₄) δ 4.08 - 4.01 (m, 1H), 3.76 - 3.68 (m, 1H), 3.41 - 3.34 (m, 1H), 3.20 (s, 2H), 2.70 - 2.26 (m, 4H), 2.38 - 2.31 (m, 2H), 2.22 (s, 2H), 2.17 (s, 3H), 2.13 - 2.03 (m, 1H), 1.97 - 1.88 (m, 1H), 1.87 - 1.79 (m, 1H), 1.79 - 1.60 (m, 7H). One exchangeable proton not observed. | **System 3** | m/z 346 (M+H)⁺ (ES⁺), at 2.25 min, 229 nm |
| | | **19 and 28** | | | **Method F** | |
| **12-2** | Isomer 2: 8-(2-(3-Methyl-1,2,4-oxadiazol-5-yl)-2-azabicyclo[2.2.2]octan-5-yl)-1-oxa-3,8-diazaspiro[4.5]decan-2-one | **A** | **AW** | ¹H NMR (400 MHz, Methanol-*d*₄) δ 4.08 - 4.01 (m, 1H), 3.76 - 3.68 (m, 1H), 3.45 - 3.35 (m, 2H), 2.73 (s, 2H), 2.46 (s, 2H), 2.39 - 2.30 (m, 2H), 2.17 (s, 3H), 2.13 - 2.05 (m, 1H), 2.01 - 1.89 (m, 3H), 1.88 - 1.64 (m, 7H). One exchangeable proton not observed. | **System 3** | m/z 348 (M+H)⁺ (ES⁺), at 2.29 min, 230 nm |
| | | **22 and 28** | | | **Method F** | |
| **12-3** | Isomer 1: 8-(8-(3-(Trifluoromethyl)-1,2,4-oxadiazol-5-yl)-8-azabicyclo[3.2.1]octan-3-yl)-2,8-diazaspiro[4.5]decan-3-one | **A** | **AX** | 1H NMR (400 MHz, Methanol-*d*₄) δ 4.56 - 4.48 (m, 2H), 3.18 (s, 2H), 2.95 - 2.81 (m, 1H), 2.67 - 2.39 (m, 4H), 2.20 (s, 2H), 2.18 - 2.09 (m, 2H), 2.06 - 1.98 (m, 2H), 1.95 - 1.86 (m, 2H), 1.75 - 1.59 (m, 6H). One exchangeable proton not observed. | **System 3** | m/z 400 (M+H)⁺ (ES⁺), at 3.07 min, 239 nm |
| | | **19 and 60** | | | **Method F** | |
| **12-3** | Isomer 2: 8-(8-(3-(Trifluoromethyl)-1,2,4-oxadiazol-5-yl)-8-azabicyclo[3.2.1]octan-3-yl)-2,8-diazaspiro[4.5]decan-3-one | **A** | **AX** | 1H NMR (400 MHz, Methanol-*d*₄) δ 4.68 - 4.61 (m, 1H), 4.48 - 4.40 (m, 2H), 3.21 (s, 2H), 2.71 - 2.38 (m, 4H), 2.38 - 2.30 (m, 1H), 2.22 (s, 2H), 2.19 - 2.12 (m, 3H), 2.12 - 2.04 (m, 2H), 2.03 - 1.94 (m, 2H), 1.76 - 1.66 (m, 4H). One exchangeable proton not observed. | **System 3** | m/z 400 (M+H)⁺ (ES⁺), at 3.40 min, 239 nm |
| | | **19 and 60** | | | **Method F** | |
| **12-4** | Isomer 1: 8-(8-(3-(Trifluoromethyl)-1,2,4-oxadiazol-5-yl)-8-azabicyclo[3.2.1]octan-3-yl)-1-oxa-3,8-diazaspiro[4.5]decan-2-one | **A** | **AY** | ¹H NMR (400 MHz, Methanol-*d*₄) δ 4.60 - 4.44 (m, 2H), 3.35 - 3.32 (m, 2H), 3.00 - 2.85 (m, 1H), 2.77 - 2.47 (m, 4H), 2.22 - 2.07 (m, 2H), 2.07 - 1.84 (m, 6H), 1.83 - 1.61 (m, 4H). One exchangeable proton not observed. | **System 3** | m/z 402 (M+H)⁺ (ES⁺), at 3.24 min, 238 nm |
| | | **22 and 60** | | | **Method F** | |
| **12-4** | Isomer 2: 8-(8-(3-(Trifluoromethyl)-1,2,4-oxadiazol-5-yl)-8-azabicyclo[3.2.1]octan-3-yl)-1-oxa-3,8-diazaspiro[4.5]decan-2-one | **A** | **AY** | ¹H NMR (400 MHz, Methanol-*d*₄) δ 4.50 - 4.37 (m, 2H), 3.36 (s, 2H), 2.85 - 2.46 (m, 4H), 2.43 - 2.33 (m, 1H), 2.25 - 1.91 (m, 10H), 1.91 - 1.76 (m, 2H). One exchangeable proton not observed. | **System 3** | m/z 402 (M+H)⁺ (ES⁺), at 3.58 min, 239 nm |
| | | **22 and 60** | | | **Method F** | |
| **12-6** | Isomer 1: (1*R*,5*S*,6*r*)-3-(2-(3-Methyl-1,2,4-oxadiazol-5-yl)-2-azaspiro[3.4]octan-6-yl)-N-(1-methylcyclobutyl)-3-azabicyclo[3.1.0]hexane-6-carboxamide | **A** | **AZ then BA** | ¹H NMR (400 MHz, Methanol-*d*₄) δ 4.16 - 3.97 (m, 4H), 3.13 - 3.03 (m, 2H), 2.71 - 2.61 (m, 1H), 2.47 - 2.36 (m, 2H), 2.32 - 2.20 (m, 2H), 2.16 (s, 3H), 2.11 - 1.90 (m, 6H), 1.88 - 1.76 (m, 6H), 1.63 - 1.48 (m, 1H), 1.41 (s, 3H). One exchangeable proton not observed. | **System 3** | m/z 384 (M-H)⁻ (ES⁻), at 3.12 min, 227 nm |
| | | **62 and 12** | | | **Method F** | |
| **12-6** | Isomer 1: (1*R*,5*S*,6*r*)-3-(2-(3-Methyl-1,2,4-oxadiazol-5-yl)-2-azaspiro[3.4]octan-6-yl)-N-(1-methylcyclobutyl)-3-azabicyclo[3.1.0]hexane-6-carboxamide | **A** | **AZ then BA** | ¹H NMR (400 MHz, Methanol-*d*₄) δ 4.14 - 3.97 (m, 4H), 3.13 - 3.03 (m, 2H), 2.70 - 2.60 (m, 1H), 2.45 - 2.35 (m, 2H), 2.33 - 2.20 (m, 2H), 2.16 (s, 3H), 2.10 - 1.90 (m, 6H), 1.88 - 1.74 (m, 6H), 1.62 - 1.50 (m, 1H), 1.41 (s, 3H). One exchangeable proton not observed. | **System 3** | m/z 384 (M-H)⁻ (ES⁻), at 3.11 min, 225 nm |
| | | **62 and 12** | | | **Method F** | |
| **12-7** | Isomer 2: 1-(8-(3-Methyl-1,2,4-oxadiazol-5-yl)-8-azabicyclo[3.2.1]octan-3-yl)-*N*-(1-(trifluoromethyl)cyclobutyl)p iperidine-4-carboxamide | **B** | **BB** | ¹H NMR (400 MHz, Methanol-*d*₄) δ 4.37 - 4.28 (m, 2H), 3.24 - 3.16 (m, 2H), 2.56 - 2.39 (m, 4H), 2.29 - 2.19 (m, 2H), 2.18 (s, 3H), 2.17 - 2.12 (m, 2H), 2.10 - 1.93 (m, 6H), 1.93 - 1.82 (m, 4H), 1.82 - 1.64 (m, 4H). One exchangeable proton not observed. | **System 3** | m/z 442 (M+H)⁺ (ES⁺), at 3.30 min, 202 nm |
| | | **24 and 63** | | | **Method F** | |
| **12-8** | Isomer 2: *N*-(1-Ethylcyclobutyl)-1-(8-(3-methyl-1,2,4-oxadiazol-5-yl)-8-azabicyclo [3.2.1]octan-3-yl)piperidine-4-carboxamide | **B** | **BC** | ¹H NMR (400 MHz, Methanol-*d*₄) δ 4.36 - 4.29 (m, 2H), 3.25 - 3.17 (m, 2H), 2.28 - 2.21 (m, 1H), 2.18 (s, 3H), 2.17 - 2.09 (m, 5H), 2.09 - 1.97 (m, 6H), 1.93 - 1.79 (m, 8H), 1.79 - 1.65 (m, 4H), 0.81 (t, *J* = 7.3 Hz, 3H). One exchangeable proton not observed. | **System 3** | m/z 402 (M+H)⁺ (ES⁺), at 3.23 min, 202 nm |
| | | **24 and 64** | | | **Method F** | |
| **12-11** | Isomer 2: 1-(8-(3-Methyl-1,2,4-oxadiazol-5-yl)-8-azabicyclo[3.2.1]octan-3-yl)-*N*-(1-methylcyclopentyl)piperidin e-4-carboxamide | **B** | **BD** | ¹H NMR (400 MHz, Methanol-*d*₄) δ 4.33 (s, 2H), 3.24 - 3.17 (m, 2H), 2.29 - 2.21 (m, 1H), 2.18 (s, 3H), 2.17 - 1.94 (m, 9H), 1.93 - 1.79 (m, 4H), 1.77 - 1.54 (m, 10H), 1.36 (s, 3H). One exchangeable proton not observed. | **System 3** | m/z 402 (M+H)⁺ (ES⁺), at 3.61 min, 202 nm |
| | | **24 and 65** | | | **Method F** | |
| **12-12** | Isomer 2: 1-(8-(3-Methyl-1,2,4-oxadiazol-5-yl)-8-azabicyclo[3.2.1]octan-3-yl)-*N*-((1-methylcyclobutyl)methyl)pi peridine-4-carboxamide | **A** | **BE** | ¹H NMR (400 MHz, Methanol-*d*₄) δ 4.36 - 4.29 (m, 2H), 3.26 - 3.18 (m, 2H), 3.16 (s, 2H), 2.30 - 2.20 (m, 2H), 2.18 (s, 3H), 2.16 - 1.98 (m, 6H), 1.95 - 1.81 (m, 8H), 1.81 - 1.72 (m, 4H), 1.69 - 1.59 (m, 2H), 1.10 (s, 3H). One exchangeable proton not observed. | **System 3** | m/z 400 (M-H)⁻ (ES⁻), at 3.33 min, 202 nm |
| | | **68 and 18** | | | **Method F** | |
| **12-13** | Isomer 2: (1*R*,5*S*,6*r*)-3-(8-(3-methyl-1,2,4-oxadiazol-5-yl)-8-azabicyclo[3.2.1]octan-3-yl)-*N*-(1-methylcyclobutyl)-3-azabicyclo[3.1.0]hexane-6-carboxamide | **A** | **BF** | ¹H NMR (400 MHz, Methanol-*d*₄) δ 4.29 - 4.20 (m, 2H), 2.49 - 2.42 (m, 1H), 2.33 - 2.15 (m, 7H), 2.18 (s, 3H), 2.07 - 1.79 (m, 14H), 1.41 (s, 3H). One exchangeable proton not observed. | **System 3** | m/z 384 (M-H)⁻ (ES⁻), at 3.77 min, 230 nm |
| | | **62 and 18** | | | **Method F** | |
| **12-14** | Isomer 1: *N*-(1-Methylcyclobutyl)-1-(8-(3-(trifluoromethyl)-1,2,4-oxadiazol-5-yl)-8-azabicyclo[3.2.1]octan-3-yl)piperidine-4-carboxamide | **B** | **BG** | ¹H NMR (400 MHz, Methanol-*d*₄) δ 4.56 - 4.48 (m, 2H), 3.04 - 2.96 (m, 2H), 2.94 - 2.83 (m, 1H), 2.30 - 2.19 (m, 2H), 2.18 - 2.05 (m, 5H), 2.03 - 1.94 (m, 4H), 1.93 - 1.87 (m, 2H), 1.87 - 1.77 (m, 2H), 1.77 - 1.60 (m, 6H), 1.40 (s, 3H). One exchangeable proton not observed. | **System 3** | m/z 442 (M-H)⁺ (ES⁺), at 3.89 min, 202 nm |
| | | **69 and 25** | | | **Method F** | |
| **12-14** | Isomer 2: *N*-(1-Methylcyclobutyl)-1-(8-(3-(trifluoromethyl)-1,2,4-oxadiazol-5-yl)-8-azabicyclo[3.2.1]octan-3-yl)piperidine-4-carboxamide | **B** | **BG** | ¹H NMR (400 MHz, Methanol-*d*₄) δ 4.46 - 4.39 (m, 2H), 3.27 - 3.19 (m, 2H), 2.34 - 2.20 (m, 3H), 2.19 - 2.09 (m, 5H), 2.08 - 2.03 (m, 2H), 2.02 - 1.93 (m, 4H), 1.90 - 1.80 (m, 4H), 1.79 - 1.64 (m, 4H), 1.41 (s, 3H). One exchangeable proton not observed. | **System 3** | m/z 442 (M-H)⁺ (ES⁺), at 4.27 min, 202 nm |
| | | **69 and 25** | | | **Method F** | |

| | | | | | | |
|---|---|---|---|---|---|---|
| (*) Reference compound. | | | | | | |

### BIOLOGICAL ACTIVITY

### EXAMPLE A

### Phospho-ERK1/2 assays

Functional assays were performed using the Alphascreen Surefire phospho-ERK1/2 assay (Crouch & Osmond, Comb. Chem. High Throughput Screen, 2008). ERK1/2 phosphorylation is a downstream consequence of both Gq/11 and Gi/o protein coupled receptor activation, making it highly suitable for the assessment of M₁, M₃ (Gq/11 coupled) and M₂, M₄ receptors (Gi/o coupled), rather than using different assay formats for different receptor subtypes. CHO cells stably expressing the human muscarinic M₁, M₂, M₃ or M₄ receptor were plated (25K / well) onto 96-well tissue culture plates in MEM-alpha + 10% dialysed FBS. Once adhered, cells were serum-starved overnight. Agonist stimulation was performed by the addition of 5 µL agonist to the cells for 5 min (37 °C). Media was removed and 50 µL of lysis buffer added. After 15 min, a 4 µL sample was transferred to 384-well plate and 7 µL of detection mixture added. Plates were incubated for 2 h with gentle agitation in the dark and then read on a PHERAstar plate reader. pEC₅₀ and Eₘₐₓ figures were calculated from the resulting data for each receptor subtype and the results are set out in Table 4 below.

For the vast majority of examples at least two diastereomers exist and these have been separated, unless otherwise stated, using the techniques of reversed phase HPLC, chiral HPLC or chiral SFC. Isomer assignment (Isomer 1, Isomer 2, etc.) is based on the retention time of the compound using the separation technique that was performed in the final purification step. By implication, this could be reversed phase HPLC, chiral HPLC or chiral SFC retention time, and this will vary from compound to compound.

Analytical data for active isomers is reported in Table 3. Data for several weakly active compounds are included in Table 4 to highlight the preference for absolute stereochemistry.

| **Table 4** | | | | |
|---|---|---|---|---|
| | **Muscarinic Activity** | | | |
| **Ex.No.** | **pEC₅₀ M1 (% Emax cf. ACh)** | **pEC₅₀ M2 (% Emax cf. ACh)** | **pEC₅₀ M3 (% Emax cf. ACh)** | **pEC₅₀ M4 (% Emax cf. ACh)** |
| ACh | 8.33(102) | 7.82 (105) | 8.12 (115) | 8.09 (110) |
| 1-1 | 4.99 (48) | NT | NT | 6.29 (77) |
| 2-1 Isomer 2 | < 4.70 (56) | < 4.70 (2) | < 4.70 (3) | 6.52 (41) |
| 2-2 Isomer 2 | 6.13 (40) | < 4.70 (2) | < 4.70 (3) | 6.93 (51) |
| 2-3 Isomer 2 | < 4.70 (9) | NT | NT | 6.29 (32) |
| 3-1 Isomer 2 | 5.08 (58) | NT | NT | 5.91 (68) |
| 4-1 (*) | 4.70 (50) | NT | NT | 6.00 (85) |
| 5-1 Isomer 1 | < 4.70 (9) | NT | NT | 5.90 (55) |
| 6-1 Isomer 1 | < 4.70 (23) | < 4.70 (2) | < 4.70 (2) | 6.53 (51) |
| 6-2 Isomer 1 | 6.80 (34) | NT | NT | < 4.70 (9) |
| 6-2 Isomer 2 | 7.20 (81) | NT | NT | 6.77 (39) |
| 6-3 Isomer 2 | 7.49 (33) | < 4.70 (3) | < 4.70 (1) | < 4.70 (7) |
| 6-4 Isomer 1 | 6.62 (49) | NT | NT | < 4.70 (15) |
| 6-4 Isomer 2 | 6.59 (87) | < 4.70 (5) | < 4.70 (32) | 6.28 (39) |
| 6-5 Isomer 2 | 6.83 (100) | < 4.70 (31) | < 4.70 (11) | 6.71 (79) |
| 7-1 Isomer 1 | < 4.70 (18) | < 4.70 (11) | < 4.70 (7) | 6.82 (85) |
| 7-1 Isomer 2 | 5.64 (33) | < 4.70 (15) | < 4.70 (6) | 7.07 (99) |
| 8-1 Isomer 2 | < 4.70 (24) | NT | NT | 5.90 (38) |
| 8-2 Isomer 1 | 6.67 (30) | NT | NT | < 4.70 (9) |
| 8-3 Isomer 1 | 6.53 (39) | < 4.70 (4) | < 4.70 (1) | < 4.70 (67) |
| 8-4 Isomer 1 | 6.24 (98) | NT | NT | 6.19 (76) |
| 9-1 Isomer 1 | < 4.70 (15) | < 4.70 (21) | < 4.70 (1) | 6.98 (77) |
| 9-2 Isomer 2 | 6.66 (109) | < 4.70 (27) | 5.20 (42) | 6.13 (74) |
| 10-1 Isomer 1 | 5.87 (32) | NT | NT | 6.89 (73) |
| 10-1 Isomer 2 | 5.18 (43) | NT | NT | 6.28 (72) |
| 10-2 Isomer 1 | 7.03 (66) | < 4.70 (3) | < 4.70 (5) | < 4.70 (10) |
| 10-2 Isomer 2 | 6.31 (53) | NT | NT | < 4.70 (15) |
| 10-3 Racemic mixture | 6.43 (97) | NT | NT | < 4.70 (10) |
| 10-4 Isomer 1 | 6.17 (123) | NT | NT | 7.00 (85) |
| 10-4 Isomer 2 | 7.26 (58) | NT | NT | 7.63 (64) |
| 10-4 Isomer 3 | 5.03 (81) | NT | NT | 5.66 (71) |
| 10-4 Isomer 4 | < 4.70 (18) | NT | NT | 6.06 (36) |
| 10-5 Isomer 1 | < 4.70 (21) | NT | NT | 6.66 (33) |
| 10-6 Isomer 1 | 6.18 (76) | < 4.70 (60) | < 4.70 (4) | 7.11 (77) |
| 10-7 Isomer 1 | 5.43 (44) | < 4.70 (15) | < 4.70 (2) | 6.98 (89) |
| 10-7 Isomer 2 | 5.37 (70) | NT | NT | 6.40 (76) |
| 10-8 Isomer 1 | 6.17 (37) | NT | NT | 7.03 (91) |
| 10-8 Isomer 2 | 5.81 (35) | NT | NT | 6.53 (73) |
| 10-9 Isomer 2 | 5.88 (53) | NT | NT | 6.32 (56) |
| 10-10 Isomer 1b | < 4.70 (6) | NT | NT | 6.29 (56) |
| 10-10 Isomer 2a | < 4.70 (3) | NT | NT | 5.84 (52) |
| 10-10 Isomer 2b | < 4.70 (14) | NT | NT | 6.50 (72) |
| 10-11 Racemic mixture | 5.40 (56) | NT | NT | 6.66 (101) |
| 10-12 Isomer 1 | < 4.70 (22) | NT | NT | 6.51 (100) |
| 10-12 Isomer 2 | < 4.70 (24) | NT | NT | 7.26 (119) |
| 11-1 Isomer 2 | 5.50 (48) | NT | NT | 5.98 (69) |
| 11-2 Isomer 2 | 8.02 (67) | < 4.70 (8) | < 4.70 (5) | < 4.70 (15) |
| 11-3 Isomer 2 | 7.15 (65) | < 4.70 (21) | < 4.70 (22) | < 4.70 (11) |
| 12-1 Isomer 1 | 6.22 (58) | NT | NT | < 4.70 (5) |
| 12-1 Isomer 2 | 7.49 (106) | < 4.70 (6) | < 4.70 (2) | 6.93 (30) |
| 12-2 Isomer 2 | 6.40 (80) | < 4.70 (14) | < 4.70 (1) | < 4.70 (18) |
| 12-3 Isomer 1 | 7.50 (67) | < 4.70 (19) | < 4.70 (31) | < 4.70 (14) |
| 12-3 Isomer 2 | 8.30 (103) | < 4.70 (14) | < 4.70 (70) | 6.70 (23) |
| 12-7 Isomer 2 | 6.27 (109) | NT | NT | 5.70 (109) |
| 12-8 Isomer 2 | 6.87 (104) | 5.61 (33) | < 4.70 (3) | 6.03 (109) |
| 12-11 Isomer 2 | 6.18 (103) | NT | NT | 5.57 (83) |
| 12-12 Isomer 2 | 8.43 (80) | NT | NT | 6.68 (107) |
| 12-13 Isomer 2 | 6.48 (102) | NT | NT | 6.09 (69) |
| 12-14 Isomer 1 | 6.04 (47) | NT | NT | < 4.70 (6) |
| 12-14 Isomer 2 | 7.73 (115) | < 4.70 (25) | < 4.70 (9) | 6.48 (40) |
| 12-15 Isomer 2 | 6.60 (113) | < 4.70 (18) | < 4.70 (6) | 5.33 (75) |
| 12-16 Isomer 1 | 7.49 (108) | 6.36 (62) | < 4.70 (13) | 7.41 (104) |
| 12-16 Isomer 2 | 7.28 (118) | < 4.70 (10) | < 4.70 (4) | 6.25 (62) |
| 12-18 Isomer 1 | 7.00 (87) | 5.23 (27) | < 4.70 (15) | 6.68 (62) |
| 12-18 Isomer 2 | 8.63 (92) | 6.59 (90) | 5.72 (48) | 7.83 (92) |
| 12-22 Isomer 2 | 8.06 (102) | 5.68 (81) | 4.90 (52) | 6.23 (108) |
| 12-23 Isomer 2 | 7.59 (132) | < 4.70 (13) | < 4.70 (25) | 6.92 (30) |
| 12-24 Isomer 1 | 6.08 (74) | NT | NT | < 4.70 (16) |
| 12-24 Isomer 2 | 7.58 (102) | 5.33 (60) | < 4.70 (25) | 6.02 (83) |
| 12-25 Isomer 1 | 6.70 (102) | < 4.70 (31) | < 4.70 (50) | < 4.70 (22) |
| 12-25 Isomer 2 | 8.55 (91) | 6.15 (84) | 5.29 (57) | 6.82 (119) |
| 12-27 Isomer 2 | 6.92 (76) | < 4.70 (14) | < 4.70 (15) | < 4.70 (21) |

| | | | | |
|---|---|---|---|---|
| (*) Reference compound. | | | | |

### Equivalents

The foregoing examples are presented for the purpose of illustrating the invention and should not be construed as imposing any limitation on the scope of the invention. It will readily be apparent that numerous modifications and alterations may be made to the specific embodiments of the invention described above and illustrated in the examples without departing from the scope of the appended claims. All such modifications and alterations are intended to be embraced by this application.

## Claims

1. A compound of the formula (1): 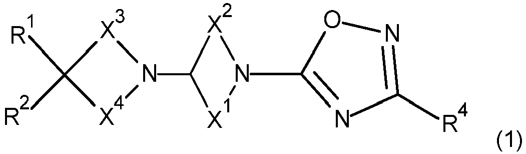
or a salt thereof,
wherein:
X¹ and X² are saturated hydrocarbon groups which together contain a total of five to nine carbon atoms and zero or one oxygen atoms and which link together such that the moiety: 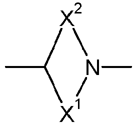
forms a monocyclic or bicyclic ring system;
X³ and X⁴ are saturated hydrocarbon groups which together contain a total of three to six carbon atoms and which link together such that the moiety: 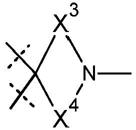
forms a monocyclic or bicyclic ring system;
R¹ is selected from NR⁵R⁶; CONR⁵R⁶; COOR⁷; an optionally substituted 5- or 6-membered ring containing 0, 1, 2 or 3 heteroatoms selected from O, N and S and oxidized forms thereof, wherein the optional substituent of the 5- or 6-membered ring is selected from C₁-C₃ alkyl, C₁-C₃ alkoxy, CONR⁵R⁶, halogen, cyano, oxo, hydroxyl, amino or pyrazole optionally substituted with C₁-C₃ alkyl;
or R¹ is linked to R² to form an optionally substituted monocyclic or bicyclic ring containing 0, 1, 2 or 3 heteroatoms selected from O, N and S and oxidized forms thereof, wherein the optional substituents of the monocyclic or bicyclic ring are selected from C₁-C₆ alkyl, C₁-C₃ alkoxy, halogen, cyano, oxo, hydroxyl, amino or a group -(CH₂)ₙ-aryl wherein n is 0-3;
R² is selected from hydrogen; fluorine; cyano; hydroxy; amino; and a C₁₋₃ hydrocarbon group which is optionally substituted with one to six fluorine atoms and wherein one of the carbon atoms of the hydrocarbon group may optionally be replaced by a heteroatom selected from O, N and S and oxidized forms thereof, or R² is linked to R¹ to form an optionally substituted monocyclic or bicyclic ring containing 0, 1, 2 or 3 heteroatoms selected from O, N and S and oxidized forms thereof, wherein the optional substituents of the monocyclic or bicyclic ring are selected from C₁-C₆ alkyl, C₁-C₃ alkoxy, halogen, cyano, oxo, hydroxyl, amino or a group -(CH₂)ₙ-aryl wherein n is 0-3;
R⁴ is H, halo, OH, CN, a C₁₋₆ alkyl group or a C₃₋₆ cycloalkyl group, wherein the alkyl and cycloalkyl groups are optionally substituted with one or more fluorine atoms, and wherein any one atom of the alkyl or cycloalkyl group may be optionally replaced by an O heteroatom;
R⁵ is selected from hydrogen, COCH₃, a non-aromatic C₁₋₁₀ hydrocarbon group optionally substituted with one or more fluorine atoms wherein any one atom of the C₁₋₁₀ hydrocarbon group may be optionally replaced by a heteroatom selected from O, N and S, a group -(CH₂)ₙ-aryl, wherein n is 0-3, or R⁵ can be joined together with R⁶ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino;
R⁶ is selected from hydrogen, a non-aromatic C₁₋₁₀ hydrocarbon group optionally substituted with one or more fluorine atoms wherein any one atom of the C₁₋₁₀ hydrocarbon group may be optionally replaced by a heteroatom selected from O, N and S, a group -(CH₂)ₙ-aryl, wherein n is 0-3, or R⁶ can be joined together with R⁵ to form an optionally substituted monocyclic or bicyclic ring containing 0, 1, 2 or 3 heteroatoms selected from O, N and S and oxidized forms thereof, wherein the optional substituents are selected from C₁-C₃ alkyl, C₁-C₃ alkoxy, halogen, cyano, oxo, hydroxyl or amino, wherein the C₁-C₃ alkyl or C₁-C₃ alkoxy groups can have an optional substituent selected from halogen, cyano, oxo, hydroxyl or amino; and
R⁷ is a non-aromatic C₁₋₆ hydrocarbon group optionally substituted with one or more fluorine atoms.

2. The compound according to claim 1, or a salt thereof, wherein:
R¹ is selected from NR⁵R⁶; CONR⁵R⁶; and COOR⁷; or
R¹ is an optionally substituted 5- or 6-membered ring containing 0, 1 or 2 nitrogen heteroatoms, wherein the optional substituents are selected from, C₁-C₃ alkyl, C₁-C₃ alkoxy, CONR⁵R⁶, halogen, cyano, oxo, hydroxyl, amino or an optionally substituted heterocyclic ring containing 1 or 2 heteroatoms selected from O or N wherein the optional substituent is C₁-C₃ alkyl.

3. The compound according to claim 1 or claim 2, or a salt thereof, wherein:
R¹ is selected from NR⁵R⁶; CONR⁵R⁶; or COOR⁷.

4. The compound according to any one of claims 1 to 3, or a salt thereof, wherein:
R² is H.

5. The compound according to any one of claims 1 to 4, or a salt thereof, wherein:
R¹ is selected from NR⁵R⁶; CONR⁵R⁶; or COOR⁷;
R⁵ is selected from COCH₃, a non-aromatic C₁₋₆ hydrocarbon group; and
R⁶ is selected from hydrogen and a non-aromatic C₁₋₆ hydrocarbon group.

6. The compound according to any one of claims 1 to 5, or a salt thereof, wherein the compound is of the formula (26): 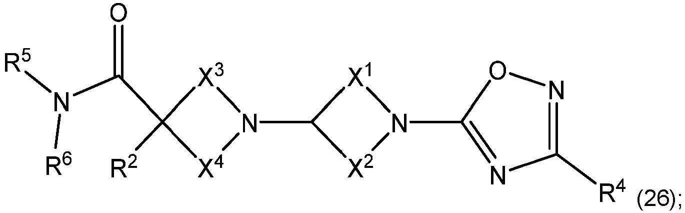
or a salt thereof;
wherein:
R², R⁴, R⁵, R⁶, X¹, X², X³, and X⁴ are as defined in any one of claims 1 to 5.

7. The compound according to claim 1, or a salt thereof, wherein R¹ is selected from:

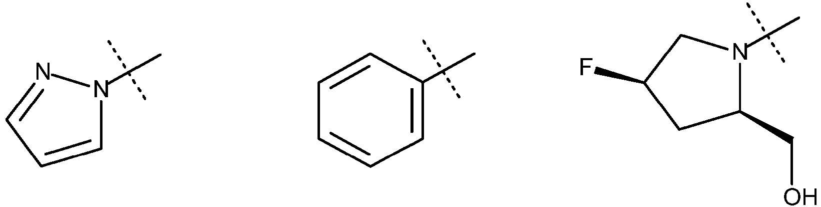

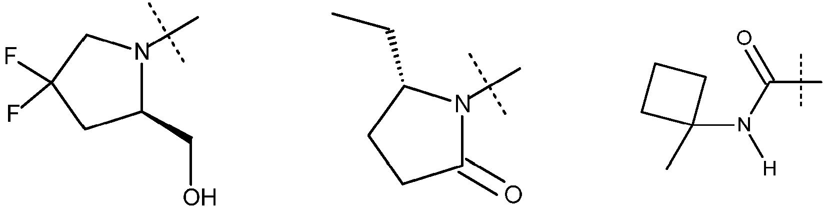

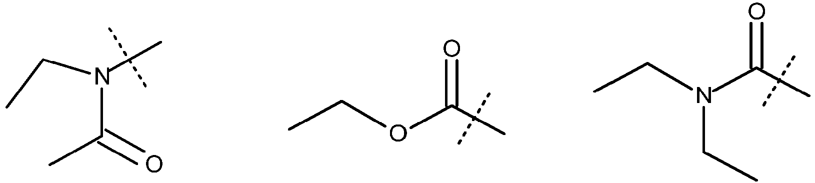

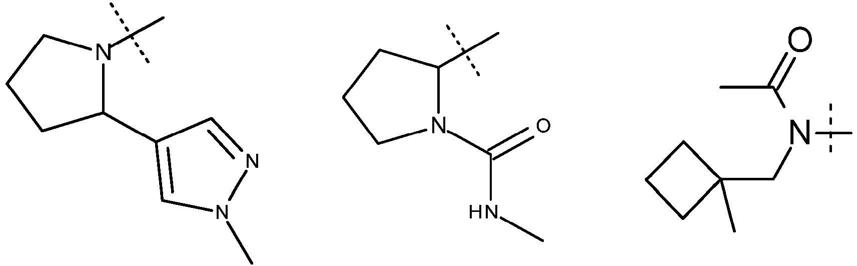

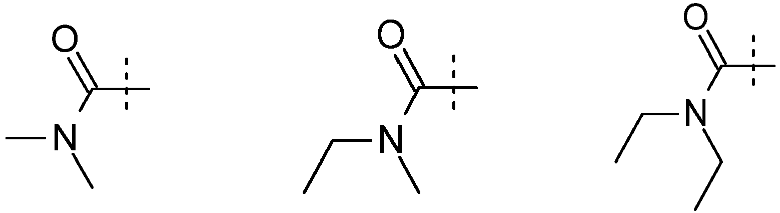

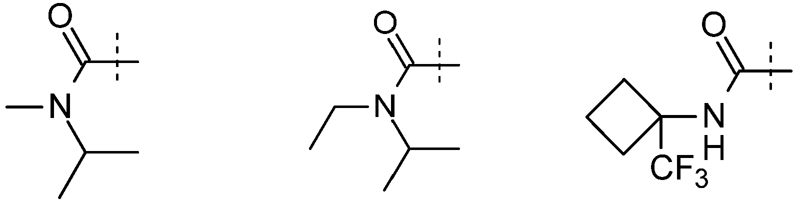

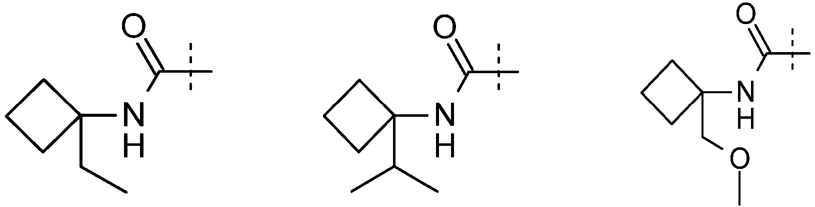

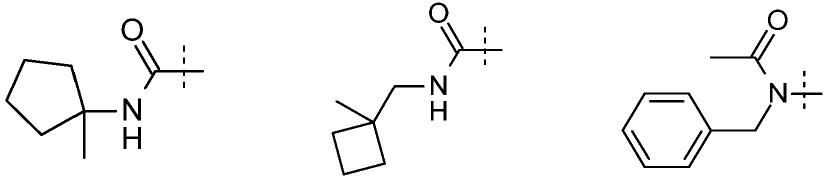

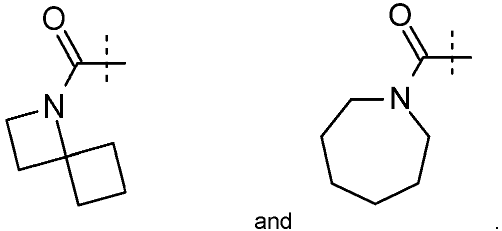

8. The compound according to any one of claims 1 to 7, or a salt thereof, wherein:
R⁴ is methyl, trifluoromethyl, ethyl, isopropyl or cyclopropyl.

9. The compound according to any one of claims 1 to 3 and 5 to 8, or a salt thereof, wherein:
R⁴ is trifluoromethyl.

10. The compound according to any one of claims 1 to 9, or a salt thereof, wherein the ring system formed by the moiety: 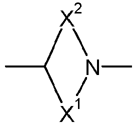 is selected from: 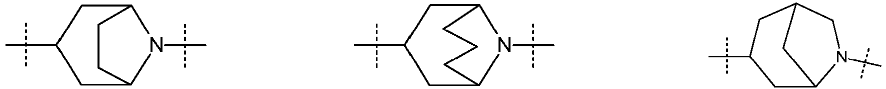 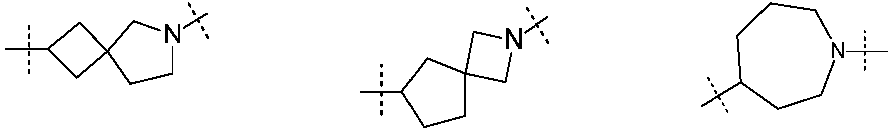 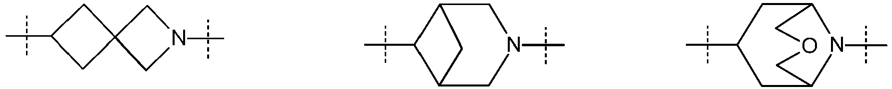 and

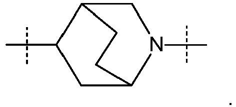

11. The compound according to claim 1, or a salt thereof, which is a compound of the formula (2): 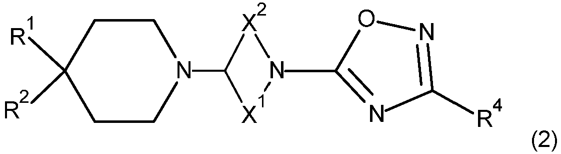
or a salt thereof;
wherein:
X¹, X², R¹, R² and R⁴ are as defined in any one of claims 1 to 10.

12. The compound according to claim 1, or a salt thereof, which is selected from:
2-(3-Methyl-1,2,4-oxadiazol-5-yl)-6-[4-(1H-pyrazol-1-yl)piperidin-1-yl]-2-azaspiro[3.3]heptane;
6-(3-Methyl-1,2,4-oxadiazol-5-yl)-2-[4-(1H-pyrazol-1-yl)piperidin-1-yl]-6-azaspiro[3.4]octane;
1-[6-(3-Methyl-1,2,4-oxadiazol-5-yl)-6-azaspiro[3.4]oct-2-yl]-4-phenylpiperidine-4-carbonitrile;
Ethyl (1R,5S,6r)-3-[6-(3-methyl-1,2,4-oxadiazol-5-yl)-6-azaspiro[3.4]oct-2-yl]-3-azabicyclo[3.1.0]hexane-6-carboxylate;
2-(3-Methyl-1,2,4-oxadiazol-5-yl)-6-[4-(1H-pyrazol-1-yl)piperidin-1-yl]-2-azaspiro[3.4]octane;
3-(3-Methyl-1,2,4-oxadiazol-5-yl)-6-[4-(1H-pyrazol-1-yl)piperidin-1-yl]-3-azabicyclo[3.1.1]heptane;
8-(3-Methyl-1,2,4-oxadiazol-5-yl)-3-[4-(1H-pyrazol-1-yl)piperidin-1-yl]-8-azabicyclo[3.2.1]octane;
8-[8-(3-Methyl-1,2,4-oxadiazol-5-yl)-8-azabicyclo[3.2.1]oct-3-yl]-2,8-diazaspiro[4.5]decan-3-one;
8-[8-(3-Ethyl-1,2,4-oxadiazol-5-yl)-8-azabicyclo[3.2.1]oct-3-yl]-2,8-diazaspiro[4.5]decan-3-one;
8-[8-(3-Methyl-1,2,4-oxadiazol-5-yl)-8-azabicyclo[3.2.1]octan-3-yl]-1-oxa-3,8-diazaspiro[4.5]decan-2-one;
N-(1-Methylcyclobutyl)-1-[8-(3-methyl-1,2,4-oxadiazol-5-yl)-8-azabicyclo[3.2.1]oct-3-yl]piperidine-4-carboxamide;
2-(3-Methyl-1,2,4-oxadiazol-5-yl)-5-[4-(1H-pyrazol-1-yl)piperidin-1-yl]-2-azabicyclo[2.2.2]octane;
9-(3-Methyl-1,2,4-oxadiazol-5-yl)-3-[4-(1H-pyrazol-1-yl)piperidin-1-yl]-9-azabicyclo[3.3.1]nonane;
8-[9-(3-Methyl-1,2,4-oxadiazol-5-yl)-9-azabicyclo[3.3.1]non-3-yl]-2,8-diazaspiro[4.5]decan-3-one;
8-[9-(3-Methyl-1,2,4-oxadiazol-5-yl)-9-azabicyclo[3.3.1]non-3-yl]-1-oxa-3,8-diazaspiro[4.5]decan-2-one;
N-(1-Methylcyclobutyl)-1-[9-(3-methyl-1,2,4-oxadiazol-5-yl)-9-azabicyclo[3.3.1]non-3-yl]piperidine-4-carboxamide;
9-(3-Methyl-1,2,4-oxadiazol-5-yl)-7-[4-(1H-pyrazol-1-yl)piperidin-1-yl]-3-oxa-9-azabicyclo[3.3.1]nonane;
(1R,5S,6r)-N,N-Diethyl-3-[9-(3-methyl-1,2,4-oxadiazol-5-yl)-3-oxa-9-azabicyclo[3.3.1]non-7-yl]-3-azabicyclo[3.1.0]hexane-6-carboxamide;
1-(3-Methyl-1,2,4-oxadiazol-5-yl)-4-[4-(1H-pyrazol-1-yl)piperidin-1-yl]azepane;
8-[1-(3-Methyl-1,2,4-oxadiazol-5-yl)azepan-4-yl]-2,8-diazaspiro[4.5]decan-3-one;
8-[1-(3-Methyl-1,2,4-oxadiazol-5-yl)azepan-4-yl]-1-oxa-3,8-diazaspiro[4.5]decan-2-one;
4-Ethyl-8-[1-(3-methyl-1,2,4-oxadiazol-5-yl)azepan-4-yl]-1-oxa-3,8-diazaspiro[4.5]decan-2-one;
4,4-Dimethyl-8-[1-(3-methyl-1,2,4-oxadiazol-5-yl)azepan-4-yl]-1-oxa-3,8-diazaspiro[4.5]decan-2-one;
1'-[1-(3-Methyl-1,2,4-oxadiazol-5-yl)azepan-4-yl]spiro[indole-3,4'-piperidin]-2(1H)-one;
[(2R,4R)-4-Fluoro-1-{1-[1-(3-methyl-1,2,4-oxadiazol-5-yl)azepan-4-yl]piperidin-4-yl}pyrrolidin-2-yl]methanol;
[(2R)-4,4-Difluoro-1-{1-[1-(3-methyl-1,2,4-oxadiazol-5-yl)azepan-4-yl]piperidin-4-yl}pyrrolidin-2-yl]methanol;
(5S)-5-Ethyl-1-{1-[1-(3-methyl-1,2,4-oxadiazol-5-yl)azepan-4-yl]piperidin-4-yl}pyrrolidin-2-one;
1-(3-Methyl-1,2,4-oxadiazol-5-yl)-4-{4-[2-(1-methyl-1H-pyrazol-4-yl)pyrrolidin-1-yl]piperidin-1-yl}azepane;
N-Ethyl-N-{1-[1-(3-methyl-1,2,4-oxadiazol-5-yl)azepan-4-yl]piperidin-4-yl}acetamide;
(2S)-N-Methyl-2-{1-[1-(3-methyl-1,2,4-oxadiazol-5-yl)azepan-4-yl]piperidin-4-yl}pyrrolidine-1-carboxamide;
6-(3-Methyl-1,2,4-oxadiazol-5-yl)-3-[4-(1H-pyrazol-1-yl)piperidin-1-yl]-6-azabicyclo[3.2.1]octane;
8-[6-(3-Methyl-1,2,4-oxadiazol-5-yl)-6-azabicyclo[3.2.1]oct-3-yl]-2,8-diazaspiro[4.5]decan-3-one;
8-[6-(3-Methyl-1,2,4-oxadiazol-5-yl)-6-azabicyclo[3.2.1]oct-3-yl]-1-oxa-3,8-diazaspiro[4.5]decan-2-one;
8-(2-(3-methyl-1,2,4-oxadiazol-5-yl)-2-azabicyclo[2.2.2]octan-5-yl)-2,8-diazaspiro[4.5]decan-3-one;
8-(2-(3-methyl-1,2,4-oxadiazol-5-yl)-2-azabicyclo[2.2.2]octan-5-yl)-1-oxa-3,8-diazaspiro[4.5]decan-2-one;
8-(8-(3-(trifluoromethyl)-1,2,4-oxadiazol-5-yl)-8-azabicyclo[3.2.1]octan-3-yl)-2,8-diazaspiro[4.5]decan-3-one;
8-(8-(3-(trifluoromethyl)-1,2,4-oxadiazol-5-yl)-8-azabicyclo[3.2.1]octan-3-yl)-1-oxa-3,8-diazaspiro[4.5]decan-2-one;
(1R,5S,6r)-3-(2-(3-methyl-1,2,4-oxadiazol-5-yl)-2-azaspiro[3.4]octan-6-yl)-N-(1-methylcyclobutyl)-3-azabicyclo[3.1.0]hexane-6-carboxamide;
1-(8-(3-methyl-1,2,4-oxadiazol-5-yl)-8-azabicyclo[3.2.1]octan-3-yl)-N-(1-(trifluoromethyl)cyclobutyl)piperidine-4-carboxamide;
N-(1-ethylcyclobutyl)-1-(8-(3-methyl-1,2,4-oxadiazol-5-yl)-8-azabicyclo[3.2.1]octan-3-yl)piperidine-4-carboxamide;
N-(1-isopropylcyclobutyl)-1-(8-(3-methyl-1,2,4-oxadiazol-5-yl)-8-azabicyclo[3.2.1]octan-3-yl)piperidine-4-carboxamide;
1-(8-(3-methyl-1,2,4-oxadiazol-5-yl)-8-azabicyclo[3.2.1]octan-3-yl)-N-(1-methylcyclopentyl)piperidine-4-carboxamide;
1-(8-(3-methyl-1,2,4-oxadiazol-5-yl)-8-azabicyclo[3.2.1]octan-3-yl)-N-((1-methylcyclobutyl)methyl)piperidine-4-carboxamide;
(1R,5S,6r)-3-(8-(3-methyl-1,2,4-oxadiazol-5-yl)-8-azabicyclo[3.2.1]octan-3-yl)-N-(1-methylcyclobutyl)-3-azabicyclo[3.1.0]hexane-6-carboxamide;
N-(1-methylcyclobutyl)-1-(8-(3-(trifluoromethyl)-1,2,4-oxadiazol-5-yl)-8-azabicyclo[3.2.1]octan-3-yl)piperidine-4-carboxamide;
1-(2-(3-methyl-1,2,4-oxadiazol-5-yl)-2-azabicyclo[2.2.2]octan-5-yl)-N-(1-methylcyclobutyl)piperidine-4-carboxamide;
1-(2-(3-methyl-1,2,4-oxadiazol-5-yl)-2-azabicyclo[2.2.2]octan-5-yl)-N-((1-methylcyclobutyl)methyl)piperidine-4-carboxamide;
N-(1-(9-(3-methyl-1,2,4-oxadiazol-5-yl)-3-oxa-9-azabicyclo[3.3.1]nonan-7-yl)piperidin-4-yl)-N-((1-methylcyclobutyl)methyl)acetamide;
N-(1-(8-(3-methyl-1,2,4-oxadiazol-5-yl)-8-azabicyclo[3.2.1]octan-3-yl)piperidin-4-yl)-N-((1-methylcyclobutyl)methyl)acetamide;
N-(1-(2-(3-methyl-1,2,4-oxadiazol-5-yl)-2-azabicyclo[2.2.2]octan-5-yl)piperidin-4-yl)-N-((1-methylcyclobutyl)methyl)acetamide;
N-benzyl-N-(1-(9-(3-methyl-1,2,4-oxadiazol-5-yl)-3-oxa-9-azabicyclo[3.3.1]nonan-7-yl)piperidin-4-yl)acetamide;
((1R,5S,6r)-3-(9-(3-methyl-1,2,4-oxadiazol-5-yl)-3-oxa-9-azabicyclo[3.3.1]nonan-7-yl)-3-azabicyclo[3.1.0]hexan-6-yl)(1-azaspiro[3.3]heptan-1-yl)methanone;
(1R,5S,6r)-N-isopropyl-N-methyl-3-(9-(3-methyl-1,2,4-oxadiazol-5-yl)-3-oxa-9-azabicyclo[3.3.1]nonan-7-yl)-3-azabicyclo[3.1.0]hexane-6-carboxamide;
(1R,5S,6r)-N,N-diethyl-3-(9-(3-(trifluoromethyl)-1,2,4-oxadiazol-5-yl)-3-oxa-9-azabicyclo[3.3.1]nonan-7-yl)-3-azabicyclo[3.1.0]hexane-6-carboxamide;
(1R,5S,6r)-N-ethyl-N-methyl-3-(8-(3-methyl-1,2,4-oxadiazol-5-yl)-8-azabicyclo[3.2.1]octan-3-yl)-3-azabicyclo[3.1.0]hexane-6-carboxamide;
(1R,5S,6r)-N,N-diethyl-3-(8-(3-methyl-1,2,4-oxadiazol-5-yl)-8-azabicyclo[3.2.1]octan-3-yl)-3-azabicyclo[3.1.0]hexane-6-carboxamide;
(1R,5S,6r)-N,N-diethyl-3-(2-(3-methyl-1,2,4-oxadiazol-5-yl)-2-azaspiro[3.4]octan-6-yl)-3-azabicyclo[3.1.0]hexane-6-carboxamide;
(1R,5S,6r)-N-ethyl-N-methyl-3-(2-(3-methyl-1,2,4-oxadiazol-5-yl)-2-azaspiro[3.4]octan-6-yl)-3-azabicyclo[3.1.0]hexane-6-carboxamide;
N,N-diethyl-1-(2-(3-methyl-1,2,4-oxadiazol-5-yl)-2-azaspiro[3.4]octan-6-yl)piperidine-4-carboxamide;
8-(8-(3-methyl-1,2,4-oxadiazol-5-yl)-8-azabicyclo[3.2.1]octan-3-yl)-2,8-diazaspiro[4.6]undecan-3-one;
8-(8-(3-methyl-1,2,4-oxadiazol-5-yl)-8-azabicyclo[3.2.1]octan-3-yl)-1-oxa-3,8-diazaspiro[4.6]undecan-2-one;
8-(2-(3-methyl-1,2,4-oxadiazol-5-yl)-2-azabicyclo[2.2.2]octan-5-yl)-2,8-diazaspiro[4.6]undecan-3-one;
8-(2-(3-methyl-1,2,4-oxadiazol-5-yl)-2-azabicyclo[2.2.2]octan-5-yl)-1-oxa-3,8-diazaspiro[4.6]undecan-2-one;
8-(6-(3-methyl-1,2,4-oxadiazol-5-yl)-6-azabicyclo[3.2.1]octan-3-yl)-2,8-diazaspiro[4.6]undecan-3-one;
8-(6-(3-methyl-1,2,4-oxadiazol-5-yl)-6-azabicyclo[3.2.1]octan-3-yl)-1-oxa-3,8-diazaspiro[4.6]undecan-2-one;
((1R,5S,6r)-3-(6-(3-methyl-1,2,4-oxadiazol-5-yl)-6-azabicyclo[3.2.1]octan-3-yl)-3-azabicyclo[3.1.0]hexan-6-yl)(1-azaspiro[3.3]heptan-1-yl)methanone;
(1R,5S,6r)-N,N-diethyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-ethyl-N-isopropyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-isopropyl-N-methyl-3-(6-(3-methyl-1,2,4-oxadiazol-5-yl)-6-azabicyclo[3.2.1]octan-3-yl)-3-azabicyclo[3.1.0]hexane-6-carboxamide;
(1R,5S,6r)-N-ethyl-N-methyl-3-(9-(3-methyl-1,2,4-oxadiazol-5-yl)-9-azabicyclo[3.3.1]nonan-3-yl)-3-azabicyclo[3.1.0]hexane-6-carboxamide;
(1R,5S,6r)-N,N-diethyl-3-(9-(3-methyl-1,2,4-oxadiazol-5-yl)-9-azabicyclo[3.3.1]nonan-3-yl)-3-azabicyclo[3.1.0]hexane-6-carboxamide;
8-(1-(3-(trifluoromethyl)-1,2,4-oxadiazol-5-yl)azepan-4-yl)-2,8-diazaspiro[4.5]decan-3-one;
8-(1-(3-(trifluoromethyl)-1,2,4-oxadiazol-5-yl)azepan-4-yl)-2,8-diazaspiro[4.6]undecan-3-one;
8-(1-(3-methyl-1,2,4-oxadiazol-5-yl)azepan-4-yl)-2,8-diazaspiro[4.6]undecan-3-one;
8-(1-(3-(trifluoromethyl)-1,2,4-oxadiazol-5-yl)azepan-4-yl)-1-oxa-3,8-diazaspiro[4.5]decan-2-one;
8-(8-(3-ethyl-1,2,4-oxadiazol-5-yl)-8-azabicyclo[3.2.1]octan-3-yl)-1-oxa-3,8-diazaspiro[4.5]decan-2-one;
8-(2-(3-methyl-1,2,4-oxadiazol-5-yl)-2-azabicyclo[2.2.2]octan-5-yl)-1-oxa-3,8-diazaspiro[4.5]decan-2-one;
8-(6-(3-(trifluoromethyl)-1,2,4-oxadiazol-5-yl)-6-azabicyclo[3.2.1]octan-3-yl)-1-oxa-3,8-diazaspiro[4.5]decan-2-one;
4-methyl-8-(8-(3-methyl-1,2,4-oxadiazol-5-yl)-8-azabicyclo[3.2.1]octan-3-yl)-1-oxa-3,8-diazaspiro[4.5]decan-2-one;
(1R,5S,6r)-3-(6-(3-methyl-1,2,4-oxadiazol-5-yl)-6-azaspiro[3.4]octan-2-yl)-N-(1-methylcyclobutyl)-3-azabicyclo[3.1.0]hexane-6-carboxamide;
N-(1-(6-(3-methyl-1,2,4-oxadiazol-5-yl)-6-azaspiro[3.4]octan-2-yl)piperidin-4-yl)-N-((1-methylcyclobutyl)methyl)acetamide;
N-(1-(9-(3-methyl-1,2,4-oxadiazol-5-yl)-3-oxa-9-azabicyclo[3.3.1]nonan-7-yl)piperidin-4-yl)-N-((1-methylcyclobutyl)methyl)acetamide;
N-(1-(8-(3-methyl-1,2,4-oxadiazol-5-yl)-8-azabicyclo[3.2.1]octan-3-yl)piperidin-4-yl)-N-((1-methylcyclobutyl)methyl)acetamide;
(1R,5S,6r)-N-isopropyl-N-methyl-3-(8-(3-methyl-1,2,4-oxadiazol-5-yl)-8-azabicyclo[3.2.1]octan-3-yl)-3-azabicyclo[3.1.0]hexane-6-carboxamide;
(1R,5S,6r)-N-ethyl-N-methyl-3-(2-(3-methyl-1,2,4-oxadiazol-5-yl)-2-azabicyclo[2.2.2]octan-5-yl)-3-azabicyclo[3.1.0]hexane-6-carboxamide;
(1R,5S,6r)-N-ethyl-N-methyl-3-(9-(3-methyl-1,2,4-oxadiazol-5-yl)-9-azabicyclo[3.3.1]nonan-3-yl)-3-azabicyclo[3.1.0]hexane-6-carboxamide;
8-(1-(3-methyl-1,2,4-oxadiazol-5-yl)azepan-4-yl)-1-oxa-3,8-diazaspiro[4.5]decan-2-one;
8-(1-(3-(trifluoromethyl)-1,2,4-oxadiazol-5-yl)azepan-4-yl)-2,8-diazaspiro[4.5]decan-3-one;
8-(1-(3-(trifluoromethyl)-1,2,4-oxadiazol-5-yl)azepan-4-yl)-1-oxa-3,8-diazaspiro[4.5]decan-2-one;
8-(8-(3-cyclopropyl-1,2,4-oxadiazol-5-yl)-8-azabicyclo[3.2.1]octan-3-yl)-2,8-diazaspiro[4.5]decan-3-one;
8-(8-(3-isopropyl-1,2,4-oxadiazol-5-yl)-8-azabicyclo[3.2.1]octan-3-yl)-2,8-diazaspiro[4.5]decan-3-one;
8-(8-(3-ethyl-1,2,4-oxadiazol-5-yl)-8-azabicyclo[3.2.1]octan-3-yl)-1-oxa-3,8-diazaspiro[4.5]decan-2-one;
4-benzyl-8-(8-(3-methyl-1,2,4-oxadiazol-5-yl)-8-azabicyclo[3.2.1]octan-3-yl)-1-oxa-3,8-diazaspiro[4.5]decan-2-one;
1-isopropyl-8-(8-(3-methyl-1,2,4-oxadiazol-5-yl)-8-azabicyclo[3.2.1]octan-3-yl)-2,8-diazaspiro[4.5]decan-3-one;
4-isopropyl-8-(8-(3-methyl-1,2,4-oxadiazol-5-yl)-8-azabicyclo[3.2.1]octan-3-yl)-1-oxa-3,8-diazaspiro[4.5]decan-2-one;
1-cyclopropyl-8-(8-(3-methyl-1,2,4-oxadiazol-5-yl)-8-azabicyclo[3.2.1]octan-3-yl)-2,8-diazaspiro[4.5]decan-3-one;
8-(8-(3-cyclopropyl-1,2,4-oxadiazol-5-yl)-8-azabicyclo[3.2.1]octan-3-yl)-1-oxa-3,8-diazaspiro[4.5]decan-2-one;
8-(8-(3-isopropyl-1,2,4-oxadiazol-5-yl)-8-azabicyclo[3.2.1]octan-3-yl)-1-oxa-3,8-diazaspiro[4.5]decan-2-one;
N-benzyl-N-(1-(8-(3-methyl-1,2,4-oxadiazol-5-yl)-8-azabicyclo[3.2.1]octan-3-yl)piperidin-4-yl)acetamide;
(1R,5S,6r)-N,N-diethyl-3-(2-(3-methyl-1,2,4-oxadiazol-5-yl)-2-azaspiro[3.3]heptan-6-yl)-3-azabicyclo[3.1.0]hexane-6-carboxamide;
((1R,5S,6r)-3-(6-(3-methyl-1,2,4-oxadiazol-5-yl)-6-azaspiro[3.4]octan-2-yl)-3-azabicyclo[3.1.0]hexan-6-yl)(1-azaspiro[3.3]heptan-1-yl)methanone;
azepan-1-yl(1-(8-(3-methyl-1,2,4-oxadiazol-5-yl)-8-azabicyclo[3.2.1]octan-3-yl)piperidin-4-yl)methanone;
1-(cyclopropylmethyl)-8-(8-(3-methyl-1,2,4-oxadiazol-5-yl)-8-azabicyclo[3.2.1]octan-3-yl)-2,8-diazaspiro[4.5]decan-3-one;
4-(cyclopropylmethyl)-8-(8-(3-methyl-1,2,4-oxadiazol-5-yl)-8-azabicyclo[3.2.1]octan-3-yl)-1-oxa-3,8-diazaspiro[4.5]decan-2-one;
4-cyclopropyl-8-(8-(3-methyl-1,2,4-oxadiazol-5-yl)-8-azabicyclo[3.2.1]octan-3-yl)-1-oxa-3,8-diazaspiro[4.5]decan-2-one;
1-benzyl-8-(6-(3-methyl-1,2,4-oxadiazol-5-yl)-6-azaspiro[3.4]octan-2-yl)-2,8-diazaspiro[4.5]decan-3-one;
4-benzyl-8-(2-(3-methyl-1,2,4-oxadiazol-5-yl)-2-azaspiro[3.3]heptan-6-yl)-1-oxa-3,8-diazaspiro[4.5]decan-2-one;
1-benzyl-8-(1-(3-methyl-1,2,4-oxadiazol-5-yl)azepan-4-yl)-2,8-diazaspiro[4.5]decan-3-one;
8-(8-(3-methyl-1,2,4-oxadiazol-5-yl)-8-azabicyclo[3.2.1]octan-3-yl)-2,8-diazaspiro[4.6]undecan-3-one;
1-benzyl-8-(8-(3-methyl-1,2,4-oxadiazol-5-yl)-8-azabicyclo[3.2.1]octan-3-yl)-2,8-diazaspiro[4.5]decan-3-one;
8-(8-(3-methyl-1,2,4-oxadiazol-5-yl)-8-azabicyclo[3.2.1]octan-3-yl)-1-oxa-3,8-diazaspiro[4.6]undecan-2-one;
(1R,5S,6r)-3-(8-(3-methyl-1,2,4-oxadiazol-5-yl)-8-azabicyclo[3.2.1]octan-3-yl)-N-(1-(trifluoromethyl)cyclobutyl)-3-azabicyclo[3.1.0]hexane-6-carboxamide; and
(1R,5S,6r)-3-(2-(3-methyl-1,2,4-oxadiazol-5-yl)-2-azabicyclo[2.2.2]octan-5-yl)-N-(1-(trifluoromethyl)cyclobutyl)-3-azabicyclo[3.1.0]hexane-6-carboxamide;
or a salt thereof.

13. The compound according to claim 1, or a salt thereof, which is:
N-(1-methylcyclobutyl)-1-(8-(3-(trifluoromethyl)-1,2,4-oxadiazol-5-yl)-8-azabicyclo[3.2.1]octan-3-yl)piperidine-4-carboxamide;
or a salt thereof.

14. The compound according to claim 1, which is:
N-(1-methylcyclobutyl)-1-(8-(3-(trifluoromethyl)-1,2,4-oxadiazol-5-yl)-8-azabicyclo[3.2.1]octan-3-yl)piperidine-4-carboxamide.

15. The compound according to claim 1, or a salt thereof, which is:
(1R,5S,6r)-N,N-diethyl-3-(9-(3-(trifluoromethyl)-1,2,4-oxadiazol-5-yl)-3-oxa-9-azabicyclo[3.3.1]nonan-7-yl)-3-azabicyclo[3.1.0]hexane-6-carboxamide;
or a salt thereof.

16. The compound according to claim 1, which is:
(1R,5S,6r)-N,N-diethyl-3-(9-(3-(trifluoromethyl)-1,2,4-oxadiazol-5-yl)-3-oxa-9-azabicyclo[3.3.1]nonan-7-yl)-3-azabicyclo[3.1.0]hexane-6-carboxamide.

17. The compound according to any one of claims 1 to 16, or a salt thereof, for use in medicine.

18. A pharmaceutical composition comprising a compound as defined in any one of claims 1 to 16, or a salt thereof, and a pharmaceutically acceptable excipient.

19. The compound according to any one of claims 1 to 16, or a salt thereof, or
the pharmaceutical composition of claim 18,
for use in the treatment of a cognitive disorder or psychotic disorder; or in the
treatment or lessening the severity of acute pain, chronic pain, neuropathic pain, or inflammatory pain.

20. The compound according to any one of claims 1 to 16, or a salt thereof, or
the pharmaceutical composition of claim 18,
for use in the treatment of a cognitive disorder or psychotic disorder.

21. The compound according to any one of claims 1 to 16, or a salt thereof, or
the pharmaceutical composition of claim 18,
for use in the treatment of a cognitive disorder.

22. The compound, salt, or composition for use according to any one of claims 19-21, wherein:
the cognitive disorder arises from or is associated with Alzheimer's disease.

23. The compound, salt, or composition for use according to any one of claims 19-21, wherein:
the cognitive disorder is dementia with Lewy bodies.

24. The compound according to any one of claims 1 to 16, or a salt thereof, or
the pharmaceutical composition of claim 18,
for use in the treatment of Alzheimer's disease.

## Patentansprüche

1. Verbindung der Formel (1): 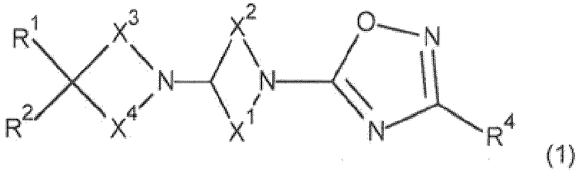
oder ein Salz davon,
worin:
X¹ und X² gesättigte Kohlenwasserstoffgruppen sind, die zusammen insgesamt fünf bis neun Kohlenstoffatome und null oder ein Sauerstoffatom(e) enthalten und so verbunden sind, dass die Gruppierung: 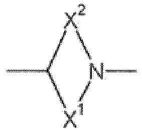
ein monozyklisches oder bizyklisches Ringsystem bildet;
X³ und X⁴ gesättigte Kohlenwasserstoffgruppen sind, die zusammen insgesamt drei bis sechs Kohlenstoffatome enthalten und so verbunden sind, dass die Gruppierung: 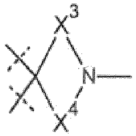
ein monozyklisches oder bizyklisches Ringsystem bildet;
R¹ aus NR⁵R⁶, CONR⁵R⁶, COOR⁷, einem gegebenenfalls substituierten 5- oder 6-gliedrigen Ring, der 0, 1, 2 oder 3 Heteroatome enthält, die aus O, N und S und oxidierten Formen davon ausgewählt sind, ausgewählt ist, wobei der optionale Substituent des 5- oder 6-gliedrigen Rings aus C₁-C₃-Alkyl, C₁-C₃-Alkoxy, CONR⁵R⁶, Halogen, Cyano, Oxo, Hydroxyl, Amino oder Pyrazol, das gegebenenfalls mit C₁-C₃-Alkyl substituiert ist, ausgewählt ist; oder R¹ mit R² verbunden ist, um einen gegebenenfalls substituierten, monozyklischen oder bizyklischen Ring, der 0, 1, 2 oder 3 Heteroatome enthält, die aus O, N und S und oxidierten Formen davon ausgewählt sind, zu bilden, wobei die optionalen Substituenten des monozyklischen oder bizyklischen Rings aus C₁-C₆-Alkyl, C₁-C₃-Alkoxy, Halogen, Cyano, Oxo, Hydroxyl, Amino und einer Gruppe -(CH₂)ₙ-Aryl, worin n = 0 bis 3 ist, ausgewählt sind;
R² aus Wasserstoff, Fluor, Cyano, Hydroxy, Amino und einer C₁₋₃-Kohlenwasserstoffgruppe ausgewählt ist, die gegebenenfalls mit ein bis sechs Fluoratomen substituiert ist und wobei eines der Kohlenstoffatome der Kohlenwasserstoffgruppe gegebenenfalls mit einem Heteroatom substituiert ist, das aus O, N und S und oxidierten Formen davon ausgewählt ist; oder R² mit R¹ verbunden ist, um einen gegebenenfalls substituierten, monozyklischen oder bizyklischen Ring, der 0, 1, 2 oder 3 Heteroatome enthält, die aus O, N und S und oxidierten Formen davon ausgewählt sind, zu bilden, wobei die optionalen Substituenten des monozyklischen oder bizyklischen Rings aus C₁-C₆-Alkyl, C₁-C₃-Alkoxy, Halogen, Cyano, Oxo, Hydroxyl, Amino und einer Gruppe (CH₂)ₙ-Aryl, worin n = 0 bis 3 ist, ausgewählt sind;
R⁴ H, Halogen, OH, CN, eine C₁₋₆-Alkylgruppe oder eine C₃₋₆-Cycloalkylgruppe ist, wobei die Alkyl- und die Cycloalkylgruppe gegebenenfalls mit einem oder mehreren Fluoratomen substituiert sind und wobei ein beliebiges Atom der Alkyl- oder der Cycloalkylgruppe gegebenenfalls durch ein O-Heteroatom ersetzt ist;
R⁵ aus Wasserstoff, COCH₃ und einer nichtaromatischen C₁₋₁₀-Kohlenwasserstoffgruppe, die gegebenenfalls mit einem oder mehreren Fluoratomen substituiert ist und wobei ein beliebiges Atom der C₁₋₁₀-Kohlenwasserstoffgruppe gegebenenfalls durch ein Heteroatom ersetzt ist, das aus O, N und S ausgewählt ist, und einer Gruppe CH₂)ₙ-Aryl, worin n = 0 bis 3 ist, ausgewählt ist; oder R⁵ mit R⁶ verbunden ist, um einen gegebenenfalls substituierten monozyklischen oder bizyklischen Ring, der 0, 1, 2 oder 3 Heteroatome enthält, die aus O, N und S und oxidierten Formen davon ausgewählt sind, zu bilden, wobei die optionalen Substituenten aus C₁-C₃-Alkyl, C₁-C₃-Alkoxy, Halogen, Cyano, Oxo, Hydroxyl und Amino ausgewählt sind, wobei die C₁-C₃-Alkyl- oder C₁-C₃-Alkoxygruppen gegebenenfalls einen optionalen Substituenten aufweisen, der aus Halogen, Cyano, Oxo, Hydroxyl und Amino ausgewählt ist;
R⁶ aus Wasserstoff, einer nichtaromatischen C₁₋₁₀-Kohlenwasserstoffgruppe, die gegebenenfalls mit einem oder mehreren Fluoratomen substituiert ist und wobei ein beliebiges Atom der C₁₋₁₀-Kohlenwasserstoffgruppe gegebenenfalls durch ein Heteroatom ersetzt ist, das aus O, N und S ausgewählt ist, und einer Gruppe (CH₂)ₙ-Aryl, worin n = 0 bis 3 ist, ausgewählt ist; oder R⁶ mit R⁵ verbunden ist, um einen gegebenenfalls substituierten monozyklischen oder bizyklischen Ring, der 0, 1, 2 oder 3 Heteroatome enthält, die aus O, N und S und oxidierten Formen davon ausgewählt sind, zu bilden, wobei die optionalen Substituenten aus C₁-C₃-Alkyl, C₁-C₃-Alkoxy, Halogen, Cyano, Oxo, Hydroxyl und Amino ausgewählt sind, wobei die C₁-C₃-Alkyl- C₁-C₃-Alkoxygruppen gegebenenfalls einen optionalen Substituenten aufweisen, der aus Halogen, Cyano, Oxo, Hydroxyl und Amino ausgewählt ist; und
R⁷ eine nichtaromatische C₁₋₆-Kohlenwasserstoffgruppe ist, die gegebenenfalls mit einem oder mehreren Fluoratomen substituiert ist.

2. Verbindung nach Anspruch 1 oder ein Salz davon, worin:
R¹ aus NR⁵R⁶, CONR⁵R⁶ und COOR⁷ ausgewählt ist; oder
R¹ ein gegebenenfalls substituierter 5- bis 6-gliedriger Ring ist, der 0, 1 oder 2 Stickstoff-Heteroatome enthält, wobei die optionalen Substituenten aus C₁-C₃-Alkyl, C₁-C₃-Alkoxy, CONR⁵R⁶, Halogen, Cyano, Oxo, Hydroxyl, Amino und einem gegebenenfalls substituierten heterozyklischen Ring, der 1 oder 2 Heteroatome enthält, die aus O oder N ausgewählt sind, ausgewählt sind, wobei der optionale Substituent C₁-C₃-Alkyl ist.

3. Verbindung nach Anspruch 1 oder Anspruch 2 oder ein Salz davon, worin:
R¹ aus NR⁵R⁶, CONR⁵R⁶ und COOR⁷ ausgewählt ist.

4. Verbindung nach einem der Ansprüche 1 bis 3 oder ein Salz davon, worin:
R² H ist.

5. Verbindung nach einem der Ansprüche 1 bis 4 oder ein Salz davon, worin:
R¹ aus NR⁵R⁶, CONR⁵R⁶ und COOR⁷ ausgewählt ist;
R⁵ aus COCH₃ und einer nichtaromatischen C₁₋₆-Kohlenwasserstoffgruppe ausgewählt ist; und
R⁶ aus Wasserstoff und einer nichtaromatischen C₁₋₆-Kohlenwasserstoffgruppe ausgewählt ist.

6. Verbindung nach einem der Ansprüche 1 bis 5 oder ein Salz davon, wobei die Verbindung der Formel (26) entspricht: 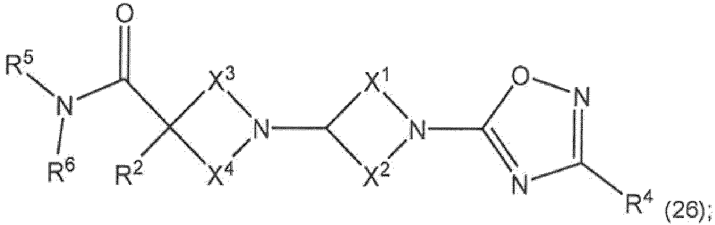
oder ein Salz davon;
worin:
R², R⁴, R⁵, R⁶, X¹, X², X³ und X⁴ wie in einem der Ansprüche 1 bis 5 definiert sind.

7. Verbindung nach Anspruch 1 oder ein Salz davon, worin R¹ aus Folgendem ausgewählt ist:

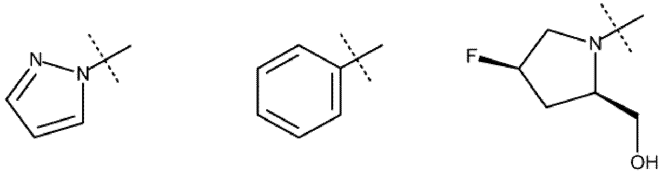

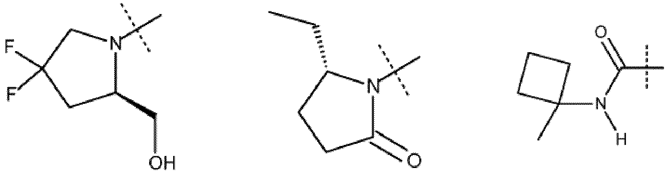

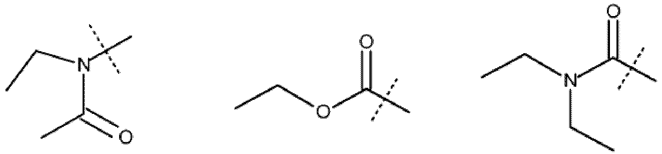

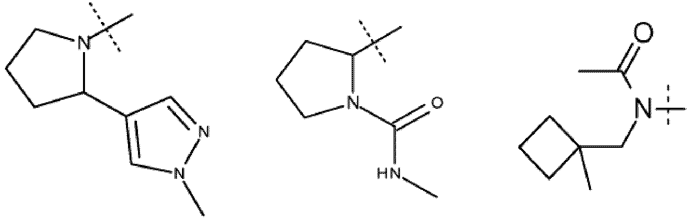

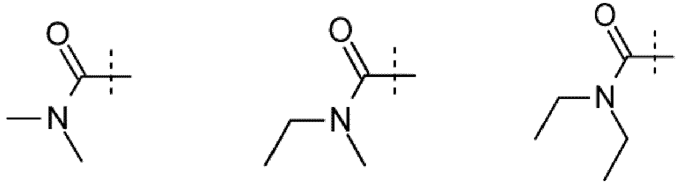

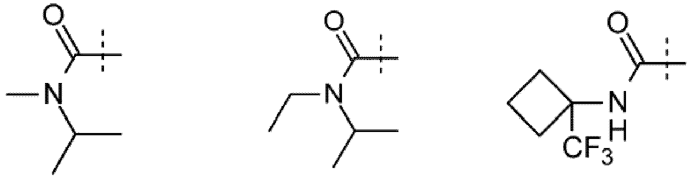

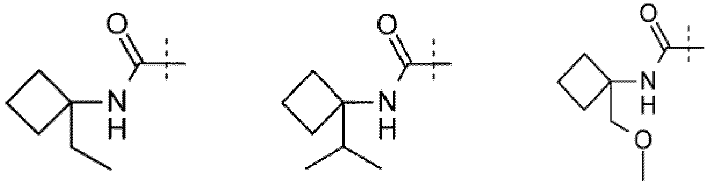

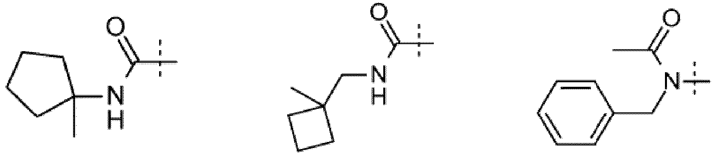

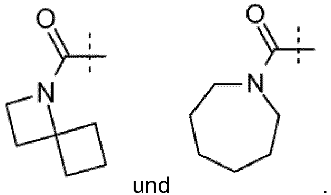

8. Verbindung nach einem der Ansprüche 1 bis 7 oder ein Salz davon, worin:
R⁴ Methyl, Trifluormethyl, Ethyl, Isopropyl oder Cyclopropyl ist.

9. Verbindung nach einem der Ansprüche 1 bis 3 und 5 bis 8 oder ein Salz davon, worin:
R⁴ Trifluormethyl ist.

10. Verbindung nach einem der Ansprüche 1 bis 9 oder ein Salz davon, wobei das durch die Gruppierung: 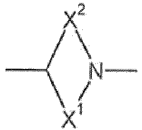 gebildete Ringsystem aus den folgenden ausgewählt ist: 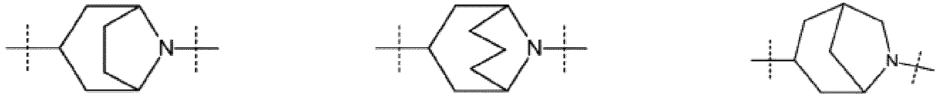 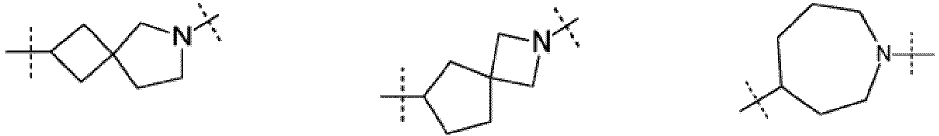 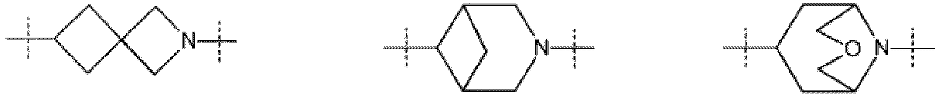 und

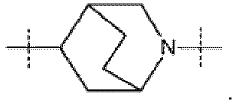

11. Verbindung nach Anspruch 1 oder ein Salz davon, die ein Verbindung der Formel (2) ist: 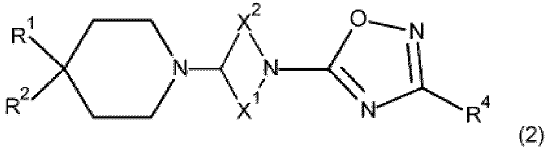
oder ein Salz davon;
worin:
X¹, X², R¹, R² und R⁴ wie in einem der Ansprüche 1 bis 10 definiert sind.

12. Verbindung nach Anspruch 1 oder ein Salz davon, die aus den folgenden ausgewählt ist:
2-(3-Methyl-1,2,4-oxadiazol-5-yl)-6-[4-(1H-pyrazol-1-yl)piperidin-1-yl]-2-azaspiro[3.3]-heptan;
6-(3-Methyl-1,2,4-oxadiazol-5-yl)-2-[4-(1H-pyrazol-1-yl)piperidin-1-yl]-6-azaspiro[3.4]-octan;
1-[6-(3-Methyl-1,2,4-oxadiazol-5-yl)-6-azaspiro[3.4]oct-2-yl]-4-phenylpiperidin-4-carbonitril;
Ethyl-(1R,5S,6r)-3-[6-(3-methyl-1,2,4-oxadiazol-5-yl)-6-azaspiro[3.4]oct-2-yl]-3-azabicyclo[3.1.0]hexan-6-carboxylat;
2-(3-Methyl-1,2,4-oxadiazol-5-yl)-6-[4-(1H-pyrazol-1-yl)piperidin-1-yl]-2-azaspiro[3.4]-octan;
3-(3-Methyl-1,2,4-oxadiazol-5-yl)-6-[4-(1H-pyrazol-1-yl)piperidin-1-yl]-3-azabicyclo-[3.1.1]heptan;
8-(3-Methyl-1,2,4-oxadiazol-5-yl)-3-[4-(1H-pyrazol-1-yl)piperidin-1-yl]-8-azabicyclo-[3.2.1]octan;
8-[8-(3-Methyl-1,2,4-oxadiazol-5-yl)-8-azabicyclo[3.2.1]oct-3-yl]-2,8-diazaspiro[4.5]-decan-3-on;
8-[8-(3-Ethyl-1,2,4-oxadiazol-5-yl)-8-azabicyclo[3.2.1]oct-3-yl]-2,8-diazaspiro[4.5]-decan-3-on;
8-[8-(3-Methyl-1,2,4-oxadiazol-5-yl)-8-azabicyclo[3.2.1]octan-3-yl]-1-oxa-3,8-diazaspiro[4.5]decan-2-on;
N-(1-Methylcyclobutyl)-1-[8-(3-methyl-1,2,4-oxadiazol-5-yl)-8-azabicyclo[3.2.1]oct-3-yl]piperidin-4-carboxamid;
2-(3-Methyl-1,2,4-oxadiazol-5-yl)-5-[4-(1H-pyrazol-1-yl)piperidin-1-yl]-2-azabicyclo-[2.2.2]octan;
9-(3-Methyl-1,2,4-oxadiazol-5-yl)-3-[4-(1H-pyrazol-1-yl)piperidin-1-yl]-9-azabicyclo-[3.3.1]nonan;
8-[9-(3-Methyl-1,2,4-oxadiazol-5-yl)-9-azabicyclo[3.3.1]non-3-yl]-2,8-diazaspiro[4.5]-decan-3-on;
8-[9-(3-Methyl-1,2,4-oxadiazol-5-yl)-9-azabicyclo[3.3.1]non-3-yl]-1-oxa-3,8-diazaspiro-[4.5]decan-2-on;
N-(1-Methylcyclobutyl)-1-[9-(3-methyl-1,2,4-oxadiazol-5-yl)-9-azabicyclo[3.3.1]non-3-yl]piperidin-4-carboxamid;
9-(3-Methyl-1,2,4-oxadiazol-5-yl)-7-[4-(1H-pyrazol-1-yl)piperidin-1-yl]-3-oxa-9-azabicyclo[3.3.1]nonan;
(1R,5S,6r)-N,N-Diethyl-3-[9-(3-methyl-1,2,4-oxadiazol-5-yl)-3-oxa-9-azabicyclo[3.3.1]-non-7-yl]-3-azabicyclo[3.1.0]hexan-6-carboxamid;
1-(3-Methyl-1,2,4-oxadiazol-5-yl)-4-[4-(1H-pyrazol-1-yl)piperidin-1-yl]azepan;
8-[1-(3-Methyl-1,2,4-oxadiazol-5-yl)azepan-4-yl]-2,8-diazaspiro[4.5]decan-3-on;
8-[1-(3-Methyl-1,2,4-oxadiazol-5-yl)azepan-4-yl]-1-oxa-3,8-diazaspiro[4.5]decan-2-on;
4-Ethyl-8-[1-(3-methyl-1,2,4-oxadiazol-5-yl)azepan-4-yl]-1-oxa-3,8-diazaspiro[4.5]-decan-2-on;
4,4-Dimethyl-8-[1-(3-methyl-1,2,4-oxadiazol-5-yl)azepan-4-yl]-1-oxa-3,8-diazaspiro-[4.5]decan-2-on;
1'-[1-(3-Methyl-1,2,4-oxadiazol-5-yl)azepan-4-yl]spiro[indol-3,4'-piperidin]-2(1H)-on;
[(2R,4R)-4-Fluor-1-{1-[1-(3-methyl-1,2,4-oxadiazol-5-yl)azepan-4-yl]piperidin-4-yl}-pyrrolidin-2-yl]methanol;
[(2R)-4,4-Difluor-1-{1-[1-(3-methyl-1,2,4-oxadiazol-5-yl)azepan-4-yl]piperidin-4-yl}-pyrrolidin-2-yl]methanol;
(5S)-5-Ethyl-1-{1-[1-(3-methyl-1,2,4-oxadiazol-5-yl)azepan-4-yl]piperidin-4-yl}-pyrrolidin-2-on;
1-(3-Methyl-1,2,4-oxadiazol-5-yl)-4-{4-[2-(1-methyl-1H-pyrazol-4-yl)pyrrolidin1-yl]-piperidin-1-yl}azepan;
N-Ethyl-N-{1-[1-(3-methyl-1,2,4-oxadiazol-5-yl)azepan-4-yl]piperidin-4-yl}acetamid;
(2S)-N-Methyl-2-{1-[1-(3-methyl-1,2,4-oxadiazol-5-yl)azepan-4-yl]piperidin-4-yl}-pyrrolidin-1-carboxamid;
6-(3-Methyl-1,2,4-oxadiazol-5-yl)-3-[4-(1H-pyrazol-1-yl)piperidin-1-yl]-6-azabicyclo-[3.2.1]octan;
8-[6-(3-Methyl-1,2,4-oxadiazol-5-yl)-6-azabicyclo[3.2.1]oct-3-yl]-2,8-diazaspiro[4.5]-decan-3-on;
8-[6-(3-Methyl-1,2,4-oxadiazol-5-yl)-6-azabicyclo[3.2.1]oct-3-yl]-1-oxa-3,8-diazaspiro-[4.5]decan-2-on;
8-(2-(3-Methyl-1,2,4-oxadiazol-5-yl)-2-azabicyclo[2.2.2]octan-5-yl)-2,8-diazaspiro[4.5]-decan-3-on;
8-(2-(3-Methyl-1,2,4-oxadiazol-5-yl)-2-azabicyclo[2.2.2]octan-5-yl)-1-oxa-3,8-diazaspiro[4.5]decan-2-on;
8-(8-(3-(Trifluormethyl)-1,2,4-oxadiazol-5-yl)-8-azabicyclo[3.2.1]octan-3-yl)-2,8-diazaspiro[4.5]decan-3-on;
8-(8-(3-(Trifluormethyl)-1,2,4-oxadiazol-5-yl)-8-azabicyclo[3.2.1]octan-3-yl)-1-oxa-3,8-diazaspiro[4.5]decan-2-on;
(1R,5S,6r)-3-(2-(3-Methyl-1,2,4-oxadiazol-5-yl)-2-azaspiro[3.4]octan-6-yl)-N-(1-methylcyclobutyl)-3-azabicyclo[3.1.0]hexan-6-carboxamid;
1-(8-(3-Methyl-1,2,4-oxadiazol-5-yl)-8-azabicyclo[3.2.1]octan-3-yl)-N-(1-(trifluormethyl)cyclobutyl) piperidin-4-carboxamid;
N-(1-Ethylcyclobutyl)-1-(8-(3-methyl-1,2,4-oxadiazol-5-yl)-8-azabicyclo[3.2.1]octan-3-yl)piperidin-4-carboxamid;
N-(1-Isopropylcyclobutyl)-1-(8-(3-methyl-1,2,4-oxadiazol-5-yl)-8-azabicyclo[3.2.1]-octan-3-yl)piperidin-4-carboxamid;
1-(8-(3-Methyl-1,2,4-oxadiazol-5-yl)-8-azabicyclo[3.2.1]octan-3-yl)-N-(1-methylcyclopentyl)piperidin-4-carboxamid;
1-(8-(3-Methyl-1,2,4-oxadiazol-5-yl)-8-azabicyclo[3.2.1]octan-3-yl)-N-((1-methyl-cyclobutyl)methyl)piperidin-4-carboxamid;
(1R,5S,6r)-3-(8-(3-Methyl-1,2,4-oxadiazol-5-yl)-8-azabicyclo[3.2.1]octan-3-yl)-N-(1-methylcyclobutyl)-3-azabicyclo[3.1.0]hexan-6-carboxamid;
N-(1-Methylcyclobutyl)-1-(8-(3-(trifluormethyl)-1,2,4-oxadiazol-5-yl)-8-azabicyclo-[3.2.1]octan-3-yl)piperidin-4-carboxamid;
1-(2-(3-Methyl-1,2,4-oxadiazol-5-yl)-2-azabicyclo[2.2.2]octan-5-yl)-N-(1-methyl-cyclobutyl)piperidin-4-carboxamid;
1-(2-(3-Methyl-1,2,4-oxadiazol-5-yl)-2-azabicyclo[2.2.2]octan-5-yl)-N-((1-methyl-cyclobutyl)methyl)piperidin-4-carboxamid;
N-(1-(9-(3-Methyl-1,2,4-oxadiazol-5-yl)-3-oxa-9-azabicyclo[3.3.1]nonan-7-yl)piperidin-4-yl)-N-((1-methylcyclobutyl)methyl)acetamid;
N-(1-(8-(3-Methyl-1,2,4-oxadiazol-5-yl)-8-azabicyclo[3.2.1]octan-3-yl)piperidin-4-yl)-N-((1-methylcyclobutyl)methyl)acetamid;
N-(1-(2-(3-Methyl-1,2,4-oxadiazol-5-yl)-2-azabicyclo[2.2.2]octan-5-yl)piperidin-4-yl)-N-((1-methylcyclobutyl)methyl)acetamid;
N-Benzyl-N-(1-(9-(3-methyl-1,2,4-oxadiazol-5-yl)-3-oxa-9-azabicyclo[3.3.1]nonan-7-yl)piperidin-4-yl)acetamid;
((1R,5S,6r)-3-(9-(3-Methyl-1,2,4-oxadiazol-5-yl)-3-oxa-9-azabicyclo[3.3.1]nonan-7-yl)-3-azabicyclo[3.1.0]hexan-6-yl)(1-azaspiro[3.3]heptan-1-yl)methanon;
(1R,5S,6r)-N-Isopropyl-N-methyl-3-(9-(3-methyl-1,2,4-oxadiazol-5-yl)-3-oxa-9-azabicyclo[3.3.1]nonan-7-yl)-3-azabicyclo[3.1.0]hexan-6-carboxamid;
(1R,5S,6r)-N,N-Diethyl-3-(9-(3-(trifluormethyl)-1,2,4-oxadiazol-5-yl)-3-oxa-9-azabicyclo[3.3.1]nonan-7-yl)-3-azabicyclo[3.1.0]hexan-6-carboxamid;
(1R,5S,6r)-N-Ethyl-N-methyl-3-(8-(3-methyl-1,2,4-oxadiazol-5-yl)-8-azabicyclo[3.2.1]-octan-3-yl)-3-azabicyclo[3.1.0]hexan-6-carboxamid;
(1R,5S,6r)-N,N-Diethyl-3-(8-(3-methyl-1,2,4-oxadiazol-5-yl)-8-azabicyclo[3.2.1]octan-3-yl)-3-azabicyclo[3.1.0]hexan-6-carboxamid;
(1R,5S,6r)-N,N-Diethyl-3-(2-(3-methyl-1,2,4-oxadiazol-5-yl)-2-azaspiro[3.4]octan-6-yl)-3-azabicyclo[3.1.0]hexan-6-carboxamid;
(1R,5S,6r)-N-Ethyl-N-methyl-3-(2-(3-methyl-1,2,4-oxadiazol-5-yl)-2-azaspiro[3.4]-octan-6-yl)-3-azabicyclo[3.1.0]hexan-6-carboxamid;
N,N-Diethyl-1-(2-(3-methyl-1,2,4-oxadiazol-5-yl)-2-azaspiro[3.4]octan-6-yl)piperidin-4-carboxamid;
8-(8-(3-Methyl-1,2,4-oxadiazol-5-yl)-8-azabicyclo[3.2.1]octan-3-yl)-2,8-diazaspiro[4.6]-undecan-3-on;
8-(8-(3-Methyl-1,2,4-oxadiazol-5-yl)-8-azabicyclo[3.2.1]octan-3-yl)-1-oxa-3,8-diazaspiro[4.6]undecan-2-on;
8-(2-(3-Methyl-1,2,4-oxadiazol-5-yl)-2-azabicyclo[2.2.2]octan-5-yl)-2,8-diazaspiro[4.6]-undecan-3-on;
8-(2-(3-Methyl-1,2,4-oxadiazol-5-yl)-2-azabicyclo[2.2.2]octan-5-yl)-1-oxa-3,8-diazaspiro[4.6]undecan-2-on;
8-(6-(3-Methyl-1,2,4-oxadiazol-5-yl)-6-azabicyclo[3.2.1]octan-3-yl)-2,8-diazaspiro[4.6]-undecan-3-on;
8-(6-(3-Methyl-1,2,4-oxadiazol-5-yl)-6-azabicyclo[3.2.1]octan-3-yl)-1-oxa-3,8-diazaspiro[4.6]undecan-2-on;
((1R,5S,6r)-3-(6-(3-Methyl-1,2,4-oxadiazol-5-yl)-6-azabicyclo[3.2.1]octan-3-yl)-3-azabicyclo[3.1.0]hexan-6-yl)(1-azaspiro[3.3]heptan-1-yl)methanon;
(1R,5S,6r)-N,N-Diethyl-3-(6-(3-methyl-1,2,4-oxadiazol-5-yl)-6-azabicyclo[3.2.1]octan-3-yl)-3-azabicyclo[3.1.0]hexan-6-carboxamid;
(1R,5S,6r)-N-Ethyl-N-isopropyl-3-(6-(3-methyl-1,2,4-oxadiazol-5-yl)-6-azabicyclo-[3.2.1]octan-3-yl)-3-azabicyclo[3.1.0]hexan-6-carboxamid;
(1R,5S,6r)-N-Isopropyl-N-methyl-3-(6-(3-methyl-1,2,4-oxadiazol-5-yl)-6-azabicyclo-[3.2.1]octan-3-yl)-3-azabicyclo[3.1.0]hexan-6-carboxamid;
(1R,5S,6r)-N-Ethyl-N-methyl-3-(9-(3-methyl-1,2,4-oxadiazol-5-yl)-9-azabicyclo[3.3.1]-nonan-3-yl)-3-azabicyclo[3.1.0]hexan-6-carboxamid;
(1R,5S,6r)-N,N-Diethyl-3-(9-(3-methyl-1,2,4-oxadiazol-5-yl)-9-azabicyclo[3.3.1]nonan-3-yl)-3-azabicyclo[3.1.0]hexan-6-carboxamid;
8-(1-(3-(Trifluormethyl)-1,2,4-oxadiazol-5-yl)azepan-4-yl)-2,8-diazaspiro[4.5]decan-3-on;
8-(1-(3-(Trifluormethyl)-1,2,4-oxadiazol-5-yl)azepan-4-yl)-2,8-diazaspiro[4.6]undecan-3-on;
8-(1-(3-Methyl-1,2,4-oxadiazol-5-yl)azepan-4-yl)-2,8-diazaspiro[4.6]undecan3-on;
8-(1-(3-(Trifluormethyl)-1,2,4-oxadiazol-5-yl)azepan-4-yl)-1-oxa-3,8-diazaspiro[4.5]-decan-2-on;
8-(8-(3-Ethyl-1,2,4-oxadiazol-5-yl)-8-azabicyclo[3.2.1]octan-3-yl)-1-oxa-3,8-diazaspiro[4.5]decan-2-on;
8-(2-(3-Methyl-1,2,4-oxadiazol-5-yl)-2-azabicyclo[2.2.2]octan-5-yl)-1-oxa-3,8-diazaspiro[4.5]decan-2-on;
8-(6-(3-(Trifluormethyl)-1,2,4-oxadiazol-5-yl)-6-azabicyclo[3.2.1]octan-3-yl)-1-oxa-3,8-diazaspiro[4.5]decan-2-on;
4-Methyl-8-(8-(3-methyl-1,2,4-oxadiazol-5-yl)-8-azabicyclo[3.2.1]octan-3-yl)-1-oxa-3,8-diazaspiro[4.5]decan-2-on;
(1R,5S,6r)-3-(6-(3-Methyl-1,2,4-oxadiazol-5-yl)-6-azaspiro[3.4]octan-2-yl)-N-(1-methylcyclobutyl)-3-azabicyclo[3.1.0]hexan-6-carboxamid;
N-(1-(6-(3-Methyl-1,2,4-oxadiazol-5-yl)-6-azaspiro[3.4]octan-2-yl)piperidin-4-yl)-N-((1-methylcyclobutyl)methyl)acetamid;
N-(1-(9-(3-Methyl-1,2,4-oxadiazol-5-yl)-3-oxa-9-azabicyclo[3.3.1]nonan-7-yl)piperidin-4-yl)-N-((1-methylcyclobutyl)methyl)acetamid;
N-(1-(8-(3-Methyl-1,2,4-oxadiazol-5-yl)-8-azabicyclo[3.2.1]octan-3-yl)piperidin-4-yl)-N-((1-methylcyclobutyl)methyl)acetamid;
(1R,5S,6r)-N-Isopropyl-N-methyl-3-(8-(3-methyl-1,2,4-oxadiazol-5-yl)-8-azabicyclo-[3.2.1]octan-3-yl)-3-azabicyclo[3.1.0]hexan-6-carboxamid;
(1R,5S,6r)-N-Ethyl-N-methyl-3-(2-(3-methyl-1,2,4-oxadiazol-5-yl)-2-azabicyclo[2.2.2]-octan-5-yl)-3-azabicyclo[3.1.0]hexan-6-carboxamid;
(1R,5S,6r)-N-Ethyl-N-methyl-3-(9-(3-methyl-1,2,4-oxadiazol-5-yl)-9-azabicyclo[3.3.1]-nonan-3-yl)-3-azabicyclo[3.1.0]hexan-6-carboxamid;
8-(1-(3-Methyl-1,2,4-oxadiazol-5-yl)azepan-4-yl)-1-oxa-3,8-diazaspiro[4.5]decan-2-on;
8-(1-(3-(Trifluormethyl)-1,2,4-oxadiazol-5-yl)azepan-4-yl)-2,8-diazaspiro[4.5]decan-3-on;
8-(1-(3-(Trifluormethyl)-1,2,4-oxadiazol-5-yl)azepan-4-yl)-1-oxa-3,8-diazaspiro[4.5]-decan-2-on;
8-(8-(3-Cyclopropyl-1,2,4-oxadiazol-5-yl)-8-azabicyclo[3.2.1]octan-3-yl)-2,8-diazaspiro[4.5]decan-3-on;
8-(8-(3-Isopropyl-1,2,4-oxadiazol-5-yl)-8-azabicyclo[3.2.1]octan-3-yl)-2,8-diazaspiro-[4.5]decan-3-on;
8-(8-(3-Ethyl-1,2,4-oxadiazol-5-yl)-8-azabicyclo[3.2.1]octan-3-yl)-1-oxa-3,8-diazaspiro[4.5]decan-2-on;
4-Benzyl-8-(8-(3-methyl-1,2,4-oxadiazol-5-yl)-8-azabicyclo[3.2.1]octan-3-yl)-1-oxa-3,8-diazaspiro[4.5]decan-2-on;
1-Isopropyl-8-(8-(3-methyl-1,2,4-oxadiazol-5-yl)-8-azabicyclo[3.2.1]octan-3-yl)-2,8-diazaspiro[4.5]decan-3-on;
4-Isopropyl-8-(8-(3-methyl-1,2,4-oxadiazol-5-yl)-8-azabicyclo[3.2.1]octan-3-yl)-1-oxa-3,8-diazaspiro[4.5]decan-2-on;
1-Cyclopropyl-8-(8-(3-methyl-1,2,4-oxadiazol-5-yl)-8-azabicyclo[3.2.1]octan-3-yl)-2,8-diazaspiro[4.5]decan-3-on;
8-(8-(3-Cyclopropyl-1,2,4-oxadiazol-5-yl)-8-azabicyclo[3.2.1]octan-3-yl)-1-oxa-3,8-diazaspiro[4.5]decan-2-on;
8-(8-(3-Isopropyl-1,2,4-oxadiazol-5-yl)-8-azabicyclo[3.2.1]octan-3-yl)-1-oxa-3,8-diazaspiro[4.5]decan-2-on;
N-Benzyl-N-(1-(8-(3-methyl-1,2,4-oxadiazol-5-yl)-8-azabicyclo[3.2.1]octan-3-yl)-piperidin-4-yl)acetamid;
(1R,5S,6r)-N,N-Diethyl-3-(2-(3-methyl-1,2,4-oxadiazol-5-yl)-2-azaspiro[3.3]heptan-6-yl)-3-azabicyclo[3.1.0]hexan-6-carboxamid;
((1R,5S,6r)-3-(6-(3-Methyl-1,2,4-oxadiazol-5-yl)-6-azaspiro[3.4]octan-2-yl)-3-azabicyclo[3.1.0]hexan-6-yl)(1-azaspiro[3.3]heptan-1-yl)methanon;
Azepan-1-yl(1-(8-(3-methyl-1,2,4-oxadiazol-5-yl)-8-azabicyclo[3.2.1]octan-3-yl)-piperidin-4-yl)methanon;
1-(Cyclopropylmethyl)-8-(8-(3-methyl-1,2,4-oxadiazol-5-yl)-8-azabicyclo[3.2.1]octan-3-yl)-2,8-diazaspiro[4.5]decan-3-on;
4-(Cyclopropylmethyl)-8-(8-(3-methyl-1,2,4-oxadiazol-5-yl)-8-azabicyclo[3.2.1]octan-3-yl)-1-oxa-3,8-diazaspiro[4.5]decan-2-on;
4-Cyclopropyl-8-(8-(3-methyl-1,2,4-oxadiazol-5-yl)-8-azabicyclo[3.2.1]octan-3-yl)-1-oxa-3,8-diazaspiro[4.5]decan-2-on;
1-Benzyl-8-(6-(3-methyl-1,2,4-oxadiazol-5-yl)-6-azaspiro[3.4]octan-2-yl)-2,8-diazaspiro[4.5]decan-3-on;
4-Benzyl-8-(2-(3-methyl-1,2,4-oxadiazol-5-yl)-2-azaspiro[3.3]heptan-6-yl)-1-oxa-3,8-diazaspiro[4.5]decan-2-on;
1-Benzyl-8-(1-(3-methyl-1,2,4-oxadiazol-5-yl)azepan-4-yl)-2,8-diazaspiro[4.5]decan-3-on;
8-(8-(3-Methyl-1,2,4-oxadiazol-5-yl)-8-azabicyclo[3.2.1]octan-3-yl)-2,8-diazaspiro[4.6]-undecan-3-on;
1-Benzyl-8-(8-(3-methyl-1,2,4-oxadiazol-5-yl)-8-azabicyclo[3.2.1]octan-3-yl)-2,8-diazaspiro[4.5]decan-3-on;
8-(8-(3-Methyl-1,2,4-oxadiazol-5-yl)-8-azabicyclo[3.2.1]octan-3-yl)-1-oxa-3,8-diazaspiro[4.6]undecan-2-on;
(1R,5S,6r)-3-(8-(3-Methyl-1,2,4-oxadiazol-5-yl)-8-azabicyclo[3.2.1]octan-3-yl)-N-(1-(trifluormethyl)cyclobutyl)-3-azabicyclo[3.1.0]hexan-6-carboxamid; und
(1R,5S,6r)-3-(2-(3-Methyl-1,2,4-oxadiazol-5-yl)-2-azabicyclo[2.2.2]octan-5-yl)-N-(1-(trifluormethyl)cyclobutyl)-3-azabicyclo[3.1.0]hexan-6-carboxamid;
oder ein Salz davon.

13. Verbindung nach Anspruch 1 oder ein Salz davon, welche die folgende ist:
N-(1-Methylcyclobutyl)-1-(8-(3-(trifluormethyl)-1,2,4-oxadiazol-5-yl)-8-azabicyclo-[3.2.1]octan-3-yl)piperidin-4-carboxamid;
oder ein Salz davon.

14. Verbindung nach Anspruch 1, welche die folgende ist:
N-(1-Methylcyclobutyl)-1-(8-(3-(trifluormethyl)-1,2,4-oxadiazol-5-yl)-8-azabicyclo-[3.2.1]octan-3-yl)piperidin-4-carboxamid.

15. Verbindung nach Anspruch 1 oder ein Salz davon, welche die folgende ist:
(1R,5S,6r)-N,N-Diethyl-3-(9-(3-(trifluormethyl)-1,2,4-oxadiazol-5-yl)-3-oxa-9-azabicyclo[3.3.1]nonan-7-yl)-3-azabicyclo[3.1.0]hexan-6-carboxamid;
oder ein Salz davon.

16. Verbindung nach Anspruch 1, welche die folgende ist:
(1R,5S,6r)-N,N-Diethyl-3-(9-(3-(trifluormethyl)-1,2,4-oxadiazol-5-yl)-3-oxa-9-azabicyclo[3.3.1]nonan-7-yl)-3-azabicyclo[3.1.0]hexan-6-carboxamid.

17. Verbindung nach einem der Ansprüche 1 bis 16 oder ein Salz davon zur Verwendung in der Medizin.

18. Pharmazeutische Zusammensetzung, die eine Verbindung nach einem der Ansprüche 1 bis 16 oder ein Salz davon und einen pharmazeutisch annehmbaren Hilfsstoff umfasst.

19. Verbindung nach einem der Ansprüche 1 bis 16 oder ein Salz davon oder pharmazeutische Zusammensetzung nach Anspruch 18 zur Verwendung bei der Behandlung einer kognitiven Störung oder psychotischen Störung; oder bei der Behandlung oder Verringerung der Schwere von akutem Schmerz, chronischem Schmerz, neuropathischem Schmerz oder Entzündungsschmerz.

20. Verbindung nach einem der Ansprüche 1 bis 16 oder ein Salz davon oder pharmazeutische Zusammensetzung nach Anspruch 18 zur Verwendung bei der Behandlung einer kognitiven Störung oder psychotischen Störung.

21. Verbindung nach einem der Ansprüche 1 bis 16 oder ein Salz davon oder pharmazeutische Zusammensetzung nach Anspruch 18 zur Verwendung bei der Behandlung einer kognitiven Störung.

22. Verbindung, Salz oder Zusammensetzung zur Verwendung nach einem der Ansprüche 19 bis 21, wobei:
die kognitive Störung aus der Alzheimer-Krankheit resultiert oder mit dieser assoziiert ist.

23. Verbindung, Salz oder Zusammensetzung zur Verwendung nach einem der Ansprüche 19 bis 21, wobei:
die kognitive Störung Demenz mit Lewy-Körperchen ist.

24. Verbindung nach einem der Ansprüche 1 bis 16 oder ein Salz davon oder pharmazeutische Zusammensetzung nach Anspruch 18 zur Verwendung bei der Behandlung der Alzheimer-Krankheit.

## Revendications

1. Composé de formule (1) : 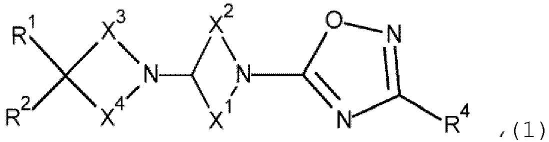
ou un sel de celui-ci,
dans lequel
X¹ et X² sont des groupes hydrocarbonés saturés qui contiennent ensemble un total de cinq à neuf atomes de carbone et zéro ou un atome d'oxygène et qui sont liés ensemble de sorte que le fragment : 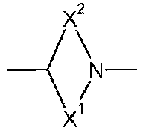
forme un système cyclique monocyclique ou bicyclique ;
X³ et X⁴ sont des groupes hydrocarbonés saturés qui contiennent ensemble un total de trois à six atomes de carbone et qui sont liés ensemble de sorte que le fragment : 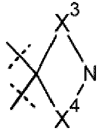
forme un système cyclique monocyclique ou bicyclique ; R¹ est choisi parmi NR⁵R⁶, CONR⁵R⁶, COOR⁷ ; un cycle à 5 ou 6 chaînons, éventuellement substitué, contenant 0, 1, 2 ou 3 hétéroatomes choisis parmi O, N et S et leurs formes oxydées, dans lequel le substituant facultatif du cycle à 5 ou 6 chaînons est choisi parmi alkyle en C₁-C₃, alcoxy en C₁-C₃, CONR⁵R⁶, halogène, cyano, oxo, hydroxyle, amino ou pyrazole éventuellement substitué par un alkyle en C₁-C₃ ;
ou R¹ est lié à R² pour former un cycle monocyclique ou bicyclique, éventuellement substitué, contenant 0, 1, 2 ou 3 hétéroatomes choisis parmi O, N et S et leurs formes oxydées, dans lequel les substituants facultatifs du cycle monocyclique ou bicyclique sont choisis parmi alkyle en C₁-C₆, alcoxy en C₁-C₃, halogène, cyano, oxo, hydroxyle, amino ou un groupe -(CH₂)ₙ-aryle dans lequel n est 0-3 ;
R² est choisi parmi hydrogène ; fluor ; cyano ; hydroxy ; amino ; et un groupe hydrocarboné en C₁-₃ qui est éventuellement substitué par un à six atomes de fluor et dans lequel un des atomes de carbone du groupe hydrocarboné peut éventuellement être remplacé par un hétéroatome choisi parmi O, N et S et leurs formes oxydées, ou R² est lié à R¹ pour former un cycle monocyclique ou bicyclique, éventuellement substitué, contenant 0, 1, 2 ou 3 hétéroatomes choisis parmi O, N et S et leurs formes oxydées, dans lequel les substituants facultatifs du cycle monocyclique ou bicyclique sont choisis parmi alkyle en C₁-C₆, alcoxy en C₁-C₃, halogène, cyano, oxo, hydroxyle, amino ou un groupe -(CH₂)ₙ-aryle dans lequel n est 0-3 ;
R⁴ est H, halogéno, OH, CN, un groupe alkyle en C₁₋₆ ou un groupe cycloalkyle en C₃₋₆, dans lequel les groupes alkyle et cycloalkyle sont éventuellement substitués par un ou plusieurs atomes de fluor, et dans lequel un atome quelconque du groupe alkyle ou cycloalkyle peut être éventuellement remplacé par un hétéroatome O ;
R⁵ est choisi parmi hydrogène, COCH₃, un groupe hydrocarboné non aromatique en C₁₋₁₀ éventuellement substitué par un ou plusieurs atomes de fluor dans lequel un atome quelconque du groupe hydrocarboné en C₁₋₁₀ peut être éventuellement remplacé par un hétéroatome choisi parmi O, N et S, un groupe -(CH₂)ₙ-aryle, dans lequel n est 0-3, ou R⁵ peut être joint à R⁶ pour former un cycle monocyclique ou bicyclique, éventuellement substitué, contenant 0, 1, 2 ou 3 hétéroatomes choisis parmi O, N et S et leurs formes oxydées, dans lequel les substituants facultatifs sont choisis parmi alkyle en C₁-C₃, alcoxy en C₁-C₃, halogène, cyano, oxo, hydroxyle ou amino, dans lequel les groupes alkyle en C₁-C₃ ou alcoxy en C₁-C₃ peuvent avoir un substituant facultatif choisi parmi halogène, cyano, oxo, hydroxyle ou amino ;
R⁶ est choisi parmi hydrogène, un groupe hydrocarboné non aromatique en C₁₋₁₀ éventuellement substitué par un ou plusieurs atomes de fluor dans lequel un atome quelconque du groupe hydrocarboné en C₁₋₁₀ peut être éventuellement remplacé par un hétéroatome choisi parmi O, N et S, un groupe -(CH₂)ₙ-aryle, dans lequel n est 0-3, ou R⁶ peut être joint à R⁵ pour former un cycle monocyclique ou bicyclique, éventuellement substitué, contenant 0, 1, 2 ou 3 hétéroatomes choisis parmi O, N et S et leurs formes oxydées, dans lequel les substituants facultatifs sont choisis parmi alkyle en C₁-C₃, alcoxy en C₁-C₃, halogène, cyano, oxo, hydroxyle ou amino, dans lequel les groupes alkyle en C₁-C₃ ou alcoxy en C₁-C₃ peuvent avoir un substituant facultatif choisi parmi halogène, cyano, oxo, hydroxyle ou amino ; et
R⁷ est un groupe hydrocarboné non aromatique en C₁₋₆ éventuellement substitué par un ou plusieurs atomes de fluor.

2. Composé selon la revendication 1, ou un sel de celui-ci, dans lequel :
R¹ est choisi parmi NR⁵R⁶ ; CONR⁵R⁶ ; et COOR⁷ ; ou
R¹ est un cycle à 5 ou 6 chaînons, éventuellement substitué, contenant 0, 1 ou 2 hétéroatomes azotés, dans lequel les substituants facultatifs sont choisis parmi alkyle en C₁-C₃, alcoxy en C₁-C₃, CONR⁵R⁶, halogène, cyano, oxo, hydroxyle, amino ou un cycle hétérocyclique éventuellement substitué contenant 1 ou 2 hétéroatomes choisis parmi O ou N dans lequel le substituant facultatif est un alkyle en C₁-C₃.

3. Composé selon la revendication 1 ou 2, ou un sel de celui-ci, dans lequel :
R¹ est choisi parmi NR⁵R⁶, CONR⁵R⁶ ou COOR⁷.

4. Composé selon l'une quelconque des revendications 1 à 3, ou un sel de celui-ci, dans lequel :
R² est H.

5. Composé selon l'une quelconque des revendications 1 à 4, ou un sel de celui-ci, dans lequel :
R¹ est choisi parmi NR⁵R⁶, CONR⁵R⁶ ou COOR⁷ ;
R⁵ est choisi parmi COCH₃, un groupe hydrocarboné non aromatique en C₁₋₆ ; et
R⁶ est choisi parmi hydrogène et un groupe hydrocarboné non aromatique en C₁₋₆.

6. Composé selon l'une quelconque des revendications 1 à 5, ou un sel de celui-ci, dans lequel le composé est de formule (26) : 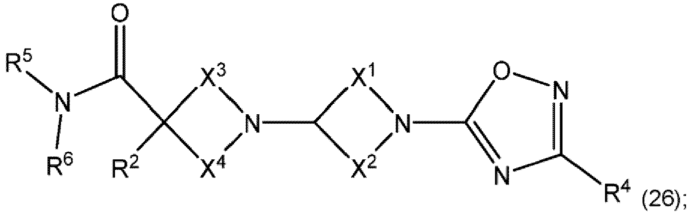 ou un sel de celui-ci ; dans lequel :
R², R⁴, R⁵, R⁶, X¹, X², X³ et X⁴ sont tels que définis dans l'une quelconque des revendications 1 à 5.

7. Composé selon la revendication 1, ou un sel de celui-ci, dans lequel R¹ est choisi parmi :

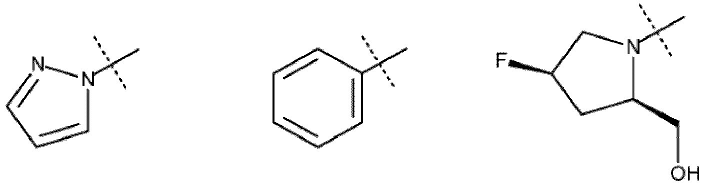

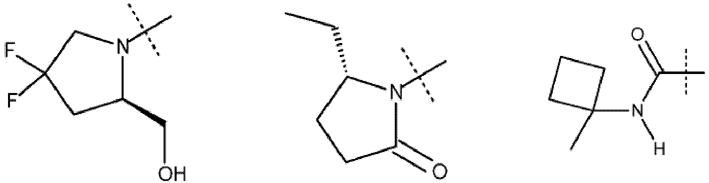

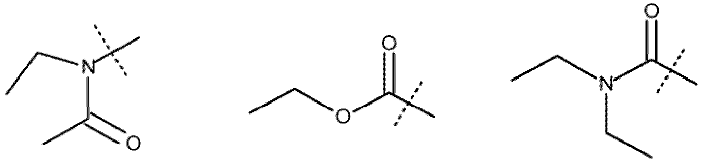

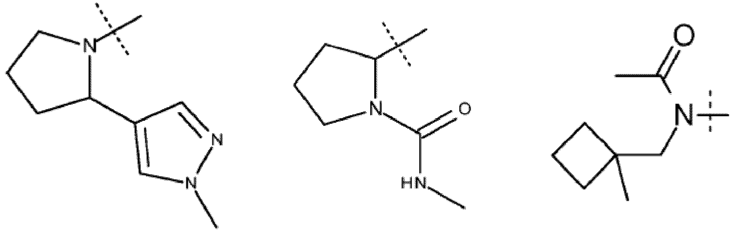

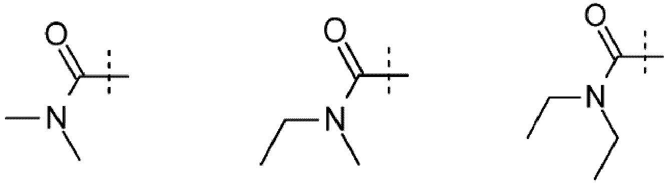

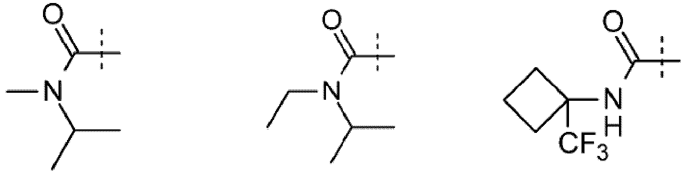

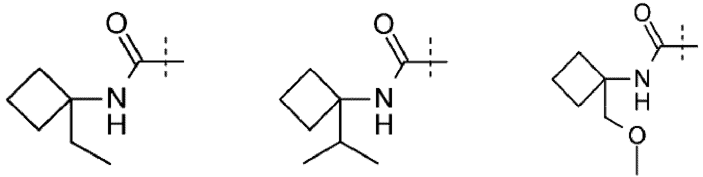

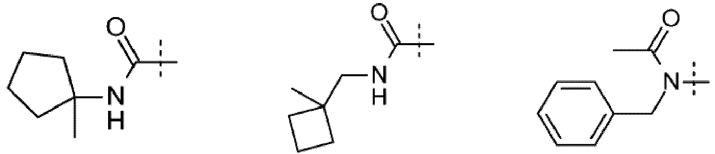

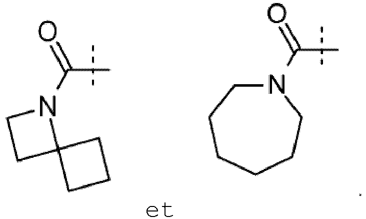

8. Composé selon l'une quelconque des revendications 1 à 7, ou un sel de celui-ci, dans lequel :
R⁴ est méthyle, trifluorométhyle, éthyle, isopropyle ou cyclopropyle.

9. Composé selon l'une quelconque des revendications 1 à 3 et 5 à 8, ou un sel de celui-ci, dans lequel :
R⁴ est trifluorométhyle.

10. Composé selon l'une quelconque des revendications 1 à 9, ou un sel de celui-ci, dans lequel le système cyclique formé par le fragment : 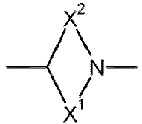 est choisi parmi : 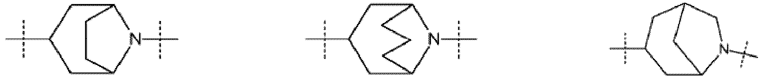 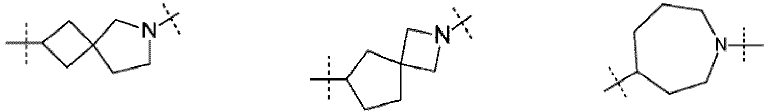 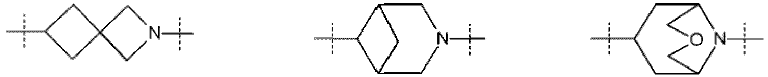 et

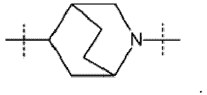

11. Composé selon la revendication 1, ou un sel de celui-ci, qui est un composé de formule (2) : 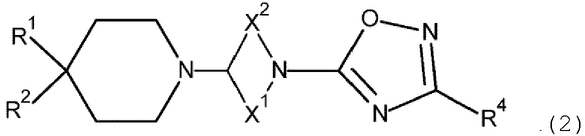 ou un sel de celui-ci, dans lequel :
X¹, X², R¹, R² et R⁴ sont tels que définis dans l'une quelconque des revendications 1 à 10.

12. Composé selon la revendication 1, ou un sel de celui-ci, qui est choisi parmi :
2-(3-méthyl-1,2,4-oxadiazol-5-yl)-6-[4-(1H-pyrazol-1-yl)pipéridin-1-yl]-2-azaspiro[3.3]heptane ;
6-(3-méthyl-1,2,4-oxadiazol-5-yl)-2-[4-(1H-pyrazol-1-yl)pipéridin-1-yl]-6-azaspiro[3.4]octane ;
1-[6-(3-méthyl-1,2,4-oxadiazol-5-yl)-6-azaspiro[3.4]oct-2-yl]-4-phénylpipéridine-4-carbonitrile ;
(1R,5S,6r)-3-[6-(3-méthyl-1,2,4-oxadiazol-5-yl)-6-azaspiro[3.4]oct-2-yl]-3-azabicyclo[3.1.0]hexane-6-carboxylate d'éthyle ;
2-(3-méthyl-1,2,4-oxadiazol-5-yl)-6-[4-(1H-pyrazol-1-yl)pipéridin-1-yl]-2-azaspiro[3.4]octane ;
3-(3-méthyl-1,2,4-oxadiazol-5-yl)-6-[4-(1H-pyrazol-1-yl)pipéridin-1-yl]-3-azabicyclo[3.1.1]heptane ;
8-(3-méthyl-1,2,4-oxadiazol-5-yl)-3-[4-(1H-pyrazol-1-yl)pipéridin-1-yl]-8-azabicyclo[3.2.1]octane ;
8-[8-(3-méthyl-1,2,4-oxadiazol-5-yl)-8-azabicyclo[3.2.1]oct-3-yl]-2,8-diazaspiro[4.5]décan-3-one ;
8-[8-(3-éthyl-1,2,4-oxadiazol-5-yl)-8-azabicyclo[3.2.1]oct-3-yl]-2,8-diazaspiro[4.5]décan-3-one ;
8-[8-(3-méthyl-1,2,4-oxadiazol-5-yl)-8-azabicyclo[3.2.1]octan-3-yl]-1-oxa-3,8-diazaspiro[4.5]décan-2-one ;
N-(1-méthylcyclobutyl)-1-[8-(3-méthyl-1,2,4-oxadiazol-5-yl)-8-azabicyclo[3.2.1]oct-3-yl]pipéridine-4-carboxamide ;
2-(3-méthyl-1,2,4-oxadiazol-5-yl)-5-[4-(1H-pyrazol-1-yl)pipéridin-1-yl]-2-azabicyclo[2.2.2]octane ;
9-(3-méthyl-1,2,4-oxadiazol-5-yl)-3-[4-(1H-pyrazol-1-yl)pipéridin-1-yl]-9-azabicyclo[3.3.1]nonane ;
8-[9-(3-méthyl-1,2,4-oxadiazol-5-yl)-9-azabicyclo[3.3.1]non-3-yl]-2,8-diazaspiro[4.5]décan-3-one ;
8-[9-(3-méthyl-1,2,4-oxadiazol-5-yl)-9-azabicyclo[3.3.1]non-3-yl]-1-oxa-3,8-diazaspiro[4.5]décan-2-one ;
N-(1-méthylcyclobutyl)-1-[9-(3-méthyl-1,2,4-oxadiazol-5-yl)-9-azabicyclo[3.3.1]non-3-yl]pipéridine-4-carboxamide ;
9-(3-méthyl-1,2,4-oxadiazol-5-yl)-7-[4-(1H-pyrazol-1-yl)pipéridin-1-yl]-3-oxa-9-azabicyclo[3.3.1]nonane ;
(1R,5S,6r)-N,N-diéthyl-3-[9-(3-méthyl-1,2,4-oxadiazol-5-yl)-3-oxa-9-azabicyclo[3.3.1]non-7-yl]-3-azabicyclo[3.1.0]hexane-6-carboxamide ;
1-(3-méthyl-1,2,4-oxadiazol-5-yl)-4-[4-(1H-pyrazol-1-yl)pipéridin-1-yl]azépane ;
8-[1-(3-méthyl-1,2,4-oxadiazol-5-yl)azépan-4-yl]-2,8-diazaspiro[4.5]décan-3-one ;
8-[1-(3-méthyl-1,2,4-oxadiazol-5-yl)azépan-4-yl]-1-oxa-3,8-diazaspiro[4.5]décan-2-one ;
4-éthyl-8-[1-(3-méthyl-1,2,4-oxadiazol-5-yl)azépan-4-yl]-1-oxa-3,8-diazaspiro[4.5]décan-2-one ;
4,4-diméthyl-8-[1-(3-méthyl-1,2,4-oxadiazol-5-yl)azépan-4-yl]-1-oxa-3,8-diazaspiro[4.5]décan-2-one ;
1'-[1-(3-méthyl-1,2,4-oxadiazol-5-yl)azépan-4-yl]spiro[indole-3,4'-pipéridin]-2(1H)-one ;
[(2R,4R)-4-fluoro-1-{1-[1-(3-méthyl-1,2,4-oxadiazol-5-yl)azépan-4-yl]pipéridin-4-yl}pyrrolidin-2-yl]méthanol ;
[(2R)-4,4-difluoro-1-{1-[1-(3-méthyl-1,2,4-oxadiazol-5-yl)azépan-4-yl]pipéridin-4-yl}pyrrolidin-2-yl]méthanol ;
(5S)-5-éthyl-1-{1-[1-(3-méthyl-1,2,4-oxadiazol-5-yl)azépan-4-yl]pipéridin-4-yl}pyrrolidin-2-one ;
1-(3-méthyl-1,2,4-oxadiazol-5-yl)-4-{4-[2-(1-méthyl-1H-pyrazol-4-yl)pyrrolidin-1-yl]pipéridin-1-yl}azépane ;
N-éthyl-N-{1-[1-(3-méthyl-1,2,4-oxadiazol-5-yl)azépan-4-yl]pipéridin-4-yl}acétamide ;
(2S)-N-méthyl-2-{1-[1-(3-méthyl-1,2,4-oxadiazol-5-yl)azépan-4-yl]pipéridin-4-yl}pyrrolidine-1-carboxamide ;
6-(3-méthyl-1,2,4-oxadiazol-5-yl)-3-[4-(1H-pyrazol-1-yl)pipéridin-1-yl]-6-azabicyclo[3.2.1]octane ;
8-[6-(3-méthyl-1,2,4-oxadiazol-5-yl)-6-azabicyclo[3.2.1]oct-3-yl]-2,8-diazaspiro[4.5]décan-3-one ;
8-[6-(3-méthyl-1,2,4-oxadiazol-5-yl)-6-azabicyclo[3.2.1]oct-3-yl]-1-oxa-3,8-diazaspiro[4.5]décan-2-one ;
8-(2-(3-méthyl-1,2,4-oxadiazol-5-yl)-2-azabicyclo[2.2.2]octan-5-yl)-2,8-diazaspiro[4.5]décan-3-one ;
8-(2-(3-méthyl-1,2,4-oxadiazol-5-yl)-2-azabicyclo[2.2.2]octan-5-yl)-1-oxa-3,8-diazaspiro[4.5]décan-2-one ;
8-(8-(3-(trifluorométhyl)-1,2,4-oxadiazol-5-yl)-8-azabicyclo[3.2.1]octan-3-yl)-2,8-diazaspiro[4.5]décan-3-one ;
8-(8-(3-(trifluorométhyl)-1,2,4-oxadiazol-5-yl)-8-azabicyclo[3.2.1]octan-3-yl)-1-oxa-3,8-diazaspiro[4.5]décan-2-one ;
(1R,5S,6r)-3-(2-(3-méthyl-1,2,4-oxadiazol-5-yl)-2-azaspiro[3.4]octan-6-yl)-N-(1-méthylcyclobutyl)-3-azabicyclo[3.1.0]hexane-6-carboxamide ;
1-(8-(3-méthyl-1,2,4-oxadiazol-5-yl)-8-azabicyclo[3.2.1]octan-3-yl)-N-(1-(trifluorométhyl)cyclobutyl)pipéridine-4-carboxamide ;
N-(1-éthylcyclobutyl)-1-(8-(3-méthyl-1,2,4-oxadiazol-5-yl)-8-azabicyclo[3.2.1]octan-3-yl)pipéridine-4-carboxamide ;
N-(1-isopropylcyclobutyl)-1-(8-(3-méthyl-1,2,4-oxadiazol-5-yl)-8-azabicyclo[3.2.1]octan-3-yl)pipéridine-4-carboxamide ;
1-(8-(3-méthyl-1,2,4-oxadiazol-5-yl)-8-azabicyclo[3.2.1]octan-3-yl)-N-(1-méthylcyclopentyl)pipéridine-4-carboxamide ;
1-(8-(3-méthyl-1,2,4-oxadiazol-5-yl)-8-azabicyclo[3.2.1]octan-3-yl)-N-((1-méthylcyclobutyl)méthyl)pipéridine-4-carboxamide ;
(1R,5S,6r)-3-(8-(3-méthyl-1,2,4-oxadiazol-5-yl)-8-azabicyclo[3.2.1]octan-3-yl)-N-(1-méthylcyclobutyl)-3-azabicyclo[3.1.0]hexane-6-carboxamide ;
N-(1-méthylcyclobutyl)-1-(8-(3-(trifluorométhyl)-1,2,4-oxadiazol-5-yl)-8-azabicyclo[3.2.1]octan-3-yl)pipéridine-4-carboxamide ;
1-(2-(3-méthyl-1,2,4-oxadiazol-5-yl)-2-azabicyclo[2.2.2]octan-5-yl)-N-(1-méthylcyclobutyl)pipéridine-4-carboxamide ;
1-(2-(3-méthyl-1,2,4-oxadiazol-5-yl)-2-azabicyclo[2.2.2]octan-5-yl)-N-((1-méthylcyclobutyl)méthyl)pipéridine-4-carboxamide ;
N-(1-(9-(3-méthyl-1,2,4-oxadiazol-5-yl)-3-oxa-9-azabicyclo[3.3.1]nonan-7-yl)pipéridin-4-yl)-N-((1-méthylcyclobutyl)méthyl)acétamide ;
N-(1-(8-(3-méthyl-1,2,4-oxadiazol-5-yl)-8-azabicyclo[3.2.1]octan-3-yl)pipéridin-4-yl)-N-((1-méthylcyclobutyl)méthyl)acétamide ;
N-(1-(2-(3-méthyl-1,2,4-oxadiazol-5-yl)-2-azabicyclo[2.2.2]octan-5-yl)pipéridin-4-yl)-N-((1-méthylcyclobutyl)méthyl)acétamide ;
N-benzyl-N-(1-(9-(3-méthyl-1,2,4-oxadiazol-5-yl)-3-oxa-9-azabicyclo[3.3.1]nonan-7-yl)pipéridin-4-yl)acétamide ;
((1R,5S,6r)-3-(9-(3-méthyl-1,2,4-oxadiazol-5-yl)-3-oxa-9-azabicyclo[3.3.1]nonan-7-yl)-3-azabicyclo[3.1.0]hexan-6-yl)(1-azaspiro[3.3]heptan-1-yl)méthanone ;
(1R,5S,6r)-N-isopropyl-N-méthyl-3-(9-(3-méthyl-1,2,4-oxadiazol-5-yl)-3-oxa-9-azabicyclo[3.3.1]nonan-7-yl)-3-azabicyclo[3.1.0]hexane-6-carboxamide ;
(1R,5S,6r)-N,N-diéthyl-3-(9-(3-(trifluorométhyl)-1,2,4-oxadiazol-5-yl)-3-oxa-9-azabicyclo[3.3.1]nonan-7-yl)-3-azabicyclo[3.1.0]hexane-6-carboxamide ;
(1R,5S,6r)-N-éthyl-N-méthyl-3-(8-(3-méthyl-1,2,4-oxadiazol-5-yl)-8-azabicyclo[3.2.1]octan-3-yl)-3-azabicyclo[3.1.0]hexane-6-carboxamide ;
(1R,5S,6r)-N,N-diéthyl-3-(8-(3-méthyl-1,2,4-oxadiazol-5-yl)-8-azabicyclo[3.2.1]octan-3-yl)-3-azabicyclo[3.1.0]hexane-6-carboxamide ;
(1R,5S,6r)-N,N-diéthyl-3-(2-(3-méthyl-1,2,4-oxadiazol-5-yl)-2-azaspiro[3.4]octan-6-yl)-3-azabicyclo[3.1.0]hexane-6-carboxamide ;
(1R,5S,6r)-N-éthyl-N-méthyl-3-(2-(3-méthyl-1,2,4-oxadiazol-5-yl)-2-azaspiro[3.4]octan-6-yl)-3-azabicyclo[3.1.0]hexane-6-carboxamide ;
N,N-diéthyl-1-(2-(3-méthyl-1,2,4-oxadiazol-5-yl)-2-azaspiro[3.4]octan-6-yl)pipéridine-4-carboxamide ;
8-(8-(3-méthyl-1,2,4-oxadiazol-5-yl)-8-azabicyclo[3.2.1]octan-3-yl)-2,8-diazaspiro[4.6]undécan-3-one ;
8-(8-(3-méthyl-1,2,4-oxadiazol-5-yl)-8-azabicyclo[3.2.1]octan-3-yl)-1-oxa-3,8-diazaspiro[4.6]undécan-2-one ;
8-(2-(3-méthyl-1,2,4-oxadiazol-5-yl)-2-azabicyclo[2.2.2]octan-5-yl)-2,8-diazaspiro[4.6]undécan-3-one ;
8-(2-(3-méthyl-1,2,4-oxadiazol-5-yl)-2-azabicyclo[2.2.2]octan-5-yl)-1-oxa-3,8-diazaspiro[4.6]undécan-2-one ;
8-(6-(3-méthyl-1,2,4-oxadiazol-5-yl)-6-azabicyclo[3.2.1]octan-3-yl)-2,8-diazaspiro[4.6]undécan-3-one ;
8-(6-(3-méthyl-1,2,4-oxadiazol-5-yl)-6-azabicyclo[3.2.1]octan-3-yl)-1-oxa-3,8-diazaspiro[4.6]undécan-2-one ;
((1R,5S,6r)-3-(6-(3-méthyl-1,2,4-oxadiazol-5-yl)-6-azabicyclo[3.2.1]octan-3-yl)-3-azabicyclo[3.1.0]hexan-6-yl)(1-azaspiro[3.3]heptan-1-yl)méthanone ;
(1R,5S,6r)-N,N-diéthyl-3-(6-(3-méthyl-1,2,4-oxadiazol-5-yl)-6-azabicyclo[3.2.1]octan-3-yl)-3-azabicyclo[3.1.0]hexane-6-carboxamide ;
(1R,5S,6r)-N-éthyl-N-isopropyl-3-(6-(3-méthyl-1,2,4-oxadiazol-5-yl)-6-azabicyclo[3.2.1]octan-3-yl)-3-azabicyclo[3.1.0]hexane-6-carboxamide ;
(1R,5S,6r)-N-isopropyl-N-méthyl-3-(6-(3-méthyl-1,2,4-oxadiazol-5-yl)-6-azabicyclo[3.2.1]octan-3-yl)-3-azabicyclo[3.1.0]hexane-6-carboxamide ;
(1R,5S,6r)-N-éthyl-N-méthyl-3-(9-(3-méthyl-1,2,4-oxadiazol-5-yl)-9-azabicyclo[3.3.1]nonan-3-yl)-3-azabicyclo[3.1.0]hexane-6-carboxamide ;
(1R,5S,6r)-N,N-diéthyl-3-(9-(3-méthyl-1,2,4-oxadiazol-5-yl)-9-azabicyclo[3.3.1]nonan-3-yl)-3-azabicyclo[3.1.0]hexane-6-carboxamide ;
8-(1-(3-(trifluorométhyl)-1,2,4-oxadiazol-5-yl)azépan-4-yl)-2,8-diazaspiro[4.5]décan-3-one ;
8-(1-(3-(trifluorométhyl)-1,2,4-oxadiazol-5-yl)azépan-4-yl)-2,8-diazaspiro[4.6]undécan-3-one ;
8-(1-(3-méthyl-1,2,4-oxadiazol-5-yl)azépan-4-yl)-2,8-diazaspiro[4.6]undécan-3-one ;
8-(1-(3-(trifluorométhyl)-1,2,4-oxadiazol-5-yl)azépan-4-yl)-1-oxa-3,8-diazaspiro[4.5]décan-2-one ;
8-(8-(3-éthyl-1,2,4-oxadiazol-5-yl)-8-azabicyclo[3.2.1]octan-3-yl)-1-oxa-3,8-diazaspiro[4.5]décan-2-one ;
8-(2-(3-méthyl-1,2,4-oxadiazol-5-yl)-2-azabicyclo[2.2.2]octan-5-yl)-1-oxa-3,8-diazaspiro[4.5]décan-2-one ;
8-(6-(3-(trifluorométhyl)-1,2,4-oxadiazol-5-yl)-6-azabicyclo[3.2.1]octan-3-yl)-1-oxa-3,8-diazaspiro[4.5]décan-2-one ;
4-méthyl-8-(8-(3-méthyl-1,2,4-oxadiazol-5-yl)-8-azabicyclo[3.2.1]octan-3-yl)-1-oxa-3,8-diazaspiro[4.5]décan-2-one ;
(1R,5S,6r)-3-(6-(3-méthyl-1,2,4-oxadiazol-5-yl)-6-azaspiro[3.4]octan-2-yl)-N-(1-méthylcyclobutyl)-3-azabicyclo[3.1.0]hexane-6-carboxamide ;
N-(1-(6-(3-méthyl-1,2,4-oxadiazol-5-yl)-6-azaspiro[3.4]octan-2-yl)pipéridin-4-yl)-N-((1-méthylcyclobutyl)méthyl)acétamide ;
N-(1-(9-(3-méthyl-1,2,4-oxadiazol-5-yl)-3-oxa-9-azabicyclo[3.3.1]nonan-7-yl)pipéridin-4-yl)-N-((1-méthylcyclobutyl)méthyl)acétamide ;
N-(1-(8-(3-méthyl-1,2,4-oxadiazol-5-yl)-8-azabicyclo[3.2.1]octan-3-yl)pipéridin-4-yl)-N-((1-méthylcyclobutyl)méthyl)acétamide ;
(1R,5S,6r)-N-isopropyl-N-méthyl-3-(8-(3-méthyl-1,2,4-oxadiazol-5-yl)-8-azabicyclo[3.2.1]octan-3-yl)-3-azabicyclo[3.1.0]hexane-6-carboxamide ;
(1R,5S,6r)-N-éthyl-N-méthyl-3-(2-(3-méthyl-1,2,4-oxadiazol-5-yl)-2-azabicyclo[2.2.2]octan-5-yl)-3-azabicyclo[3.1.0]hexane-6-carboxamide ;
(1R,5S,6r)-N-éthyl-N-méthyl-3-(9-(3-méthyl-1,2,4-oxadiazol-5-yl)-9-azabicyclo[3.3.1]nonan-3-yl)-3-azabicyclo[3.1.0]hexane-6-carboxamide ;
8-(1-(3-méthyl-1,2,4-oxadiazol-5-yl)azépan-4-yl)-1-oxa-3,8-diazaspiro[4.5]décan-2-one ;
8-(1-(3-(trifluorométhyl)-1,2,4-oxadiazol-5-yl)azépan-4-yl)-2,8-diazaspiro[4.5]décan-3-one ;
8-(1-(3-(trifluorométhyl)-1,2,4-oxadiazol-5-yl)azépan-4-yl)-1-oxa-3,8-diazaspiro[4.5]décan-2-one ;
8-(8-(3-cyclopropyl-1,2,4-oxadiazol-5-yl)-8-azabicyclo[3.2.1]octan-3-yl)-2,8-diazaspiro[4.5]décan-3-one ;
8-(8-(3-isopropyl-1,2,4-oxadiazol-5-yl)-8-azabicyclo[3.2.1]octan-3-yl)-2,8-diazaspiro[4.5]décan-3-one ;
8-(8-(3-éthyl-1,2,4-oxadiazol-5-yl)-8-azabicyclo[3.2.1]octan-3-yl)-1-oxa-3,8-diazaspiro[4.5]décan-2-one ;
4-benzyl-8-(8-(3-méthyl-1,2,4-oxadiazol-5-yl)-8-azabicyclo[3.2.1]octan-3-yl)-1-oxa-3,8-diazaspiro[4.5]décan-2-one ;
1-isopropyl-8-(8-(3-méthyl-1,2,4-oxadiazol-5-yl)-8-azabicyclo[3.2.1]octan-3-yl)-2,8-diazaspiro[4.5]décan-3-one ;
4-isopropyl-8-(8-(3-méthyl-1,2,4-oxadiazol-5-yl)-8-azabicyclo[3.2.1]octan-3-yl)-1-oxa-3,8-diazaspiro[4.5]décan-2-one ;
1-cyclopropyl-8-(8-(3-méthyl-1,2,4-oxadiazol-5-yl)-8-azabicyclo[3.2.1]octan-3-yl)-2,8-diazaspiro[4.5]décan-3-one ;
8-(8-(3-cyclopropyl-1,2,4-oxadiazol-5-yl)-8-azabicyclo[3.2.1]octan-3-yl)-1-oxa-3,8-diazaspiro[4.5]décan-2-one ;
8-(8-(3-isopropyl-1,2,4-oxadiazol-5-yl)-8-azabicyclo[3.2.1]octan-3-yl)-1-oxa-3,8-diazaspiro[4.5]décan-2-one ;
N-benzyl-N-(1-(8-(3-méthyl-1,2,4-oxadiazol-5-yl)-8-azabicyclo[3.2.1]octan-3-yl)pipéridin-4-yl)acétamide ;
(1R,5S,6r)-N,N-diéthyl-3-(2-(3-méthyl-1,2,4-oxadiazol-5-yl)-2-azaspiro[3.3]heptan-6-yl)-3-azabicyclo[3.1.0]hexane-6-carboxamide ;
((1R,5S,6r)-3-(6-(3-méthyl-1,2,4-oxadiazol-5-yl)-6-azaspiro[3.4]octan-2-yl)-3-azabicyclo[3.1.0]hexan-6-yl)(1-azaspiro[3.3]heptan-1-yl)méthanone ;
azépan-1-yl(1-(8-(3-méthyl-1,2,4-oxadiazol-5-yl)-8-azabicyclo[3.2.1]octan-3-yl)pipéridin-4-yl)méthanone ;
1-(cyclopropylméthyl)-8-(8-(3-méthyl-1,2,4-oxadiazol-5-yl)-8-azabicyclo[3.2.1]octan-3-yl)-2,8-diazaspiro[4.5]décan-3-one ;
4-(cyclopropylméthyl)-8-(8-(3-méthyl-1,2,4-oxadiazol-5-yl)-8-azabicyclo[3.2.1]octan-3-yl)-1-oxa-3,8-diazaspiro[4.5]décan-2-one ;
4-cyclopropyl-8-(8-(3-méthyl-1,2,4-oxadiazol-5-yl)-8-azabicyclo[3.2.1]octan-3-yl)-1-oxa-3,8-diazaspiro[4.5]décan-2-one ;
1-benzyl-8-(6-(3-méthyl-1,2,4-oxadiazol-5-yl)-6-azaspiro[3.4]octan-2-yl)-2,8-diazaspiro[4.5]décan-3-one ;
4-benzyl-8-(2-(3-méthyl-1,2,4-oxadiazol-5-yl)-2-azaspiro[3.3]heptan-6-yl)-1-oxa-3,8-diazaspiro[4.5]décan-2-one ;
1-benzyl-8-(1-(3-méthyl-1,2,4-oxadiazol-5-yl)azépan-4-yl)-2,8-diazaspiro[4.5]décan-3-one ;
8-(8-(3-méthyl-1,2,4-oxadiazol-5-yl)-8-azabicyclo[3.2.1]octan-3-yl)-2,8-diazaspiro[4.6]undécan-3-one ;
1-benzyl-8-(8-(3-méthyl-1,2,4-oxadiazol-5-yl)-8-azabicyclo[3.2.1]octan-3-yl)-2,8-diazaspiro[4.5]décan-3-one ;
8-(8-(3-méthyl-1,2,4-oxadiazol-5-yl)-8-azabicyclo[3.2.1]octan-3-yl)-1-oxa-3,8-diazaspiro[4.6]undécan-2-one ;
(1R,5S,6r)-3-(8-(3-méthyl-1,2,4-oxadiazol-5-yl)-8-azabicyclo[3.2.1]octan-3-yl)-N-(1-(trifluorométhyl)cyclobutyl)-3-azabicyclo[3.1.0]hexane-6-carboxamide ; et
(1R,5S,6r)-3-(2-(3-méthyl-1,2,4-oxadiazol-5-yl)-2-azabicyclo[2.2.2]octan-5-yl)-N-(1-(trifluorométhyl)cyclobutyl)-3-azabicyclo[3.1.0]hexane-6-carboxamide ;
ou un sel de celui-ci.

13. Composé selon la revendication 1, ou un sel de celui-ci, qui est :
N-(1-méthylcyclobutyl)-1-(8-(3-(trifluorométhyl)-1,2,4-oxadiazol-5-yl)-8-azabicyclo[3.2.1]octan-3-yl)pipéridine-4-carboxamide ; ou un sel de celui-ci.

14. Composé selon la revendication 1, qui est :
N-(1-méthylcyclobutyl)-1-(8-(3-(trifluorométhyl)-1,2,4-oxadiazol-5-yl)-8-azabicyclo[3.2.1]octan-3-yl)pipéridine-4-carboxamide.

15. Composé selon la revendication 1, ou un sel de celui-ci, qui est :
(1R,5S,6r)-N,N-diéthyl-3-(9-(3-(trifluorométhyl)-1,2,4-oxadiazol-5-yl)-3-oxa-9-azabicyclo[3.3.1]nonan-7-yl)-3-azabicyclo[3.1.0]hexane-6-carboxamide ; ou un sel de celui-ci.

16. Composé selon la revendication 1, qui est :
(1R,5S,6r)-N,N-diéthyl-3-(9-(3-(trifluorométhyl)-1,2,4-oxadiazol-5-yl)-3-oxa-9-azabicyclo[3.3.1]nonan-7-yl)-3-azabicyclo[3.1.0]hexane-6-carboxamide.

17. Composé selon l'une quelconque des revendications 1 à 16, ou un sel de celui-ci, pour une utilisation en médecine.

18. Composition pharmaceutique comprenant un composé tel que défini dans l'une quelconque des revendications 1 à 16, ou un sel de celui-ci, et un excipient pharmaceutiquement acceptable.

19. Composé selon l'une quelconque des revendications 1 à 16, ou un sel de celui-ci, ou la composition pharmaceutique selon la revendication 18, pour une utilisation dans le traitement d'un trouble cognitif ou d'un trouble psychotique ; ou dans le traitement ou l'atténuation de la sévérité d'une douleur aiguë, d'une douleur chronique, d'une douleur neuropathique ou d'une douleur inflammatoire.

20. Composé selon l'une quelconque des revendications 1 à 16, ou un sel de celui-ci, ou la composition pharmaceutique selon la revendication 18, pour une utilisation dans le traitement d'un trouble cognitif ou d'un trouble psychotique.

21. Composé selon l'une quelconque des revendications 1 à 16, ou un sel de celui-ci, ou la composition pharmaceutique selon la revendication 18, pour une utilisation dans le traitement d'un trouble cognitif.

22. Composé, sel ou composition pour une utilisation selon l'une quelconque des revendications 19 à 21, dans lequel :
le trouble cognitif résulte de ou est associé à la maladie d'Alzheimer.

23. Composé, sel ou composition pour une utilisation selon l'une quelconque des revendications 19 à 21, dans lequel :
le trouble cognitif est une démence à corps de Lewy.

24. Composé selon l'une quelconque des revendications 1 à 16, ou un sel de celui-ci, ou la composition pharmaceutique selon la revendication 18, pour une utilisation dans le traitement de la maladie d'Alzheimer.
